# EUROPEAN PATENT SPECIFICATION

(11) **EP 2 320 932 B1**
(45) Date of publication and mention of the grant of the patent: **15.07.2015**
(21) Application number: 09791315.6
(22) Date of filing: 07.08.2009
(51) Int. Cl.: A61K 38/17, A61K 38/08, A61K 38/10, C07K 7/06, C07K 7/08, C07K 14/47, A61K 39/00, G01N 33/68, G01N 33/574, C12N 15/11, A61P 35/00

(54) **METHODS OF PREDICTING CANCER LETHALITY USING REPLIKIN COUNTS**
VERFAHREN ZUR VORHERSAGE VON KREBSLETALITÄT UNTER VERWENDUNG VON REPLIKIN-ZAHLEN
PROCÉDÉS DE PRÉDICTION DE LA LÉTALITÉ DU CANCER EN UTILISANT DES COMPTAGES DE REPLIKIN

(30) Priority: 08.08.2008 US 87354 P; 09.01.2009 US 143618 P; 23.04.2009 US 172115 P; 23.04.2009 US 429044; 23.04.2009 WO PCT/US2009/004156; 19.05.2009 US 179686 P; 08.06.2009 US 185160 P
(43) Date of publication of application: 18.05.2011
(73) Proprietor: Bogoch, Samuel, New York, NY 10128 (US); Bogoch, Elenore S., New York, NY 10128 (US); Bogoch, Samuel Winston, Oakland, CA 94609 (US); Borsanyi, Anne Elenore, Brookline, MA 02466 (US)
(72) Inventor: Bogoch, Samuel, New York, NY 10128 (US); Bogoch, Elenore S., New York, NY 10128 (US); Bogoch, Samuel Winston, Oakland, CA 94609 (US); Borsanyi, Anne Elenore, Brookline, MA 02466 (US)
(74) Representative: Ledl, Andreas
(86) International application number: PCT/US2009/053208
(87) International publication number: WO 2010/017514

(56) References cited:
- WO-A-02/085093
- WO-A-2006/088962
- WO-A2-2008/143717

## Description

This application claims priority to U.S. Provisional Appln. Ser. No. 61/185,160, filed June 8, 2009, U.S. Provisional Appln. Ser. No. 61/179,686, filed May 19, 2009, U.S. Provisional Appln. Ser. No. 61/172,115, filed April 23, 2009, U.S. Appln. Ser. No. 12/429,044, filed April 23, 2009, PCT/US2009/41565, filed April 23, 2009, U.S. Provisional Appln. Ser. No. 61/143,618, filed January 9, 2009, and U.S. Provisional Appln. Ser. No. 61/087,354, filed August 8, 2008. This application additionally refers to: U.S. Provisional Appln. Ser. No. 61/054,010, filed May 16, 2008, U.S. Appln. Ser. No. 12/108,458, filed April 23, 2008, U.S. Appln. Ser. No. 12/010,027, filed January 18, 2008, U.S. Provisional Appln. Ser. No. 60/991,676, filed November 30, 2007, U.S. Appln. Ser. No. 11/923,559, filed October 24, 2007, U.S. Provisional Appln. Ser. No. 60/982,336, filed October 24, 2007, U.S. Provisional Appln. Ser. No. 60/982,333, filed October 24, 2007, U.S. Provisional Appln. Ser. No. 60/982,338, filed October 24, 2007, U.S. Provisional Appln. Ser. No. 60/935,816, filed August 31, 2007, U.S. Provisional Appln. Ser. No. 60/935,499 filed August 16, 2007, U.S. Provisional Appln. Ser. No. 60/954,743, filed August 8, 2007, U.S. Appln. Ser. No. 11/755,597, filed May 30, 2007, U.S. Provisional Appln. Ser. No. 60/898,097, filed January 30, 2007, U.S. Provisional Appln. Ser. No. 60/880,966, filed January 18, 2007, U.S. Provisional Appln. Ser. No. 60/853,744, filed October 24, 2006, U.S. Appln. Ser. No. 11/355,120, filed February 16, 2006, U.S. Appln. Ser. No. 11/116,203, filed April 28, 2005, U.S. Appln. Ser. No. 10/860,050, filed June 4, 2004, now U.S. Patent No. 7,442,761, U.S. Appln. Ser. No. 10/189,437, filed July 8, 2002, now U.S. Patent No. 7,452,963, U.S. Appln. Ser. No. 10/105,232, filed March 26, 2002, now U.S. Patent No. 7,189,800, U.S. Appln. Ser. No. 09/984,057, filed October 26, 2001, now U.S. Patent No. 7,420,028, and U.S. Appln. Ser. No. 09/984,056, filed October 26, 2001, now U.S. Patent No. 7,176,275.

### TECHNICAL FIELD OF THE INVENTION

This invention relates generally to identifying and quantifying the lethality of different histological types of malignancies and variations of lethality within these types. The invention is further directed to diagnosis, prevention and treatment of cancer.

### BACKGROUND OF THE INVENTION

Cancer is a class of diseases in which cells divide absent limits that normally control growth of cells in tissue. Uncontrolled cancer cell growth often leads to invasion and destruction of tissues adjacent to the cancer cells since cancer cells are typically capable of living in environments different from the tissue from which the cells were transformed. As a result, cancer cells often spread to other locations in the body where they may rapidly replicate causing additional tumors, resulting trauma, and sometimes death. The rate at which a line of cancer cells replicates is often a determining factor in the aggressiveness and eventual lethality of the cancer. Rates of replication for particular types of cancer are also considered in developing strategies for cancer therapy.

Nearly all cancer cells are abnormal in their genetic material as compared to cells from which they were transformed. Some progress has been made in developing therapies that more directly target the molecular abnormalities in cancer cells. These therapies ideally inhibit or kill cancer cells while not extensively damaging normal cells. Nevertheless, the progress that has been made in developing targeted therapies remains severely insufficient since about one-quarter of deaths in the United States in 2009 are expected to result from cancer.

Cancer prognosis has traditionally been based on histological identification of the type of cancer, as well as the stage of cancer development and the extent of progression of the disease in the body. More recently, histologic grading and the presence of specific molecular markers have become useful in prognosis and the development of individual treatments.

While histologic grading is useful for providing qualitative prognoses for patients suffering from a malignancy, histopathology does not provide a quantitative measure of the degree of malignancy or the rate of growth, rate of replication, aggressiveness, or lethality of a malignancy. Furthermore, histopathological procedures provide inconsistent measures of degrees of malignancies. Quantitative methods of determining the rate of growth, rate of replication, aggressiveness, or lethality of a malignancy are therefore needed.

Replikin peptides are a family of small peptides that have been correlated with the phenomenon of rapid replication in malignancies, as well as viruses, and other infectious organisms. The association of Replikin peptides with rapid replication has been described in U.S. Patent No. 7,189,800, U.S. Patent No. 7,176,275, U.S. Appln. Ser. No. 11/355,120, and U.S. Patent No. 7,442,761, among others. Both Replikin concentration (number of Replikins per 100 amino acids) and Replikin composition have been correlated with the functional phenomenon of rapid replication. US 61/087,354 describes methods of predicting the relative replication rate or relative lethality of a first malignancy as compared to another malignancy or as compared to a plurality of malignancies, including methods of prerdicting the relative replication rate or relative lethality of a primary malignancy as compared to a metastatic malignancy, comprising comparing the concentration of Replikin sequences in the Replikin Peak Gene of the first malignancy with the concentration of Replikin sequences in the Replikin Peak Genes identified within a malignancy for diagnostic, therapeutic, preventive, and predictive purposes.

There continues to be a need in the art for methods of determining the source, aggressiveness and lethality of malignancies in order to design optimally appropriate therapies. Analysis of Replikin sequences in the genome of malignancies provides such methods. Additionally, a need for methods of preventing and treating aggressive malignancies continues to exist in the art. Replikin concentration in the genome of the cell or organism and Replikin sequences identified in malignancies provide methods of preventing and treating aggressive malignancies.

### SUMMARY OF THE INVENTION

In one aspect, the present invention provides methods of predicting the relative rate of growth, rate of replication, and/or lethality of a first malignancy as compared to a second malignancy or as compared to a plurality of other malignancies comprising comparing a Replikin Count (Replikin concentration) in a cell from the first malignancy with a Replikin Count (Replikin concentration) in a cell from the second malignancy, or with the Replikin Counts (Replikin concentrations) in a plurality of cells from a plurality of Malignancies as further defined in claim 1. In another aspect, the present invention further provides a kit for providing a prognosis of a patient suffering from a malignancy as defined in claim 23.

A first non-limiting aspect of the invention provides a method of predicting the relative rate of growth of at least one first malignancy of a known or unknown type of malignancy as compared to at least one second malignancy of a known or unknown type of malignancy comprising: comparing a Replikin Count of the at least one first malignancy with a Replikin Count of at least one second malignancy; and predicting the at least one first malignancy to have a relative rate of growth that is faster than the relative rate of growth of the at least one second malignancy if the Replikin Count of the at least one first malignancy is greater than the Replikin Count of the at least one second malignancy as defined in claim 1. In a non-limiting embodiment of the first aspect of the invention, the at least one second malignancy is of an unknown type or of a different type of malignancy than the first malignancy. In another non-limiting embodiment, the first malignancy is a metastasis or metastases of the second malignancy.

In another non-limiting embodiment, the method of prediction further comprises determining a Replikin Count of the at least one first malignancy and a Replikin Count of the at least one second malignancy before the comparing step. In another non-limiting embodiment, the at least one first malignancy is a metastasis of the at least one second malignancy, is a malignancy of unknown type or unknown origin in a subject suffering from the at least one second malignancy, or is a malignant cell of the same malignancy as the at least one second malignancy, or the at least one first malignancy and the at least one second malignancy are metastases of a third malignancy.

In another non-limiting embodiment, the Replikin Count of the at least one first malignancy is the Replikin Count of a Replikin Peak Gene of the at least one first malignancy and the Replikin Count of the at least one second malignancy is a Replikin Count of a Replikin Peak Gene of the at least one second malignancy.

In another non-limiting embodiment, the Replikin Count of the at least one second malignancy is a mean Replikin Count of a plurality of malignancies or a mean Replikin Count of a plurality of cells of a malignancy, the Replikin Count of the at least one first malignancy is a mean Replikin Count of a plurality of malignancies or a mean Replikin Count of a plurality of cells of a malignancy, or the Replikin Count of the at least one second malignancy is a mean Replikin Count of a plurality of malignancies or a mean Replikin Count of a plurality of cells of a malignancy and the Replikin Count of the at least one first malignancy is a mean Replikin Count of a plurality of malignancies or a mean Replikin Count of a plurality of cells of a malignancy.

In a further non-limiting embodiment, the Replikin Count of the at least one second malignancy is a mean, median, or other average Replikin Count of a plurality of malignancies where the plurality of malignancies represents a survey of malignancies. In another non-limiting embodiment, the Replikin Count of the at least one first malignancy or the mean Replikin Count of the at least one first malignancy is greater than the mean Replikin Count of the at least one second malignancy plus the standard deviation of the mean Replikin Count of said at least one second malignancy.

In another non-limiting embodiment, the relative rate of growth correlates with relative lethality.

In another non-limiting embodiment, the Replikin Count of the at least one first malignancy is a Replikin Count of an expressed protein, protein fragment or peptide isolated from a cell of the at least one first malignancy or a Replikin Count of the genome, a gene, a gene fragment, or any other nucleic acid sequence isolated from a cell of the at least one first malignancy and the Replikin Count of the at least one second malignancy is a Replikin Count of an expressed protein, protein fragment or peptide isolated from a cell of the at least one second malignancy or a Replikin Count of the genome, a gene, a gene fragment, or any other nucleic acid sequence isolated from a cell of the at least one second malignancy.

In a further non-limiting embodiment, the Replikin Count of the at least one first malignancy is the highest Replikin Count among a plurality of Replikin Counts of a first plurality of malignancies of a first type and the Replikin Count of the at least one second malignancy is the highest Replikin Count among a plurality of Replikin Counts of a second plurality of malignancies of a second type. In a further non-limiting embodiment, a Replikin Count of the Replikin Peak Gene of the malignancy having the highest Replikin Count among a plurality of Replikin Counts of the first type of malignancy is further compared to the Replikin Count of the Replikin Peak Gene of the malignancy having the highest Replikin Count among a plurality of Replikin Counts of the second type of malignancy, and if the first type of malignancy has a higher Replikin Count of the Replikin Peak Gene than the second type of malignancy, then the first type of malignancy is predicted to have a greater lethality than the second type of malignancy.

In another non-limiting embodiment, the first malignancy or the second malignancy is a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, or a glioblastoma malignancy.

Another non-limiting embodiment of the first aspect of the invention provides a method of predicting the relative rate of growth of at least one first malignancy of a known or unknown type of malignancy as compared to at least one second malignancy of a known or unknown type of malignancy performed by a processor comprising: comparing a Replikin Count of the at least one first malignancy with a Replikin Count of at least one second malignancy; and predicting the at least one first malignancy to have a relative rate of growth that is faster than the relative rate of growth of the at least one second malignancy if the Replikin Count of the at least one first malignancy is greater than the Replikin Count of the at least one second malignancy. In a further non-limiting embodiment, at least one Replikin Count is outputted to a user or to a display. In another non-limiting embodiment, at least one representation of a prediction of the relative rate of growth of at least one first malignancy is outputted to a user or to a display.

Another non-limiting embodiment of the first aspect of the invention provides a machine-readable storage medium having stored thereon executable instructions that, when executed by a processor, performs the method of claim 1.

Another non-limiting embodiment of the first aspect of the invention provides a computer system, including a processor coupled to a network and a memory coupled to the processor, the memory containing a plurality of instructions to perform a method of predicting the relative rate of growth of at least one first malignancy as compared to at least one second malignancy.

A second non-limiting aspect of the present invention provides a method of determining the relative rate of increase in the relative rate of growth of at least one first malignancy as compared to the relative rate of increase in the relative rate of growth of at least one second malignancy comprising: comparing the standard deviation of the mean Replikin Count of a plurality of cells of said at least one first malignancy to the standard deviation of the mean Replikin Count of a plurality of cells of said at least one second malignancy; and predicting that the relative increase in the relative rate of growth of said at least one first malignancy is greater than the relative rate of increase in the relative rate of growth of said at least one second malignancy if the standard deviation of the mean Replikin Count of the plurality of cells of the at least one first malignancy is greater than the standard deviation of the mean Replikin Count of a plurality of cells of the at least one second malignancy, as further defined in claim 15.

In a non-limiting embodiment of the second aspect of the present invention, the at least one first malignancy may be a metastasis of the at least one second malignancy or the at least one first malignancy, or may be a malignancy of unknown origin or unknown type in a subject suffering from the at least one second malignancy. In another non-limiting embodiment, the at least one first malignancy and the at least one second malignancy are both metastases of a third malignancy. In another non-limiting embodiment, the at least one first malignancy is a plurality of malignancies differing from the at least one second malignancy, or the at least one second malignancy is a plurality of malignancies differing from the at least one first malignancy, or the at least one first malignancy and the at least one second malignancy are both a plurality of malignancies differing one from the other. In another non-limiting embodiment, the at least one first malignancy is a plurality of malignancies of a first type of malignancy and the at least one second malignancy is a plurality of malignancies of a second type of malignancy.

Another non-limiting aspect of the present invention provides a method of predicting an expansion of a malignancy comprising: determining a mean Replikin Count and a standard deviation of said mean Replikin Count for a plurality of cells of a first malignancy for a first time period in a first anatomic region or for a first plurality of malignancies including said first malignancy of claim 1; determining a Replikin Count of said second malignancy of claim 1 wherein said Replikin Count for said second malignancy is defined as a Replikin Count of at least one other cell, said at least one other cell being distinct from the plurality of cells and being at least one cell of the first malignancy, of a metastasis of the first malignancy, of a malignancy of unknown origin or unknown type in a subject suffering from the first malignancy at a second time period and/or in a second anatomic region, wherein said second time period is different from said first time period and/or said second anatomic region is different from said first anatomic region, or of a second malignancy in a different subject; and predicting an expansion of said first malignancy, said metastasis, said malignancy of unknown origin or unknown type, or said second malignancy in a different subject if the Replikin Count of the at least one other cell is greater than the mean Replikin Count of the plurality of cells plus one standard deviation of the mean Replikin Count of the plurality of cells, or if the Replikin Count of said at least one other cell is greater than the mean Replikin Count of the first plurality of malignancies plus one standard deviation of the mean.

In a non-limiting embodiment of the present invention, the at least one other cell is a second plurality of cells from the second time period and/or the second anatomic region or said second malignancy in a different subject, and the Replikin Count of each cell of the plurality of cells from the second time period and/or second anatomic region or said second malignancy in a different subject is compared separately to the mean Replikin Count plus one standard deviation of said mean Replikin Count. In a further non-limiting embodiment, the expansion of the first malignancy in the second time period and/or the second anatomic region or the expansion of said second malignancy in a different subject is predicted if the number of Replikin Counts of the second plurality of cells that is greater than the mean Replikin Count of the first plurality of cells plus one standard deviation of the mean of the Replikin Count is greater than the number of Replikin Counts of the second plurality of cells that is less than the mean Replikin Count of the first plurality of cells or the first plurality of malignancies minus one standard deviation of the mean.

Another non-limiting aspect of the present invention contemplates a kit for providing a prognosis of a patient suffering from a malignancy, said prognosis concerning the lethality of the malignancy, as defined in claim 23.

Another non-limiting aspect of the present invention provides a method of claim 1 further comprising: determining a Replikin Count of a first malignancy performing a regression analysis with the Replikin Count of a plurality of second malignancies, including said second malignancy of claim 1, versus the lethality of the plurality of second malignancies; and predicting the lethality of the first malignancy based on the regression analysis of the plurality of second malignancies. In a non-limiting embodiment of the eleventh aspect of the invention, the regression analysis of the plurality of second malignancies is performed using the Replikin Count of each individual second malignancy of the plurality of second malignancies and a patient survival outcome for each individual second malignancy. In another non-limiting embodiment, the patient survival outcome is a length of survival of patient following diagnosis of the second malignancy. In another non-limiting embodiment, the method is performed by a processor wherein at least one representation of the prediction of lethality is outputted to a user or display.

Another non-limiting embodiment of the eleventh aspect of the invention provides a machine-readable storage medium having stored thereon executable instructions that, when executed by a processor, performs the method of claim 1.

### BRIEF DESCRIPTION OF THE DRAWINGS

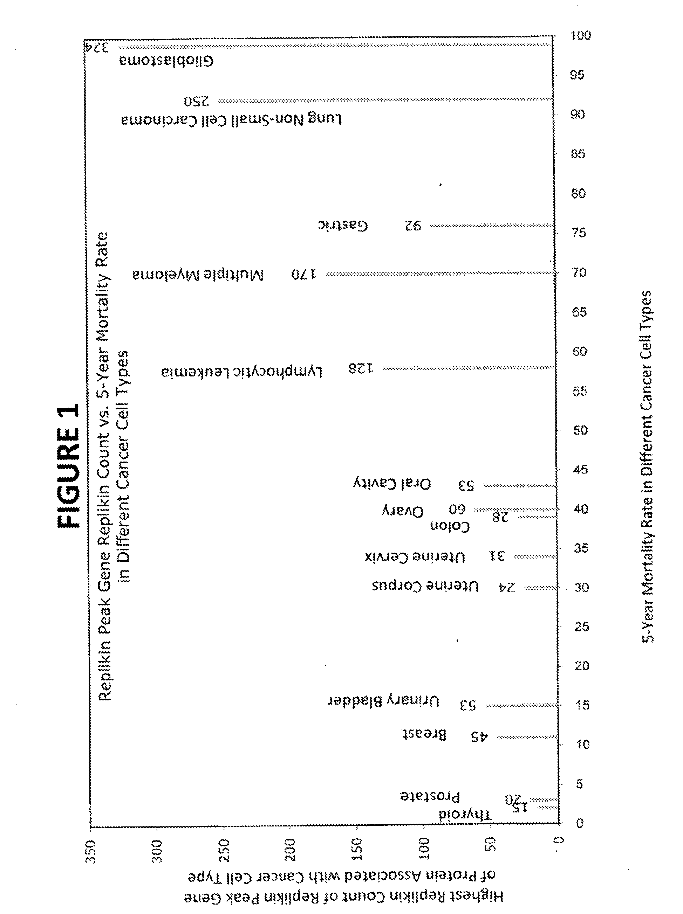

Figure 1 illustrates a quantitative relationship between the concentration of Replikin peptides in the Replikin Peak Gene of individual proteins associated with cancer cells of a plurality of common human malignancies and five-year mortality rates for each of the plurality of common human malignancies. Replikin Count was determined from the highest Replikin Count identified in a Replikin Peak Gene of sequences surveyed at www.pubmed.com. The five-year mortality rates are as reported in Brenner, H., "Long-term survival rates of cancer patients achieved by the end of the 20th century: a period analysis," The Lancet, 360 (October 12, 2002), 1131-1135. The lowest Replikin concentrations are seen in thyroid cancer (15 Replikin sequences per 100 amino acids) and in prostate cancer (20 Replikin sequences per 100 amino acids) and the lowest five-year mortality rates are seen in thyroid cancer (2%) and prostate cancer (3%). The highest Replikin concentrations are seen in non-small cell lung carcinoma (250 Replikin sequences per 100 amino acids) and in glioblastoma (324 Replikin sequences per 100 amino acids) and the highest five-year mortality rates are seen in non-small cell lung carcinoma (92%) and glioblastoma (99%). These data illustrate a relationship between Replikin concentration in a given type of cancer and lethality in that type of cancer as compared to the Replikin concentration and lethality in other types of cancer.

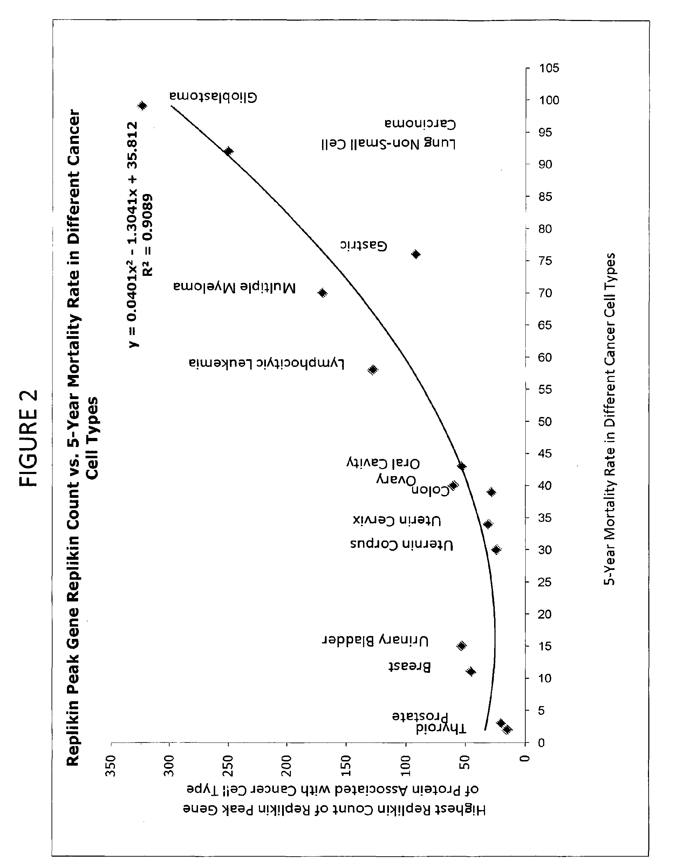

Figure 2 illustrates polynomial regression analysis of the data presented in Figure 1 and in Table 1. The regression formula provided by the regression analysis is y =0.0401x² - 1.3041 x + 35.812 with an r² value of 0.9089.

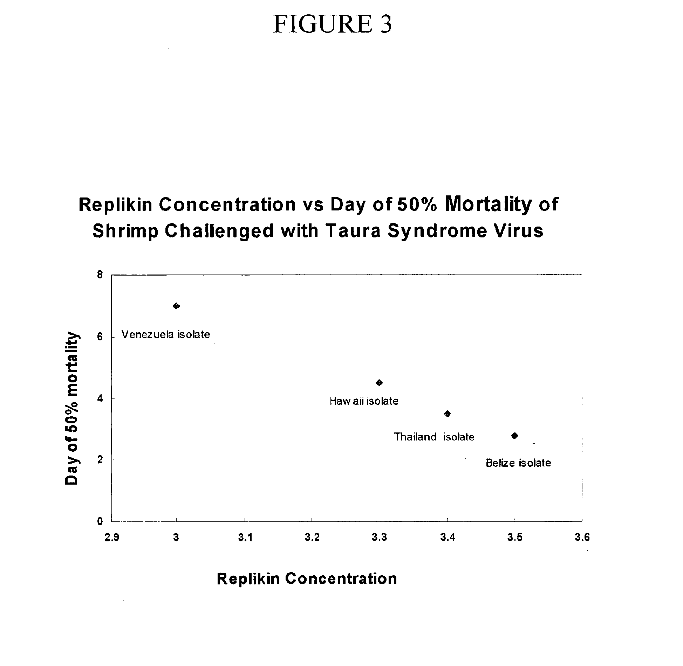

- Figure 3 illustrates a direct sequential correlation between Replikin concentration of isolates of taura syndrome virus (TSV) collected from Belize, Thailand, Hawaii and Venezuela, respectively, and mean number of days to 50% mortality in *Litopenaeus vannamei* shrimp challenged with the respective TSV isolates. Statistical differences between the Replikin concentration for each isolate are significant at a level of p<0.001. The data illustrated in Figure 3 are described in Example 7 below.

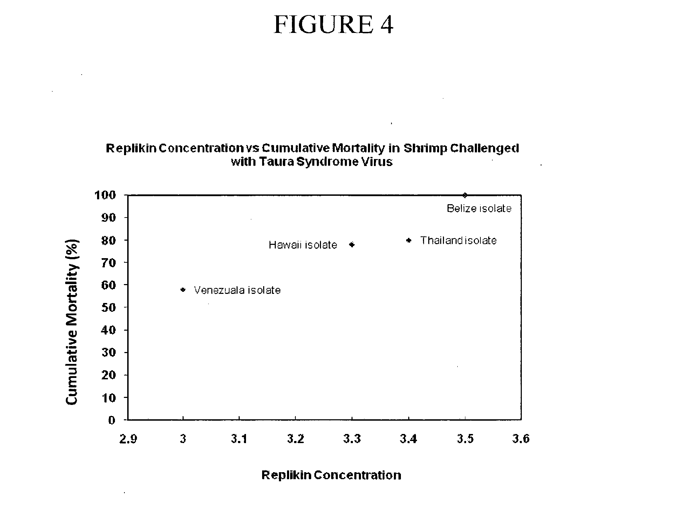

Figure 4 illustrates a direct correlation between Replikin concentration in isolates oftaura syndrome virus (TSV) collected from Belize, Thailand, Hawaii and Venezuela, respectively, and mean cumulative survival of *Litopenaeus vannamei* shrimp at 15 days after challenge with respective TSV isolates. Statistical differences between the Replikin concentrations for each isolate are significant at a level of p<0.001. The data illustrated in Figure 4 are described in Example 7 below.

### DETAILED DESCRIPTION OF THE INVENTION

### Definitions

As used herein, a "Replikin Peak Gene (RPG)" means a segment of a genome, protein, segment of protein, or protein fragment in which an expressed gene or gene segment has a highest concentration of continuous, non-interrupted and/or overlapping Replikin sequences (number of Replikin sequences per 100 amino acids) when compared to other segments or named genes of the genome. Generally, a whole protein or gene or gene segment that contains the amino acid portion (or expressed amino acid portion) having the highest concentration of continuous Replikin sequences is also referred to as the Replikin Peak Gene. More than one RPG may be identified within a gene, gene segment, protein, or protein fragment. An RPG may have a terminal lysine or a terminal histidine, two terminal lysines, or a terminal lysine and a terminal histidine. For diagnostic, therapeutic and preventive purposes, an RPG may have a terminal lysine or a terminal histidine, two terminal lysines, or a terminal lysine and a terminal histidine, or may have neither a terminal lysine nor a terminal histidine so long as the terminal portion of the RPG contains a Replikin sequence or Replikin sequences defined by the definition of a Replikin sequence, namely, an amino acid sequence having about 7 to about 50 amino acids comprising:
(1) at least one lysine residue located six to ten amino acid residues from a second lysine residue;
(2) at least one histidine residue; and
(3) at least 6% lysine residues.
Further, for diagnostic, therapeutic, preventive and predictive purposes, an RPG may include the protein or protein fragment that contains an identified RPG. For predictive purposes, a Replikin Count in the RPG may be used to identify relative rates of replication and/or lethality. Likewise, the RPG may be used as an immunogenic compound or as a vaccine. Whole proteins or protein fragments containing RPGs are likewise useful for diagnostic, therapeutic and preventive purposes, such as, for example, to be included in immunogenic compounds, vaccines and for production of therapeutic or diagnostic antibodies.

As used herein, a "Replikin sequence" is an amino acid sequence of 7 to about 50 amino acids comprising or consisting of a Replikin motif wherein the Replikin motif comprises:
(1) at least one lysine residue located at a first terminus of said isolated peptide and at least one lysine residue or at least one histidine residue located at a second terminus of said isolated peptide;
(2) a first lysine residue located six to ten residues from a second lysine residue;
(3) at least one histidine residue; and
(4) at least 6% lysine residues.
For the purpose of determining Replikin concentration, a Replikin sequence must have a lysine residue at one terminus and a lysine or a histidine residue at the other terminus. For diagnostic, therapeutic, and preventive purposes, a Replikin sequence may or may not have defined termini.

The term "Replikin sequence" can also refer to a nucleic acid sequence encoding an amino acid sequence having about 7 to about 50 amino acids comprising:
(1) at least one lysine residue located six to ten amino acid residues from a second lysine residue;
(2) at least one histidine residue; and
(3) at least 6% lysine residues,
wherein the amino acid sequence may comprise a terminal lysine and may further comprise a terminal lysine or a terminal histidine.

As used herein, the term "peptide" or "protein" refers to a compound of two or more amino acids in which the carboxyl group of one amino acid is attached to an amino group of another amino acid via a peptide bond. As used herein, "isolated" or "synthesized" peptide or protein or biologically active portion of a peptide or protein refers to a peptide that is, after purification, substantially free of cellular material or other contaminating proteins or peptides from the cell or tissue source from which the peptide is derived, or substantially free from chemical precursors or other chemicals when chemically synthesized by any method, or substantially free from contaminating peptides when synthesized by recombinant gene techniques or a protein or peptide that has been isolated *in silico* from nucleic acid or amino acid sequences that are available through public or private databases or sequence collections. An "encoded" or "expressed" protein, protein sequence, protein fragment sequence, or peptide sequence is a sequence encoded by a nucleic acid sequence that encodes the amino acids of the protein or peptide sequence with any codon known to one of ordinary skill in the art now or hereafter. It should be noted that it is well-known in the art that, due to redundancy in the genetic code, individual nucleotides can be readily exchanged in a codon and still result in an identical amino acid sequence. As will be understood by one of skill in the art, a method of identifying a Replikin amino acid sequence also encompasses a method of identifying a nucleic acid sequence that encodes a Replikin amino acid sequence wherein the Replikin amino acid sequence is encoded by the identified nucleic acid sequence.

As used herein, "Replikin Count" or "Replikin Concentration" refers to the number of Replikins per 100 amino acids in a protein, protein fragment, or genome of a cell or virus.

As used herein, the term "continuous Replikin sequences" means a series of two or more Replikin sequences that are overlapped or are directly covalently linked or are both overlapped and directly covalently linked.

As used herein, the term cancer "type" refers to malignancies that share histology or origin. One of ordinary skill in the art knows how to separate different malignancies by cancer "type." Malignancies subject to aspects of the invention may be of the same cancer type or of different cancer types. The malignancies may also be of unknown type or may be metastatic and of known or unknown type. Many cancers histologically diagnosed in a primary malignancy are of unknown cancer type such as when a metastasis that is being examined has changed and has become difficult or impossible to type by histological methods. In such cases, the present methods of prediction are of use as an independent method of predicting the relative rate of replication and lethality of unknown and metastatic cancers. The methods of prediction of relative replication rate and/or lethality disclosed herein provide a tool for predicting the relative rate of growth, relative replication rate, and/or relative lethality of malignancies that are of unknown type or of unknown histological origin and/or are metastatic using the Replikin Count of any malignancy whether of known or unknown cancer type.

As used herein, "homologous" or "homology" or "sequence identity" are used to indicate that a nucleic acid sequence or amino acid sequence exhibits substantial structural or functional equivalence with another sequence. Any structural or functional differences between sequences having sequence identity or homology will be *de minimus;* that is, they will not affect the ability of the sequence to function as indicated in the desired application. Differences may be due to inherent variations in codon usage among different species, for example. Structural differences are considered *de minimus* if there is a significant amount of sequence overlap or similarity between two or more different sequences or if the different sequences exhibit similar physical characteristics even if the sequences differ in length or structure. Such characteristics include, for example, the ability to hybridize under defined conditions, or in the case of proteins, immunological crossreactivity, similar enzymatic activity, etc. The skilled practitioner can readily determine each of these characteristics by art known methods.

To determine the percent identity or percent homology of two sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, at least 30%, 40%, 50%, 60%, 70%, 80%, or 90% or more of the length of a reference sequence is aligned for comparison purposes. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity and similarity between two sequences can be accomplished using a mathematical algorithm. (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991).

The nucleic acid and protein sequences of the present invention can further be used as a "query sequence" to perform a search against sequence databases to, for example, identify other family members or related sequences. Such searches can be performed using the NBLAST and XBLAST programs (version 2.0) of Altschul et al. (1990) J. Mol. Biol. 215:403-10. BLAST nucleotide searches can be performed with the NBLAST program. BLAST protein searches can be performed with the XBLAST program to obtain amino acid sequences homologous to the proteins of the invention. To obtain gapped alignments for comparison purposes, Gapped BLAST can be utilized as described in Altschul et al. (1997) Nucleic Acids Res. 25(17):3389-3402. When utilizing BLAST and gapped BLAST programs, the default parameters of the respective programs (e.g., XBLAST and NBLAST) can be used.

As used herein a "vaccine" is any substance, compound, composition, mixture, or other therapeutic substance that, when administered to a human or animal via any method of administration known to the skilled artisan now or hereafter, produces an immune response, a humoral response, an antibody response, or a protective effect in the human or animal.

As used herein, the terms, "growth," "replication," "aggressiveness," and "lethality" are interchangeable for the purposes of providing a prediction of the behavior of a malignancy. The rate of growth, rate of replication, aggressiveness, or lethality of a malignancy, for the purposes of providing predictions based on Replikin concentrations, may be considered to correlate.

### Relative Rate of Growth, Rate of Replication, and Lethality among Cancer Cells

An embodiment of predictive aspects of the present invention provides methods of predicting the relative rate of growth, the relative rate of replication, and/or the relative lethality of a first malignancy as compared to a second malignancy or as compared to a plurality of malignancies comprising: comparing a Replikin Count in the first malignancy with a Replikin Count in the second malignancy or a mean, median, mode (or other average), range, or other aggregating or segregating measure of Replikin Count in a plurality of malignancies. The present invention further provides methods of predicting relative rate of growth, relative rate or replication, and/or relative lethality in a primary malignancy or metastatic malignancy, whether such primary malignancy or metastatic malignancy is of known or unknown type or origin. The Replikin Count is a measure of the Replikin concentration of a malignancy and is the number of Replikin sequences identified in the genome of the malignancy or in a segment of the genome of the malignancy or identified in a protein or protein fragment of the malignancy per 100 encoded or expressed amino acids. A malignancy having a higher Replikin concentration has been demonstrated to have a relatively higher rate of replication or growth and relatively greater lethality. *See* Figure 1. Likewise, a malignancy having a lower Replikin concentration has been demonstrated to have a relatively lower rate of replication or growth and relatively lower lethality. *See id.*

The relative rate of growth, replication, or lethality of a malignancy may be predicted by comparing the Replikin Count of a first malignancy to the Replikin Count of a second malignancy. A cell from a first malignancy may have a higher or lower Replikin Count than a cell from a second malignancy, a higher or lower Replikin Count than a mean Replikin Count of a plurality of cells from a single malignancy, a plurality of malignancies, a plurality of cells from a single patient suffering from a malignancy (whether the malignant cells are of known or unknown histology or origin), each of the plurality of cells from a malignancy or a plurality of other malignancies, or a plurality of cells from the plurality of other malignancies. Higher Replikin Counts represent a higher rate of growth, replication, and/or greater lethality. Lower Replikin Counts represent a lower rate of growth, replication, and/or lower lethality.

A first malignancy may be predicted to have a higher rate of growth, replication, and/or a greater lethality as compared to a plurality of other malignancies if a cell from the first malignancy has a higher Replikin Count than cells from the plurality of the other malignancies. A first malignancy may likewise be predicted to have higher growth, replication, and/or lethality if a cell from the first malignancy has a higher Replikin Count than the mean of the Replikin Counts of cells from the plurality of the other malignancies or than a plurality of means of a plurality of the other malignancies.

A first malignancy may be predicted to have a lower rate of growth, replication, and/or a lower lethality as compared to a plurality of other malignancies if a cell from the first malignancy has a lower Replikin Count than cells from the plurality of the other malignancies. A first malignancy may likewise be predicted to have lower growth, replication, and/or lethality if a cell from the first malignancy has a lower Replikin Count than the mean of the Replikin Counts of cells from the plurality of the other malignancies or than a plurality of means of a plurality of the other malignancies.

In an embodiment of a predictive method of the invention, the relative rate of growth, relative rate of replication, or relative lethality of a malignancy may be determined by comparing a Replikin Count of at least one cell of a first malignancy to a Replikin Count of at least one other cell of the first malignancy. The method may apply to a metastatic cell of a first malignancy where the one other cell is a different metastatic cell of the first malignancy, is a non-metastatic cell of the first malignancy, or is a malignant cell of unknown origin within a patient suffering from the first malignancy.

The Replikin Count of a first malignancy may be compared to a plurality of malignancies or to a mean or average of the Replikin Count of a plurality of malignancies. The Replikin Count of a first malignancy may also be compared to a plurality of means and/or averages of the Replikin Count of a plurality of malignancies.

A mean, median, mode (or other average), range, or other aggregating measure of Replikin Count may be used when analyzing a plurality of Replikin Counts. Likewise, any other tool known to one of skill in the art now and hereafter may be used to aggregate or segregate the Replikin Counts of particular malignancies or particular cells within a malignancy.

The rate of growth, rate of replication, and/or lethality of a malignancy or of a type of malignancy may be determined by any method known to one of skill in the art now or hereafter. A rate of growth may be determined by, for example, the rate of increase in volume of a tumor (or any other measure of size including diameter, circumference, *etc*.), the rate of increase in mass of a tumor, the rate of spread of a tumor, the rate of metastasis of tumor, *etc.* A rate of replication may be determined by, for example, any measure of the rate of growth as well as any measure of the rate of increase in number of cells from a cell, or tumor, or mass of a malignancy. A rate of lethality may be determined, for example, by any measure of growth, or replication, or any measure of metastasis of a malignancy, life expectancy with a malignancy, invasiveness of a malignancy, or rate of death of a subject suffering from a malignancy. One measure of lethality, among many other possible measures, is the five year mortality rate.

### Computer Methods for Determining Relative Rate of Growth, Rate of Replication, and Lethality among Cancer Cells

A prediction of the relative rate of growth, replication, and/or lethality of a malignancy may be performed by a processor. A prediction may be output to a user or display. Likewise, a particular Replikin peptide or Replikin Peak Gene within a malignancy predicted to have a higher rate of growth, replication or lethality may be output to a user or display. A machine-readable storage medium may contain executable instructions that, when executed by a processor, cause the processor to provide sufficient data to a user, a printout, or a display such that the user or a user of the printout or display may predict the relative rate of growth, replication, or lethality of a malignancy. A process for predicting a relative rate of growth may comprise: comparing a Replikin Count of at least one first malignancy with a Replikin Count of at least one second malignancy; and predicting the first malignancy to have a relative rate of growth that is faster than the relative rate of growth of the second malignancy if the Replikin Count of the first malignancy is greater than the Replikin Count of the second malignancy.

A computer system may include a processor coupled to a network, and a memory coupled to a processor, wherein the memory contains a plurality of instruction to perform the methods of prediction discussed herein.

A user of outputted data from a processor, storage medium, machine-readable medium, or computer system may include any person or any machine that records or analyzes the outputted data. A display or printout may include any mechanism by which data is outputted so that any person or any machine may record or analyze the outputted data, including a printed document, a visual impulse, an aural impulse, or any other perceivable impulse, a computer monitor, a set of numbers, or any other display or printout of data including a digital recording medium.

Outputs of data may include an output of a prediction of the relative growth rate, replication rate or lethality of a malignancy, a plurality of malignancies, or a cell or plurality of cells of a malignancy. Outputs of data may also include a Replikin Count or a number or numbers sufficient for a user to determine a Replikin Count, any portion or component of a cell of a malignancy or of a malignancy identified by a processor as predicted to have a higher or lower rate of growth or replication or a higher or lower lethality, including a Replikin peptide or a Replikin Peak Gene of a malignancy, or any information that will assist a user in providing a prediction concerning the relative rate of growth, replication, and/or lethality of a malignancy or that will assist a user in providing any portion or component of a cell or a malignancy identified by a processor as predicted to have a higher or lower rate of growth or replication or a higher or lower lethality.

A representation of a prediction of the relative rate of growth of a malignancy is outputted to a user or to a display when any information is outputted to a user or display that provides sufficient information for the user or a machine receiving the output or display to determine or provide a prediction concerning the relative rate of growth, replication, or lethality of a malignancy, a plurality of malignancies, a cell of a malignancy, or a plurality of cells of a malignancy. Sufficient information to provide a prediction includes but is not limited to a Replikin Count, a prediction, a number or symbol or image that signifies a prediction, a listing of a cell, malignancy or symbol of a cell or malignancy that is predicted to have a lower or higher rate of growth, replication or lethality, or any other piece of information or collection of information to provide a user or display sufficient information to determine or provide a prediction concerning the relative rate of growth, replication, or lethality of a malignancy, a plurality of malignancies, a cell of a malignancy, or a plurality of cells of a malignancy.

### Predictions May Be Made by Comparing Cells from Cancer Types that are the Same or Different in Histology or Origin, including Unknown Cancer Types

Malignancies subject to aspects of the invention may be of the same cancer type or of different cancer types where cancer types may be determined by histology, tissue origin, genetic makeup or anomaly, biochemical makeup or structure, metastatic origin from a primary, secondary, tertiary (or other) malignancy, by time or progression of a malignancy, by anatomic position of a malignancy, or by any other method known to one of skill in the art now or hereafter whereby the skilled artisan may differentiate cancer type. Malignancies may also be of unknown type or origin or may be metastatic and of known or unknown type or origin.

The methods of prediction of relative growth, replication, and/or lethality disclosed herein provide a tool for predicting the relative rate of growth, relative rate of replication, and/or relative lethality of malignancies that are of unknown type or of unknown histological origin and/or are metastatic using the Replikin Count of any malignancy whether of known or unknown cancer type. Many cancers histologically diagnosed in a primary malignancy are of unknown cancer type such as when a metastasis that is being examined has changed and has become difficult or impossible to type by histological methods. In such cases, the present methods of prediction are of use as an independent method of predicting the relative rate of replication and lethality of unknown and metastatic cancers.

An embodiment of the present invention provides a method of predicting the relative rate of growth of at least one first malignancy of an unknown or known type of malignancy comprising: comparing a Replikin Count of the at least one first malignancy with a Replikin Count of at least one second malignancy of an unknown type or of a different type of malignancy than the at least one first malignancy; and predicting the at least one first malignancy has a relative rate of growth that is faster than the relative rate of growth of the at least one second malignancy if the Replikin Count of the at least one first malignancy is greater than the Replikin Count of the at least one second malignancy. A further embodiment of the present invention provides for a determination of the Replikin Count of the at least one first malignancy and a Replikin Count of the at least one second malignancy. The Replikin Count of a malignancy may be determined in any portion of the genome or in any expressed protein or protein fragment of the malignancy. Comparison of the Replikin Count of two malignancies may be made between whole genomes of the malignancies, genome fragments of the malignancies, or expressed proteins or protein fragments of the malignancies. When Replikin Counts of individual cells or mean Replikin Counts of pluralities of cells are compared, comparable fragments of the genome or comparable expressed proteins or expressed protein fragments of the cells may be compared. For example, the Replikin Count of a particular expressed protein or of a Replikin Peak Gene of a particular expressed protein or protein fragment in the individual cells may be compared.

The relative growth or replication rate or relative lethality of a malignancy may be determined in any malignant cell, malignant tissue or tumor, or any malignancy in a patient (human or animal) suffering from a malignancy including a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, a glioblastoma malignancy, or any other kind of malignancy.

Any Replikin Count in individual cells may be compared. Over a mean Replikin Count, Replikin Counts of different sections of the genome or different expressed proteins or protein fragments of individual cells may be compared. With respect to mean Replikin Counts determined from a plurality of cells, any available information providing sequences of the genome or sequences of proteins or expressed protein fragments of a malignancy or malignancies may be compared. Such information may include sequencing of the genome of a malignancy, cDNA sequencing of a malignancy, sequencing of mRNAs of a malignancy or any other nucleic acid sequence of a malignancy. Such information may also include sequencing of expressed proteins, protein fragments, or peptides of a malignancy. Such information may further include *in silico* information providing nucleic acid or amino acid sequences of a malignancy or malignancies including research data, published journals, databases that contain sequence information such as those found at www.pubmed.com, or any other source of sequence information.

In comparing Replikin Counts between individual cells of a malignancy or cells from different malignancies or cells from a primary malignancy and metastatic cells from the same malignancy or unknown cancer cells in a patient suffering from a primary malignancy, a Replikin Count may be a mean Replikin Count of a plurality of cells of a malignancy, a mean Replikin Count of a plurality of cells of different malignancies, a mean Replikin Count of a plurality of cells from a primary malignancy and metastases of the malignancy, or unknown cancer cells from a patient suffering from a malignancy. A Replikin Count may also represent only a Replikin Count from a single cell in any one of the malignancies discussed above and herein. One of ordinary skill in the art would understand how to compare the Replikin Count of individual cells to the mean Replikin Count of a plurality of cells or mean Replikin Counts between different groupings of pluralities of cells. In an embodiment of a predictive method of the invention, a mean Replikin Count of a plurality of malignancies or a plurality of cells of a malignancy may be compared to a Replikin Count of single cell of a malignancy or a mean Replikin Count of a plurality of cells of various malignancies (including, for example, metastases of a primary malignancy) or the same malignancy may be compared to a mean Replikin Count of a plurality of cells of various malignancies (including, for example, metastases of a primary malignancy) or the same malignancy.

A plurality of malignancies may represent a survey of malignancies of a particular type of cancer. A plurality may also represent a survey of malignancies of unknown types of cancer. A plurality may also represent a survey of malignancies of all types of cancer. Such surveys provide baseline data for Replikin Counts and standard deviations from Replikin Counts among malignancies of known, unknown, and all types of cancer. The Replikin Counts with standard deviations may then be compared to a Replikin Count from a specific malignancy. If the Replikin Count of a specific malignancy is greater or less than that of a survey of cancers of the same type, the specific malignancy may be predicted to have a higher or lower rate of growth, rate of replication, or lethality, respectively, than the mean, median, or other average rates of growth, replication or lethality of the type of cancer. Likewise if the Replikin Count of a specific malignancy is greater or less than that of a survey of cancers of unknown type, and the specific malignancy is of known or unknown type, the specific malignancy may be predicted to have a higher or lower rate of growth, rate of replication, or lethality, respectively, than the mean, average, or median rates of growth, replication or lethality of the unknown types of cancer. Further, if the Replikin Count of a specific malignancy is greater or less than that of a survey of cancers of all types, the specific malignancy may be predicted to have a higher or lower rate of growth, rate of replication, or lethality, respectively, than the mean, median, or other average rates of growth, replication or lethality of all types of cancer included in the survey.

A survey may represent data collected from any number of malignancies from one to as many malignancies as may be included in the data set of survey. A survey may collect data on Replikin Count as determined in any portion of the genome of cells of a malignancy or any portion of the expressed proteins and protein fragments of the cells of a malignancy. A survey may collect data on rate of growth or replication of a malignancy, rate of expansion of volume or other measure of size of a tumor of a malignancy, rate of increase in mass of a tumor of a malignancy, lethality of a malignancy including time to death or percent mortality at a specific time of a malignancy. A survey may likewise collect any data understood now or hereafter by one of skill in the art to reflect on Replikin sequences present in a malignancy and/or rates of growth, replication, and/or lethality of a malignancy.

One aspect of a survey provides baseline data against which a Replikin Count of an individual malignancy or a plurality of malignancies may be compared to predict the rate of growth, replication, or lethality of a malignancy or group of malignancies.

The relative rate of growth of a cell or a tissue in which a Replikin Count (or a plurality of Replikin Counts) has been determined may be compared to the relative rate of replication of the cell or tissue. As is understood by one of ordinary skill in the art, the relative rate of growth and the relative rate of replication of a malignancy may also be correlated with the relative lethality of a malignancy. As such, an embodiment of a method of prediction of the present invention provides methods of predicting or determining the relative rate of growth of a malignancy, the relative rate of replication of a malignancy, or the relative lethality of a malignancy.

In a further embodiment, the first malignancy may be a primary malignancy and the relative rate of growth, the relative rate of replication, and/or the relative lethality of the primary malignancy is compared to any said second malignancy. In a further embodiment, the first malignancy may be a primary malignancy and the second malignancy may be a metastatic malignancy. In a further embodiment, the primary malignancy and the metastatic malignancy (or two metastatic malignancies) may be compared to yet another malignancy or plurality of malignancies.

A method of predicting the relative replication rate or relative lethality of a malignancy may predict the relative replication rate or relative lethality of any malignancy or group of malignancies of a certain cancer type or any malignancy or group of malignancies of an unknown type so long as the individuals of the group of malignancies share similar, or otherwise aggregatable or segregatable Replikin concentrations in their genome, in a segment of their genome, in a protein or proteins, or in a fragment or fragments of a protein or a plurality of their proteins. A method of predicting may be applied to a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, a glioblastoma malignancy, or any human or animal malignancy wherein the Replikin Count of the malignancy is known or may be known or may be established by methods herein described.

### Replikin Counts in Replikin Peak Genes Predict Relative Rates of Growth and Relative Lethality

Replikin concentration in a malignancy may be determined in the Replikin Peak Gene of a malignancy. The Replikin Peak Gene of a malignancy is the segment of a genome, protein, or protein fragment of a malignancy that has a highest concentration of continuous, non-interrupted and/or overlapping Replikin sequences as compared to other segments of the genome of the malignancy or as compared to expressed proteins or protein fragments of the malignancy.

When comparing the Replikin Count of a first malignancy to the Replikin Count of a second malignancy, the Replikin Count of a Replikin Peak Gene of the first malignancy may be compared to the Replikin Count of a Replikin Peak Gene of the second malignancy. The Replikin Peak Gene may be a genome segment, a protein, or a protein fragment. The Replikin Count of a first malignancy may also be compared to a plurality of malignancies or to a mean or average of the Replikin Count of a plurality of malignancies. The Replikin Count of a first malignancy may also be compared to a plurality of means or other averages or methods of aggregation or segregation of the Replikin Count of a plurality of malignancies.

A prediction of the relative rate of growth, replication, or lethality of a malignancy may be performed by a processor. A prediction may be output to a user or display. Likewise, a particular Replikin peptide or Replikin Peak Gene within a malignancy that is predicted to have a higher rate of growth, replication or lethality may be output to a user or display.

An embodiment of a predictive method of the present invention provides a method of predicting the relative rate of growth, relative rate of replication, or the relative lethality of a first malignancy as compared to a second malignancy or as compared to a plurality of other malignancies comprising (1) identifying some or all Replikin sequences in the genome, in a protein, or in a protein fragment of a first malignancy, (2) identifying some or all Replikin sequences in the genome, in a protein, or in a protein fragment of the second malignancy or in the genome, in a protein, or in a protein fragment of each of a plurality of other malignancies, (3) determining a Replikin Count of the segment of the genome, of the protein, or of the protein fragment of the first malignancy that has the highest concentration of continuous non-interrupted and/or overlapping Replikin sequences when compared to other segments of the genome, proteins, or protein fragments of the first malignancy, (4) determining a Replikin Count of the segment of the genome, of the protein, or of the protein fragment of the second malignancy or of each of the plurality of other malignancies that has or have the highest concentration of continuous non-interrupted and/or overlapping Replikin sequences when compared to other segments of the genome, proteins, or protein fragments of the second malignancy or of each of the plurality of other malignancies, (5) identifying the first malignancy as having a higher Replikin Count than the second malignancy or than each of the plurality of other malignancies, and (6) predicting the first malignancy to have a relatively higher rate of replication or a relatively greater lethality than the second malignancy or than the plurality of other malignancies.

### Methods of Comparing Relative Lethality of Cancer Cells, Tissues, or Types

Described herewith is a method of predicting the relative replication and/or relative lethality of a first type of malignancy comprising (1) analyzing publicly available nucleotide or amino acid sequences of at least a first type and a second type of malignancy published at www.pubmed.com, (2) determining the Replikin Count for each accession number with a nucleotide or amino acid sequence isolated from the first and at least the second type of malignancy, (3) determining the mean Replikin Count for all data for the first type and at least the second type of malignancy in a given year for which accession numbers are available, (4) selecting at least one year in which the mean Replikin Count for the first malignancy is notably higher, preferably statistically higher, than other years, (5) selecting at least one year in which the mean Replikin Count for at least the second malignancy is notably higher, preferably statistically higher, than other years, (6) identifying the Replikin Peak Gene of each published sequence in the selected year or years for the first malignancy and identifying the Replikin Peak Gene of each published sequence in the selected year or years for the second malignancy, (7) determining the concentration of Replikin sequences for each identified Replikin Peak Gene, (8) selecting the highest Replikin Count as the representative highest Replikin Count for the first malignancy and for at least the second malignancy, and (9) predicting the first malignancy to have a higher growth rate, replication rate and/or greater lethality than at least the second malignancy if the selected highest Replikin Count of the first malignancy is greater than the highest Replikin Count of at least the second malignancy or predicting the first malignancy to have a lower growth rate, replication rate and/or less lethality than at least the second malignancy if the selected highest Replikin Count of the first malignancy is lower than the highest Replikin Count of at least the second malignancy. In a further embodiment, the predicted lethality of the first and at least second malignancies is a five-year mortality rate.

Figure 1 illustrates a quantitative relationship between the concentration of Replikin peptides in the Replikin Peak Gene of individual proteins associated with cancer cells of different common human malignancies and five-year mortality rates of the different common human malignancies. Replikin Count was determined from the highest Replikin Count identified in a Replikin Peak Gene of sequences surveyed at www.pubmed.com after initially selecting for further analysis (1) those years in which the mean Replikin Counts were the highest, and from those years, (2) those sequences having the highest mean Replikin Count among all available sequences. The five-year mortality rates are as reported in Brenner, H., "Long-term survival rates of cancer patients achieved by the end of the 20th century: a period analysis," The Lancet, 360 (October 12, 2002), 1131-1135. The lowest Replikin concentrations are seen in thyroid cancer (15 Replikin sequences per 100 amino acids) and in prostate cancer (20 Replikin sequences per 100 amino acids) and the lowest five-year mortality rates are seen in thyroid cancer (2%) and prostate cancer (3%). The highest Replikin concentrations are seen in non-small cell lung carcinoma (250 Replikin sequences per 100 amino acids) and in glioblastoma (324 Replikin sequences per 100 amino acids) and the highest five-year mortality rates are seen in non-small cell lung carcinoma (92%) and glioblastoma (99%). A quantitative progression is seen in relative lethality of each cancer type as compared to the analyzed Replikin Count of each cancer type.

The data for gastric cancer in Figure 1 demonstrate a higher mortality rate than would be expected from Replikin Count alone as compared to other malignancies. This higher-than-expected mortality rate may be due to a world-wide recognized problem of late detection in gastric cancer. Likewise, the data for urinary bladder cancer and breast cancer in Figure 1 demonstrate a lower mortality rate than would be expected from Replikin Count alone as compared to other malignancies. The lower-than-expected mortality rate for urinary bladder may be due to art-recognized early detection in bladder cancer. The lower-than-expected mortality rate for breast cancer may similarly be due to increased screening for breast cancer, which results in relatively earlier detection. Breast cancer mortality rate has been decreasing as screening has increased over the past decades.

The data in Table 1 below reflect the data in Figure 1 and demonstrate a quantitative relationship between Replikin Count in the Replikin Peak Gene of the genome of common types of human cancer and five-year mortality in that cancer as reported in Brenner, H., "Long-term survival rates of cancer patients achieved by the end of the 20th century: a period analysis," The Lancet, 360 (October 12, 2002), 1131-1135. The discovery of the relation of Replikin sequences to rapid replication as reflected in Table 1 offers a new approach and provides means to inhibit rapid replication and resulting lethality in cancers in animals and humans.

**Table 1**

| **Human Cancer Type** | **5-Year Mortality of Human Cancer Type** | **Highest Replikin Count of Replikin Peak Gene** |
|---|---|---|
| Thyroid | 2 | 15 |
| Prostate | 3 | 20 |
| Breast | 11 | 45 |
| Urinary Bladder | 15 | 53 |
| Uterine Corpus | 30 | 24 |
| Uterine Cervix | 34 | 31 |
| Colon | 39 | 28 |
| Ovary | 40 | 60 |
| Oral Cavity | 43 | 53 |
| Lymphocytic Leukemia | 58 | 128 |
| Multiple Myeloma | 70 | 170 |
| Gastric | 76 | 92 |
| Non-Small Cell Lung Carcinoma | 92 | 250 |
| Glioblastoma | 99 | 324 |

Overall, the data in Table 1 and Figure 1 provide an illustration of a quantitative relationship between Replikin concentration in a given type of cancer and lethality in that type of cancer as compared to the Replikin concentration and lethality in other types of cancer. The data in Figure 1 also provide further support for a general association between Replikin concentration and lethality within a particular type of cancer (such as, for example, lung cancer) as described in U.S. Patent Appln. Ser. No. 12/010,027, filed January 18, 2008.

The association seen in Figure 1 is surprising to one of ordinary skill in the art because the Replikin Count in these disparate human malignancies is quantitatively related to mean five-year mortality of sufferers of the malignancies even though the malignancies would be expected to have notable differences in disease progression and even though mortality outcomes are significantly dependent upon multiple variables including time of detection and efficacy of disparate treatments. Despite the expected significant differences in time of detection and efficacy of treatment across the population surveyed by Brenner (Lancet 2002), the Replikin concentration in these different human malignancies emerges as a significant variable that quantitatively relates to the mean mortalities reported therein.

### Standard Deviation Measures Change in Rate of Growth

When a mean Replikin Count is determined within a plurality of cells of a malignancy, a larger standard deviation in the mean demonstrates that Replikin Count is changing within the malignancy. These changes within the malignancy point to an increase in the relative rate of growth by demonstrating that certain cells within the malignancy have a relatively higher rate of replication and will be expected to increase the overall rate of growth of the malignancy.

An embodiment of a predictive method of the present invention provides a method of predicting the relative rate of growth of a malignancy comprising: determining the standard deviation of the mean Replikin Count of a plurality of cells of a first malignancy, of a plurality of cells of a metastatic malignancy or a plurality of metastatic malignancies from a patient suffering from a first malignancy, or of a combination of cells of a primary first malignancy and a metastatic malignancy or a plurality of metastatic malignancies of the first malignancy; and determining that the standard deviation of the mean of the first malignancy is relatively larger than the standard deviation of the mean Replikin Count of at least one other malignancy or plurality of malignancies. A standard deviation may be compared between malignancies of the same type or of different types or of metastases within a single patient suffering from a malignancy or between different patients suffering from malignancy. A standard deviation may also be compared between a malignancy, a plurality of malignancies, or a plurality of cells of a malignancy to the standard deviation of a survey of malignancies of the same type, a different type, unknown type, or all types of malignancy.

The standard deviation may be considered relatively large if the standard deviation from the mean Replikin Count is 50% or more of the mean, 40% or more of the mean, 30% or more of the mean, 20% or more of the mean, 10% or more of the mean, 5% or more of the mean, 2% or more of the mean, 1% or more of the mean, 0.5% or more of the mean, or any difference from the mean understood by one of ordinary skill in the art to demonstrate that the population from which the cancer cells in which Replikin Counts have been determined has a changing concentration of Replikin peptides that points to an increase in Replikin Counts within the populations and, as a result, an increase in the rate of growth, rate of replication, or lethality of the cancer cell population.

In an embodiment, a mean Replikin Count with standard deviation may represent a plurality of metastatic cells of the first malignancy. A Replikin Count with standard deviation may also represent a plurality of different metastatic cells of the first malignancy, a plurality of non-metastatic cells of the first malignancy, or a plurality of malignant cells of unknown type from a patient suffering from the first malignancy (or any combination thereof). The Replikin Count of a single cell or a plurality of cells may be compared with a Replikin Count of a single cell or a plurality of cells. A cell or plurality of cells may be predicted to have a greater rate of growth, a greater rate of replication, or a greater lethality than another cell or plurality of cells if the Replikin Count of said cell or malignancy is greater than the mean Replikin Count of the other cell or plurality of cells plus the standard deviation of the mean.

A plurality of cells may also be predicted to have a greater increase in rate of growth, a greater increase in rate of replication, or a greater increase in lethality than another plurality of cells if the standard deviation of the mean Replikin Count of the plurality of cells is greater than the standard deviation of the mean Replikin Count of the other plurality of cells.

Mean Replikin Counts with standard deviation may be determined with a relatively small or large number of cells isolated from a malignancy, may be determined from a relatively small or large number of cells isolated from a plurality of malignancies of the same or similar type, or may be determined from a relatively small or large number of cells isolated from a plurality of cancer types. Replikin Counts with standard deviation may be established from cells isolated from malignancies of the same or similar histological types in a plurality of patients or from malignancies of different histological types in a plurality of patients. A baseline control for Replikin Count and for standard deviation may represent Replikin Counts from a broad collection of malignancies from a broad collection of patients in a particular type of cancer or in all types of cancer. Such controls provide the ordinary skilled artisan with baseline numbers for Replikin Counts and standard deviations in Replikin Counts. Surveys of malignancies are one method of providing such controls.

### Predicting Expansions of Malignancies

One aspect of the present invention provides methods of predicting expansions of malignancies using a Replikin Count Expansion Index. In one embodiment of this aspect of the invention, an expansion of a malignancy (of known or unknown type) or of a metastasis of a malignancy (of known or unknown type) is predicted by (1) determining a mean Replikin Count and a standard deviation from the mean Replikin Count for a plurality of cells of a first malignancy for a first time period in a first anatomic region, (2) determining a Replikin Count of at least one malignant cell (whether primary, metastatic, or unknown) in the body of the patient suffering from said malignancy at a second time period and/or in a second anatomic region or determining a Replikin Count in at least one malignant cell (whether primary, metastatic, or unknown) in the body of a different patient suffering from a second malignancy, and (3) predicting an expansion of the malignancy at the second time period and/or in the second anatomic region or the second malignancy in body of the different patient if the Replikin Count of the at least one malignant cell from the second time period and/or a second anatomic region or the at least one malignant cell from the body of a different patient is greater than one standard deviation of the mean of the Replikin Count of the plurality of cells from the first time period in the first anatomic region, as defined in claim 20.

In another embodiment of the aspect of the invention, an expansion of a malignancy (of known or unknown type) or of a metastasis of a malignancy (of known or unknown type) is predicted by (1) determining a mean Replikin Count and a standard deviation from the mean Replikin Count for a plurality of cells of a first malignancy or a plurality of first malignancies, (2) determining a Replikin Count of at least one malignant cell (whether primary, metastatic, or unknown) in the body of a patient suffering from a second malignancy, and (3) predicting an expansion of the second malignancy if the Replikin Count of the at least one malignant cell from the second malignancy is greater than one standard deviation of the mean of the Replikin Count of the plurality of cells of the first malignancy or the plurality of first malignancies.

In the above-described methods, at least one cell of the malignancy from a second time period and/or second anatomic region or from a second malignancy may be a plurality of cells from the second time period and/or second anatomic region or from the second malignancy. In this case, the Replikin Count of each cell of the plurality of cells from the second time period and/or second anatomic region or from the second malignancy is compared separately to one standard deviation of the mean of the Replikin Count of the plurality of cells from the first time period in the first anatomic region or from the first malignancy.

An expansion of the malignancy isolated in the second time period and/or second anatomic region or in the second malignancy may also be predicted if the number of Replikin Counts of a plurality of cells from the second period and/or second anatomic region or second malignancy that is greater than the mean Replikin Count of the first malignancy plus one standard deviation of the mean is greater than the number of Replikin Counts of said plurality of cells from the second period and/or second anatomic region or second malignancy that is less than the mean Replikin Count of the first malignancy minus one standard deviation of the mean.

An anatomic region of a malignancy or cell of a malignancy is any region of a body of a subject that suffers from a malignancy from which a malignancy, cell of a malignancy, or portion or component of a cell of a malignancy may be identified or isolated. A region may include the entire body of a subject. A region may also be limited to a general region of a body of a subject, such as the thorax, the neck, the knee, the toe, or the leg, may be limited to an organ of a subject, such as the eye, the liver, the skeleton, the blood vessels, or the pancreas of a subject, may be limited to a part of an organ of a subject, may be limited to a particular tissue of a subject, or may be limited to any region of the subject understood by one of skill in the art to provide helpful information in categorizing a malignancy or a cell of a malignancy.

A time period in which a Replikin Count is determined for a malignancy, a plurality of malignancies, a cell of a malignancy, or a plurality of cells of a malignancy may be any time period available to one of skill in the art that is helpful for categorizing a malignancy or the cell of a malignancy. For example, a time period may be prior to surgical removal and another time period may be after surgical removal, a time period may be before, during, or after a therapy such as chemotherapy, radiation, or biological therapy, a time period may be a specific day, a specific week, a specific month, or a specific year of a malignancy in a subject. A time period is any time period that is understood by one of skill in the art to provide helpful information in categorizing, following, diagnosing, or providing prognosis for a malignancy.

The method may also employ a ratio of the number of Replikin Counts that are greater than one standard deviation of the mean divided by the number of Replikin Counts that are less than one standard deviation of the mean. The ratio is called a Replikin Count Expansion Index (RCE Index). Another way to determine the RCE Index is to divide the percent of Replikin Counts in a plurality of cells grouped by time and/or anatomic region that are higher than one standard deviation of the mean by the percent of Replikin Counts that are lower than one standard deviation of the mean. An RCE Index may be used to quantify the future risk of an expansion of a malignancy by tracking Replikin Counts in malignant cells in a patient suffering from malignancy over time.

In determining a RCE Index, the mean Replikin Count of the plurality of cells from the first time period and first anatomic region is considered a control. A control population preferably has a relatively large number of cells with relatively small variability in the Replikin Count of the cells, but any population may be deemed a control when a comparison between the control and a related cell or plurality of cells is desired. A control may be related to the population of cells that is being studied.

For example, if glioma is being studied, the mean Replikin Count with standard deviation of a plurality of cells from the initial biopsy may be compared to Replikin Counts in a plurality of cells from the removed tumor or to Replikin Counts in cells from a metastatic tumor.

A control may also be established from cells isolated from malignancies of the same or similar histological types in a plurality of patients or from malignancies of different histological types in a plurality of patients. A control may represent Replikin Counts from a broad collection of malignancies from a broad collection of patients in a particular type of cancer, in all types of cancer, or in unknown types of cancer. Such controls provide the ordinary skilled artisan with baseline numbers for Replikin Counts and standard deviations in Replikin Counts.

In determining an RCE index, any measure of Replikin concentration may be used. Replikin Count may reflect the concentration of Replikin peptides identified encoded in the genome of a cell. Replikin Count may also reflect the concentration of Replikin peptides identified in the expressed proteins of a cell or in at least one protein or protein fragment of a cell. Replikin Count may also reflect the concentration of Replikin peptides identified in a Replikin Peak Gene of a cell.

Any Replikin peptide, Replikin Peak Gene, protein, protein fragment, or nucleic acid sequence encoding any Replikin peptide, Replikin Peak Gene, protein, or protein fragment in a cell predicted by the methods of the invention to be expanding may be used for diagnostic, therapeutic, and/or preventive purposes. Further, a vaccine may be manufactured by identifying a portion of the structure or genome of a cell predicted to expand in population and using that portion in a vaccine composition.

Methods of the invention also provide methods of predicting a decrease in aggressiveness and/or lethality of a malignancy and/or predicting a contraction of a malignancy wherein a Replikin Count of at least one cell of a malignancy from a second time period and/or second anatomic region is less than one standard deviation of the mean of the Replikin Count of a plurality of cells from a first time period and first anatomic region. A decrease in aggressiveness may also be predicted where the number of Replikin Counts of a plurality of cells from a second time period and/or a second anatomic region that are greater than one standard deviation of the mean is less than the number of Replikin Counts less than one standard deviation of the mean. A decrease or contraction is predicted if the ratio of the Replikin Count Expansion Index is less than one.

When a population contains cells with Replikin Counts above one standard deviation of the mean of a control and does not contain cells with Replikin Counts below one standard deviation of the mean of the control, the ratio of the RCE Index is considered to have a denominator of one to avoid an index of infinity.

In determining a Replikin Count Expansion Index, Replikin Counts from Replikin Peak Genes may be analyzed from anatomic regions in a given time period (such as at initial biopsies or removal of tumors). Within a patient at a particular time, there may be a range of values. The ordinary skilled artisan may select a mean Replikin Count as a control from the range of values.

When comparing the Replikin Count of an individual cell or related group of cells to a control, all Replikin Count values within the group of related cells that fall within one standard deviation of the mean may be treated as a group. Additionally, all values that fall outside the range of one standard deviation from the mean may be treated as two outlying groups. A first group is the group of Replikin Counts that are greater than the mean plus one standard deviation. A second group is the group of Replikin Counts that are less than the mean minus one standard deviation. Because higher Replikin Counts are associated with an expansion of the malignancy and lower Replikin Counts are associated with a decrease in the rate of growth of the malignancy, the ratio of the percent of isolates having Replikin Counts above mean plus one standard deviation to the percent of isolates having Replikin Counts below the mean minus one standard deviation provides a quantitative index of the viability and expansion of the malignancy. The index provides a snapshot of current status of the cancer cell population and the propensity for change in that population. If the ratio is greater than one, the RCE Index predicts an expanding population. If the ratio is less than one, the RCE Index predicts a reduction in growth of the population.

### Conserved Replikin Peptides in Cancer Provide Diagnostic and Therapeutic Targets

Replikin sequences have been observed to be conserved in human cancers generally (and in many pathogenic organisms and viruses) and an increase in concentration of expressed proteins containing Replikin sequences has been observed in cancer as replication increases. *See, e.g.,* discussion of malignin production in a range of cancer types in U.S. Appln. Ser. No. 12/010,027, filed January 18, 2008. In viruses and trypanosomes, cycles of a comparable excess in the Replikin Count have also been related quantitatively to lethality in Taura Syndrome virus in shrimp as well as influenza H5N1 virus in birds and humans, *Pl. falciparum* malaria in humans, and other human viruses. *See, e.g.,* U.S. Appln. Ser. No. 12/108,458, filed April 23, 2008, U.S. Appln., Ser. No. 12/010,027, filed January 18, 2008, and U.S. Provisional Appln. Ser. No. 61/054,010, filed May 16, 2008. This quantitative relationship has successfully been used to predict differences in lethality. *See, e.g.,* U.S. Appln. Ser. No. 12/108,458, filed April 23, 2008, U.S. Appln., Ser. No. 12/010,027, filed January 18, 2008, and U.S. Provisional Appln. Ser. No. 61/054,010, filed May 16, 2008. Likewise, concentration of Replikin sequences in viral genomes has been shown to increase prior to strain-specific outbreaks of the viruses and increased mortality has been associated with increases in Replikin Count in SARS coronavirus, in influenza, in H5N1 bird flu, and in many other viral and non-viral pathogens. *See id.* As such, the findings in both infectious diseases and cancer suggest that regardless of the vector and the host, Replikin sequences in the genome are quantitative markers of the degree of lethality produced in the host. Replikin sequences, however, have not been associated in growth, replication, and lethality in malignancies of different types or of unknown types or across malignancies that do not share specific histology or type.

Additionally, Replikin peptide sequences by themselves have been demonstrated to be non-toxic when administered to animals for the purpose of stimulating the immune system. *See, e.g.,* Examples 6 and 7 of U.S. Appln. Ser. No. 11/355,120, filed February 16, 2006. Further, a specific Replikin peptide vaccine was found to be 91 % protective against lethal Taura Syndrome virus when administered to shrimp, *see* U.S. Appln. Ser. No. 12/108,458, filed April 23, 2008, and a vaccine containing a combination of twelve specific Replikin peptides from a low-pathogenic strain of H5N1 influenza virus was found to provide a protective effect and to block both infection and excretion of virus in vaccinated chickens subjected to challenge with the same strain of low-pathogenic H5N1. *See* U.S. Appln. Ser. No. 12/429,044, filed April 23, 2009.

In cancer, vaccines containing Replikin sequences are likewise effective. For example, peptide sequences from brain cancer, breast cancer, and lymphomas that contain a Replikin sequence each induced production of antimalignin antibodies when administered to animals. The antimalignin antibodies that are produced 1) are demonstrated to be cytotoxic to cancer cells in low doses (picomoles per cell), 2) are demonstrated to increase in concentration in the serum of healthy humans as age and risk of cancer increase, and 3) are quantifiable in serum as an approved test for the early detection of first occurrence and recurrence of malignancies (AMAS^{®} test available from Oncolab, Inc., Boston, MA). *See, e.g.,* U.S. Patent No. 7,381,411, issued June 3, 2008.

Replikin peptide vaccines may be produced by sequencing cancer cell proteins or expressed cancer cell genes, or identifying specific Replikin peptide sequences in the expressed cell proteins, and using the identified Replikin peptide sequences as a basis for production of synthetic cancer vaccines comprising the identified Replikin peptide sequences (service available through Replikins LLC, Boston, MA). Concerning cancer vaccines, Replikin peptide sequences are preferred as vaccines over DNA-based vaccines because of the recent demonstration in *C. elegans* that DNA can be incorporated into the genome of a vaccinated subject and may be reproduced in later generations with unknown consequences.

An isolated or synthesized Replikin peptide or Replikin peptides or Replikin Peak Gene or Peak Genes may be used for diagnostic, therapeutic, and/or preventive purposes identified in a malignancy that is predicted to have a higher rate of growth or rate of replication or greater lethality than a second malignancy or than a plurality of other malignancies. A Replikin peptide may be identified in the segment of the genome of the malignancy, of the protein of the malignancy, or of the protein fragment of the malignancy that has the highest concentration of continuous, non-interrupted and/or overlapping Replikin sequences when compared to other segments of the genome of the malignancy, other proteins of the malignancy, or other protein fragments of the malignancy. Another embodiment of one aspect of the invention provides an isolated or synthesized Replikin Peak Gene identified in a malignancy that is predicted to have a higher replication rate or greater lethality than a second malignancy or than a plurality of malignancies. The Replikin Peak Gene of the invention may be used for diagnostic, therapeutic, preventive, and predictive purposes.

Replikin peptides or Replikin Peak Genes for diagnostic, therapeutic, and/or preventive purposes identified in a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, or a glioblastoma malignancy is described herewith.

### Replikins in Diagnostics and Therapies

Identified Replikin peptides and Replikin Peak Genes are also useful where a malignancy is predicted via analysis of Replikin concentration to have a relatively higher or lower rate of replication and/or a greater or lower lethality than another malignancy or wherein a primary malignancy is predicted via analysis of Replikin concentration to have a relatively higher or lower rate of replication or relatively greater or lower lethality than its metastases.

A composition comprising a component of at least one first malignancy may be an immunogenic composition. The composition may be a vaccine or may be comprised in a vaccine. A vaccine may produce an immune response, an antibody response, and/or a protective effect when administered to a subject. The composition may further be combined with a pharmaceutically acceptable carrier or adjuvant or both.

Replikin peptides identified in a malignancy that is relatively more lethal are useful for diagnostic, therapeutic, and preventive purposes with respect to relatively more lethal malignancies. *See, e.g.,* U.S. Patent Appln. Ser. No. 12/010,027, filed January 18, 2008, 211-218. For example, a Replikin peptide or a Replikin Peak Gene identified in a relatively more lethal malignancy is useful as a peptide to stimulate the immune system of a human or animal to produce an immune response against malignancies of the type of cancer in which the Replikin peptide was isolated or against other lethal cancers or to produce antibodies against cancers predicted to have higher lethality. One of ordinary skill in the art will recognize that antibodies against these malignancies are useful for diagnosing malignancies of higher lethality in a subject or are useful as therapies against the malignancy or against malignancies of the same or different type or against recurrence of the malignancy in a patient that has suffered from the malignancy. Antibodies against Replikin peptides or Replikin Peak Genes identified in a relatively more lethal malignancy provide a tool for diagnosing or attacking structures in malignancies that are particularly related to rapid replication and/or lethality in a particular malignancy, in a group of malignancies, or in malignancies generally.

Likewise, Replikin peptides identified in relatively lethal malignancies that are conserved in other malignancies or conserved in relatively lethal malignancies are useful as compounds for diagnostic, therapeutic, and preventive purposes. As such, these conserved Replikin peptides are of use as compounds or in compositions for stimulating the immune system of a subject to produce an immune response, an antibody response, and/or a protective effect in the subject.

Because Replikin sequences and Replikin Peak Gene sequences are chemically defined, the sequences may be synthesized by organic chemistry or biological techniques. Replikin sequences synthesized by organic chemistry may be particularly specific, highly reproducible, and highly reliable as compared to other vaccines and therapies. Chemically defined Replikin sequences are likewise potentially freer from adverse reactions characteristic of biologically derived vaccines and antibodies.

An embodiment of an aspect of the invention further contemplates use of Replikin peptides and/or Replikin Peak Genes as immunogenic compositions and contemplates construction of immunogenic compositions as vaccines, including vaccines that provide an immune response, vaccines that provide a humoral immune response, vaccines that provide an antigenic immune response, and vaccines that provide a protective effect.

An immunogenic composition may comprise a Replikin peptide identified in a malignancy predicted to have a higher replication rate or greater lethality than another malignancy or than a plurality of other malignancies. A Replikin peptide may be identified in the segment of the genome, of the protein, or of the protein fragment of the malignancy that has the highest concentration of continuous non-interrupted and/or overlapping Replikin sequences when compared to other segments of the genome, or proteins, or protein fragments of the malignancy. An immunogenic composition may comprise a Replikin Peak Gene identified in a malignancy predicted to have a higher rate of replication or greater lethality or a Replikin Peak Gene identified separately in a primary malignancy and in a metastasis from a primary malignancy or in a metastasis from an unknown primary source.

An immunogenic composition may be comprised in a vaccine for treatment and/or prevention of a malignancy. The malignancy may be predicted to have a higher rate of replication or greater lethality than another malignancy or than a plurality of other malignancies or may be a primary malignancy predicted to have a lower or higher rate of replication or lower or greater lethality than a metastasis of the same malignancy. A vaccine comprising the immunogenic composition may be for the treatment and/or prevention of a malignancy that is of the same type as a malignancy predicted to have a higher rate of replication or greater lethality than another malignancy or than a plurality of other malignancies.

A Replikin peptide or Replikin Peak Gene identified in a first malignancy may be used for the treatment of the first malignancy or may be used for the treatment of another malignancy wherein the other malignancy is related to first malignancy in origin and/or histology, including a metastasis of the first malignancy. A Replikin peptide or Replikin Peak Gene identified in a first malignancy may also be used for the treatment of another malignancy that is unrelated in origin or histology to the first malignancy, including a malignancy of unknown histology or origin.

A Replikin peptide or Replikin Peak Gene identified in a first malignancy may be used for the manufacture of a medicament for the treatment of a malignancy including a malignancy that is related to the first malignancy or unrelated to the first malignancy by histology or origin or any other relationship known to one of skill in the art now or hereafter.

### Antibodies against Replikins in Diagnostics and Therapies

An antibody or antibody fragment directed against a Replikin peptide or against a Replikin Peak Gene may be used for diagnostic, therapeutic, and/or preventive purposes in cancer, including any cancer known to one of ordinary skill in the art now and hereafter, which may include a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, a glioblastoma malignancy, or any other malignancy of an animal or a human.

Described herewith -is also a method of stimulating the immune system of any animal or human capable of an immune response by administering at least one Replikin peptide or at least one Replikin Peak Gene identified in at least one first malignancy predicted, according to methods of the invention, to have a relative rate of growth that is faster than the relative rate of growth of at least one second malignancy. Another embodiment provides a method of making an antibody or an antibody fragment that binds to at least one Replikin peptide or at least one Replikin Peak Gene identified in at least one first malignancy that has a relative rate of growth faster than the relative rate of growth of at least one second malignancy comprising identifying the at least one Replikin peptide or the at least one Replikin Peak Gene and making an antibody or antibody fragment that binds to the at least one Replikin peptide or the at least one Replikin Peak Gene. One of ordinary skill in the art would know myriad ways of making antibodies or antibody fragments that bind to a Replikin peptide or Replikin Peak Gene that is isolated from a cell of a malignancy or for which an amino acid sequence is known.

### Vaccines, Treatments and Therapeutics

A peptide vaccine of the invention may include a single Replikin peptide sequence or Replikin Peak Gene or may include a plurality of Replikin sequences and/or Replikin Peak Genes observed in particular malignancies. A vaccine may include a conserved Replikin peptide or peptides in combination with a Replikin peptide or Replikin peptides in a particular malignancy or may be based on other Replikin peptide sequences such as UTOPES. *See* U.S. Patent 7,442,761. Replikin peptides can be synthesized by any method, including chemical synthesis or recombinant gene technology, and may include non-Replikin sequences. Vaccine compositions of the invention may also contain a pharmaceutically acceptable carrier and/or adjuvant.

The vaccines can be administered alone or in combination with chemotherapies, hormone therapies or other anti-cancer therapies and/or treatments. The vaccine may be administered to any animal capable of producing antibodies in an immune response or to any animal capable of producing a humoral response, a protective effect, or any immune or immune-like response. For example, the vaccine may be administered to a rabbit, a chicken, a pig, a human, or any other animal capable of producing an immune response and/or antibodies in response to an antigen. Because of the universal nature of Replikin sequences, a vaccine may be directed at a range of malignancies.

The Replikin peptides of the invention, alone or in various combinations may be administered to a subject by any manner known to one of ordinary skill in the art including by intravenous or intramuscular injection, ocular swab or spray, nasal spray and/or inhalation spray, or any other method of administration in order to stimulate the immune system of the subject to produce an immune response. Generally the dosage of peptides is in the range of from about 0.1 µg to about 10 mg, about 10 µg to about 1 mg, and about 50 µg to about 500 µg. The skilled practitioner can readily determine the dosage and number of doses needed to produce an effective immune response.

### Anti-Sense Nucleic Acids and siRNA

Nucleic acid sequence described herewith is the one that is antisense to a nucleic acid that encodes for a Replikin peptide or a Replikin Peak Gene identified in a first malignancy predicted to have a higher rate of growth or replication or a greater lethality. Nucleic acid sequences include, for example, one or more small interfering nucleic acid sequences that interfere with a nucleic acid sequence that is 50%, 60%, 70%, 80%, or 90% or more homologous with a nucleic acid that encodes for a Replikin peptide or a Replikin Peak Gene identified in a first malignancy predicted to have a higher rate of growth or replication or a greater lethality, or is 50%, 60%, 70%, 80%, or 90% or more homologous with a nucleic acid that is antisense to a nucleic acid that encodes for a Replikin peptide or a Replikin Peak Gene identified in a first malignancy predicted to have a higher rate of growth or replication or a greater lethality.

Such nucleotide sequences may be used in hybridization assays of biopsied tissue or blood, *e.g*., Southern or Northern analysis, including *in situ* hybridization assays, to diagnose the presence of a particular organism in a tissue sample or an environmental sample, for example. The present invention also contemplates kits containing antibodies specific for particular Replikins that are present in a particular pathogen of interest, or containing nucleic acid molecules (sense or antisense) that hybridize specifically to a particular Replikin, and optionally, various buffers and/or reagents needed for diagnosis.

Also described herewith are oligoribonucleotide sequences that include antisense RNA and DNA molecules and ribozymes that function to inhibit the translation of Replikin-containing mRNA. Both antisense RNA and DNA molecules and ribozymes may be prepared by any method known in the art. The antisense molecules can be incorporated into a wide variety of vectors for delivery to a subject. The skilled practitioner can readily determine the best route of delivery. Intravenous or intramuscular delivery is one possible method of delivery and is one, among many, routine delivery methods in the art of small molecule delivery. The dosage amount is also readily ascertainable. Dosage may range from 0.01 mg to 10 mg, from 0.1 mg to 5 mg, from 0.5 mg to 2 mg, and from 0.75 mg to 1.25 mg, but is not limited to such ranges.

An antisense nucleic acid molecule may be complementary to a nucleotide sequence encoding a Replikin peptide or a Replikin Peak Gene as described herein. A nucleic acid sequence may be anti-sense to a nucleic acid sequence that has been demonstrated to be conserved in a malignancy or generally conserved in a range of malignancies of a particular cancer type or of different cancer types.

An effective amount of an RNAi-inducing entity can be delivered to a cell or organism prior to, simultaneously with, or after diagnosis of a malignancy or a metastasis. A dosage may be sufficient to reduce or delay replication of the malignancy or metastasis. Compositions of the invention may comprise a single siRNA species targeted to a target transcript or may comprise a plurality of different siRNA species targeting one or more target transcripts.

The invention describes a small interfering nucleic acid sequence that is about 10 to about 50 nucleic acids in length and is 50%, 60%, 70%, 80%, or 90% or more homologous with a nucleic acid that encodes for any portion of at least one Replikin peptide or at least one Replikin Peak Gene, or is 50%, 60%, 70%, 80%, or 90% or more homologous with a nucleic acid that is antisense to a nucleic acid that encodes for any portion of at least one Replikin peptide or at least one Replikin Peak Gene. Preferred nucleic acid sequence is about 15 to about 30 nucleic acids. Further preferred nucleic acid sequence is about 20 to about 25 nucleic acids. Most preferred the nucleic acids sequence is about 21 nucleic acids.

### Prognosis for Individual Malignancies based on Replikin Count of Malignancy and Survey of Plurality of Malignancies

An individual prognosis for a malignancy may be provided for a patient suffering from the malignancy based on Replikin Count and lethality data from a survey of patients suffering from malignancies of the same type, malignancies of related types, or malignancies in general. For example, a survey may be undertaken in which Replikin Counts of tissues from tumors removed from patients are analyzed and compared with patient outcome.

For example, in one non-limiting embodiment, a Replikin Count Expansion Index may be created from a survey of malignancies. To create a Replikin Count Expansion Index, a mean Replikin Count plus one standard deviation of the mean is determined from a plurality of malignancies and the measured lethality of each malignancy is determined by any method.

A Replikin Count of a malignancy having an unknown lethality may then be compared to the mean Replikin Count plus one standard deviation of the mean. If the Replikin Count of the malignancy having an unknown lethality is greater than the mean Replikin Count plus one standard deviation of the mean, the malignancy of unknown lethality is predicted to have a greater lethality than the mean measured lethality of plurality of malignancies. If the Replikin Count of the malignancy having an unknown lethality is less than the mean Replikin Count minus one standard deviation of the mean, the malignancy of unknown lethality is predicted to have a lower lethality than the mean measured lethality of the plurality of malignancies.

To provide other kinds of precision in the prediction of lethality, a plurality of malignancies may be grouped into a plurality of groups of malignancies having related lethalities or related types. A mean Replikin Count plus one standard deviation of the mean may be determined for some or all of these groups. A Replikin Count of a malignancy having an unknown lethality may then be compared to the mean Replikin Count plus or minus one standard deviation of the mean of a particular group having a related lethality.

In another non-limiting embodiment, regression analysis may be undertaken based on the Replikin Count data and the data of patient outcome. The resulting regression formula may be used to provide a prognosis for a patient suffering from a malignancy wherein a Replikin Count has been determined from the malignancy.

For example, a Replikin Count determined in a lung tumor (or any other malignancy) of a patient suffering from lung cancer (or any other malignancy) may be entered into a formula generated using survey data of Replikin Counts of individual lung tumors (or any other malignancy) and patient outcome from the individual lung tumors (or other malignancies). The formula may provide a prognosis concerning the rate of growth, aggressiveness, or lethality of the lung tumor (or other malignancy) based on the Replikin Count of the lung tumor.

Patient outcome may include but is not limited to five year mortality rate, life expectancy, aggressiveness of growth, rate of growth, rate of replication, rate of decline of lung capacity, *etc.* Patient outcome may include any measure of patient well-being that may be compared to Replikin Count of a malignancy.

Replikin Count may be any measure of Replikin Count in a malignancy including Replikin Count of the entire genome or of a portion or fragment of the genome, Replikin Count of an expressed protein, Replikin Count of a Replikin Peak Gene, Replikin Count of a protein fragment, Replikin Count of a combination of proteins and/or protein fragments, or mean Replikin Count of a plurality of cells in a malignancy, *etc.*

In a survey, the Replikin Count measurement having the best correlation with the patient outcome measurement may be used for regression analysis. The greater the size of the survey, the more precision that is expected from the regression analysis. As such, a survey of a plurality of malignancies would be appropriate as a survey. Likewise, a survey of 10 malignancies, 50 malignancies, 100 malignancies, 500 malignancies, 1,000 malignancies, or 10,000 or more malignancies would be appropriate for generating a Replikin Count Expansion Index or a formula from regression analysis.

Within a survey of malignancies, more than one regression analysis may be peformed. For example, two regression curves may be generated within one data set from a survey. Two regression curves may be appropriate where a data set contains, for example, both benign and malignant tumors. A regression curve for benign tumors may be quite different from a regression curve for malignant tumors. Likewise, regression curves may be different for different types of malignancies. The closer a regression curve fits to the particular data to which it is applied, the more precision that would be expected in predicting patient outcome from a malignancy of unknown lethality.

For example, benign tumors may be expected to have a growth rate that allows many years of growth of the tumor while a malignant tumor may have a growth rate that allows less years of growth, including tumors that are lethal in days, months, or one or two or more years. Different regression curves that apply to different data sets provide one of ordinary skill in the art with many ways to separate different types of malignancies and many types of curves for predicting lethality. Regression curves may be linear, polynomial, exponential, or any other type of regression curve known to one of skill in the art now or hereafter. One, two, three, or more regression curves may be applied to a single data set based on the understanding of one of ordinary skill in the art.

Health practitioners may employ a formula generated from a Replikin Count Expansion Index or regression analysis, or any other kind of analysis to provide a prognosis for a patient suffering from a malignancy. For example, if a patient is suffering from a lung malignancy, a health practitioner may take a biopsy of the malignancy and have the Replikin Count of a Replikin Peak Gene of a particular protein of the malignancy determined. The health practitioner may then enter the Replikin Count of the Replikin Peak Gene of the particular protein of the lung malignancy into an index or formula generated from a survey based on Replikin Counts of the Replikin Peak Gene of the lung malignancy from which the patient is suffering. The index or formula may provide a prognosis concerning the rate of growth of the malignancy, the aggressiveness of the malignancy, or the life expectancy of the patient suffering from the malignancy. The resulting prognosis may then be provided to the patient suffering from the lung malignancy.

A survey and resulting Replikin Count Expansion Index or regression analysis may be undertaken for any malignancy or any type of malignancy known to one of skill in the art now or hereafter. A survey and resulting Replikin Count Expansion Index or regression analysis may also be done for malignancies generally wherein the Replikin Count of any malignancy may be entered into an index or formula to provide a prognosis concerning the growth or lethality of the malignancy.

A survey of a specific type of malignancy will provide a more defined prognosis for a patient suffering from that specific malignancy. Further, a survey of a specific type of malignancy at a specific stage of growth may provide an even more defined prognosis for a patient suffering from that specific malignancy at that specific stage of growth.

Figure 2 provides a regression analysis of the data in Figure 1, which compares the highest Replikin Count of a Replikin Peak Gene of various common human cancers with the five-year mortality rate of the cancer as provided in Brenner, H., The Lancet, 360 (October 12, 2002), 1131-1135. The regression formula provided in Figure 2 is y = 0.0401x² - 1.3041x + 35.812 with an r² value of 0.9089. This regression formula may be used to provide a prediction of the relative five year mortality of a malignancy based on the highest Replikin Count of a Replikin Peak Gene in a representative malignancy of a known type of malignancy. By introducing the Replikin Count of the Replikin Peak Gene of the representative malignancy into the y value of the regression formula, the resulting x value will provide a predicted five year mortality rate. Any other kind of regression analysis may be applied to the data in Figure 2.

The process of creating a regression formula for a given type of malignancy or for a range of malignancies may be undertaken from a survey of any plurality of malignancies. Once baseline data is provided by the survey, the formula may be used to provide a patient suffering from a malignancy a prognosis based on a prediction of the rate of growth, rate of replication, aggressiveness, or lethality of a malignancy.

A prognosis based on a Replikin Count Expansion Index or based on a regression analysis of Replikin Count and measured lethality may be provided for any malignancy including but not limited to, a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, or a glioblastoma malignancy.

The efficacy of histopathologically-determined degrees of malignancy may also be quantified by comparing the histopathological degree of malignancy given to a tumor with a quantitative prognosis determined from a Replikin Count of a malignancy entered into a Replikin Count Expansion Index or a formula developed from regression analysis of Replikin Counts of malignancies of the same or related types or from malignancies of all types.

A kit for providing a prognosis to a patient suffering from a malignancy is contemplated comprising a Replikin Count Expansion Index or a formula for determining prognosis based on Replikin Count of the malignancy. Such a kit may be provided to laboratories and practitioners so that prognosis based on Replikin sequence analysis of individual malignancies may be provided directly from the practitioner or laboratory to the patient.

### Example 1

### Analysis of Replikin Count in Replikin Peak Gene of Common. Human Cancer Types

Applicants analyzed publicly available sequences of the common human malignancies listed in Table 1 as those sequences had been published at www.pubmed.com. Using REPLIKINSFORECAST^{®} software service available through Replikins LLC (Boston, MA), Applicants determined the Replikin Count for each accession number with a sequence isolated from the listed malignancies (*i.e*., all accession numbers containing sequences published as isolated from prostate cancers, thyroid cancers, breast cancers, urinary bladder cancers, *etc.*) Applicants then determined mean Replikin Count for all data for each listed common human malignancy in a given year for which accession numbers were available.

For those years in which mean Replikin Count was notably higher than other years, Applicants identified the Replikin Peak Gene of each published sequence. The Replikin Peak Gene is defined as the segment of the reported genome, reported protein, or reported protein fragment containing the highest number of continuous and/or overlapping Replikin sequences per 100 amino acids (Replikin Count) for each identified Replikin Peak Gene. For the purpose of the Replikin Count, each Replikin sequence is defined as a peptide sequence consisting of 7 to 50 amino acids (or a nucleic acid sequence encoding a peptide sequence consisting of 7 to 50 amino acids) and having (or encoding a sequence having) (1) at least one lysine residue located at a first terminus of the sequence and at least one lysine residue or at least one histidine residue located at a second terminus of the sequence, (2) a first lysine residue located six to ten residues from a second lysine residue, (3) at least one histidine residue, and (4) at least 6% lysine residues.

For each identified Replikin Peak Gene, Applicants determined the concentration of Replikin sequences by determining the number of Replikin sequences per 100 amino acids (Replikin Count) in the identified Replikin Peak Gene. From the Replikin Count data for each identified Replikin Peak Gene, Applicants selected the highest Replikin Count as the representative highest Replikin Count for a given common human malignancy.

Applicants then compared the representative highest Replikin Count for each common human malignancy to the percent five-year mortality rate reported at Brenner, H., "Long-term survival rates of cancer patients achieved by the end of the 20th century: a period analysis," The Lancet, 360 (October 12, 2002), 1131-1135. The comparative data are shown in Table 1 and the comparison is graphically presented in Figure 1. Figure 1 graphically demonstrates a quantitative relationship between highest Replikin Count in a malignancy and reported five-year mortality rate.

### Example 2

### Prediction of Relative Mortality in Human Malignancies

The Replikin Count of an identified Replikin Peak Gene is analyzed following sequencing of some or all of the genome of at least a first and a second malignancy or following the sequencing of at least one protein or protein fragment of at least a first and a second malignancy. The Replikin Count of the Replikin Peak Gene of the first malignancy is compared to the Replikin Count of the Replikin Peak Gene of at least the second malignancy. If the Replikin Count is determined to be higher than at least the second malignancy, the first malignancy is predicted to have a relatively higher rate of replication and/or greater lethality than at least the second malignancy. If the Replikin Count is determined to be lower than at least the second malignancy, the first malignancy is predicted to have a relatively lower rate of replication and/or greater lethality than at least the second malignancy.

### Example 3

### Prediction of Relative Rate of Increase of Growth Rate of Malignancy

The relative rate of change in the rate of growth of a metastasis of a malignancy is predicted by comparing the standard deviation of the mean Replikin Count of the genome, a genome fragment, all expressed proteins, an expressed protein, or a protein fragment of a plurality of cells of the primary malignancy to the standard deviation of the mean Replikin Count of the genome, a genome fragment, all expressed proteins, an expressed protein, or a protein fragment of a plurality of cells of a metastasis of the malignancy. The mean Replikin Count of a metastasis may likewise be compared to a mean Replikin Count of a plurality of metastases from patients having a similar malignancy or from a broad range of malignancies generally. If the standard deviation of the mean Replikin Count in a plurality of cells of the primary malignancy is greater than the standard deviation of the mean Replikin Count in a plurality of cells of the metastasis of the malignancy (or of a mean Replikin Count of a plurality of metastases from patients having a similar malignancy or from a broad range of malignancies generally), the metastasis of the malignancy is predicted to experience a decrease in the relative rate of change in the rate of growth as compared to the primary malignancy. On the other hand, if the standard deviation of the mean Replikin Count in a plurality of cells of the primary malignancy is less than the standard deviation of the mean Replikin Count in a plurality of cells of the metastasis of the malignancy (or of a mean Replikin Count of a plurality of metastases from patients having a similar malignancy or from a broad range of malignancies generally), the metastasis of the malignancy is predicted to experience an increase in the relative rate of change in the rate of growth as compared to the primary malignancy.

### Example 4

### Replikin Count Expansion Index in Primary and Metastatic Malignancy over Time

Replikin Count of a genome or genome fragment or expressed proteins, an expressed protein, or protein fragment of a plurality of cells from a malignancy are determined. Replikin Counts from cells from a specific time and/or specific anatomical region of the malignancy are grouped and a mean Replikin Count with standard deviation is determined for each group. The cells may be from the primary malignancy or a metastasis of the malignancy, or may be of unknown origin within the body of the patient suffering from the malignancy.

Replikin Count of a genome or genome fragment or expressed proteins, an expressed protein, or protein fragment of at least one cell of a metastasis of the malignancy (or from a malignant cell of unknown origin or type within the body of the patient suffering from the malignancy) from a second time and/or second anatomical region in the patient suffering from the malignancy is determined. If Replikin Count is determined for more than one cell, a mean Replikin Count with standard deviation is determined.

The time period and/or anatomical region having the largest number of cells or the least variability among Replikin Count in cells (or both) is chosen as a control against which other Replikin Counts are analyzed. The Replikin Count for each individual cell in a given anatomical region at a particular time is compared to one standard deviation from the mean Replikin Count for all cells from the control region. Within each region, the number of Replikin Counts greater than one standard deviation of the mean and the number of Replikin Counts less than one standard deviation of the mean is determined. For each region at each time period, the percent of Replikin Counts greater than one standard deviation of the mean is then divided by the percent of Replikin Counts less than one standard deviation of the mean to provide a ratio, or Replikin Count Expansion (RCE) Index. In anatomical regions having an RCE Index of greater than one, an expansion of the rate of growth of the malignancy is predicted. In anatomical regions having an RCE Index of less than one, a contraction of the rate of growth of the malignancy is predicted.

In anatomical regions wherein the rate of growth of a malignancy is predicted to expand, a Replikin Peak Gene is identified in a cell having a Replikin Count that is higher than the mean Replikin Count for the anatomical region. The Replikin Peak Gene and/or a Replikin peptide (or plurality of Replikin peptides) within the Replikin Peak Gene is selected as an immunogenic compound for diagnostic and/or therapeutic purposes. A vaccine against the expanding malignancy is manufactured comprising the immunogenic compound. The vaccine is administered to mitigate the expanding malignancy.

### Example 5

### Determination of Replikin Count or Replikin Peak Gene in 1999 Isolate of Glioblastoma Topoisomerase in Rat and Development of Glioblastoma Vaccine in Rat Model

Accession numbers of amino acid sequences of glioblastoma malignancies in rats were queried at www.pubmed.com. Replikin Count data from the queried accession numbers provided predictions of the relative lethality of each malignancy. The highest Replikin Count in a Replikin Peak Gene in an amino acid sequence from rat glioblastoma was identified in a 1999 topoisomerase isolate of glioblastoma in Accession Number Q9WUL0 taken from a study of the C6 glioblastoma cell line in Norway rats. Among the queried accession numbers, the topoisomerase in Accession Number Q9WUL0 had the highest Replikin Count in a Replikin Peak Gene with a Replikin Count of 316 Replikin sequences per 100 amino acids within the Replikin Peak Gene.

Accession Number Q9WUL0 contains the following amino acid sequence:

The following Replikin sequences were identified in the amino terminal of Accession Number Q9WUL0:
h⁵ l⁶ h⁷ h⁸ d⁹ s¹⁰ q¹¹ i¹² e¹³ a¹⁴ d¹⁵ f¹⁶ r¹⁷ l¹⁸ n¹⁹ d²⁰ s²¹ h²² k²³ h²⁴ k²⁵ d²⁶ k²⁷ h²⁸ k²⁹ (SEQ ID NO: 2)
h⁵ l⁶ h⁷ n⁸ d⁹ s¹⁰ q¹¹ i¹² e¹³ a¹⁴ d¹⁵ f¹⁶ r¹⁷ l¹⁸ n¹⁹ d²⁰ s²¹ h²² k²³ h²⁴ k²⁵ d²⁶ k²⁷ h²⁸ k²⁹ d³⁰ r³¹ e³² h³³ r³⁴ h³⁵ k³⁶ (SEQ ID NO: 3)
h⁷ n⁸ d⁹ s¹⁰ q¹¹ i¹² e¹³ a¹⁴ d¹⁵ f¹⁶ r¹⁷ l¹⁸ n¹⁹ d²⁰ s²¹ h²² k²³ h²⁴ k²⁵ d²⁶ k²⁷ h²⁸ k²⁹ (SEQ ID NO: 9)
h⁷ n⁸ d⁹ s¹⁰ q¹¹ i¹² e¹³ a¹⁴ d¹⁵ f¹⁶ r¹⁷ l¹⁸ n¹⁹ d²⁰ s²¹ h²² k²³ h²⁴ k²⁵ d²⁶ k²⁷ h²⁸ k²⁹ d³⁰ r³¹ e³² h³³ r³⁴ h³⁵ k³⁶ (SEQ ID NO: 10)
h²² k²³ h²⁴ k²⁵ d²⁶ k²⁷ h²⁸ k²⁹ (SEQ ID NO: 17)
h²² k²³ h²⁴ k²⁵ d²⁶ k²⁷ h²⁸ k²⁹ d³⁰ r³¹ e³² h³³ r³⁴ h³⁵ k³⁶ (SEQ ID NO: 18)
h²² k²³ h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ (SEQ ID NO: 1 9)
h²² k²³ h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² (SEQ ID NO: 20)
h²² k²³ h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ (SEQ ID NO: 2 1)
h²² k²³ h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ (SEQ ID NO: 22)
h²² k²³ h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ (SEQ ID NO: 23)
k²³ h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 30)
k²³ h²⁴ k²⁵ d²⁶ k²⁷ h²⁸ k²⁹ (SEQ ID NO: 34)
k²³ h²⁴ k²⁵ d²⁶ k²⁷ h²⁸ k²⁹ d³⁰ r³¹ e³² h³³ (SEQ ID NO: 35)
k²³ h²⁴ k²⁵ d²⁶ k²⁷ h²⁸ k²⁹ d³⁰ r³¹ e³² h³³ r^{34 h35} (SEQ ID NO: 36)
k²³ h²⁴ k²⁵ d²⁶ k²⁷ h²⁸ k²⁹ d³⁰ r³¹ e³² h³³ r³⁴ h³⁵ k³⁶ e³⁷ h³⁸ (SEQ ID NO: 37)
h²⁴ k²⁵ d²⁶ k²⁷ h²⁸ k²⁹ d³⁰ r³¹ e³² h³³ r³⁴ h³⁵ k³⁶ (SEQ ID NO: 38)
h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ (SEQ ID NO: 39)
h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² (SEQ ID NO: 40)
h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ (SEQ ID NO: 41)
h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ (SEQ ID NO: 42)
h²⁴ k²⁵ d²⁶ k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ (SEQ ID NO: 43)
k²⁷ h²⁸ k²⁹ d³⁰ r³¹ e³² h³³ r³⁴ h³⁵ k³⁶ (SEQ ID NO: 50)
k²⁷ h²⁸ k²⁹ d³⁰ r³¹ e³² h³³ r³⁴ h³⁵ k³⁶ e³⁷ h³⁸ (SEQ ID NO: 51)
k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r^{4G} e⁴⁷ k⁴⁸ s⁴⁹ k⁵⁰ h⁵¹ (SEQ ID NO: 52)
k²⁷ h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ (SEQ ID NO: 53)
h²⁸ k²⁹ d³⁰ r³¹ e³² h³³ r³⁴ h³⁵ k³⁶ (SEQ ID NO: 56)
h²⁸ k²⁹ d³⁰ r³¹ e³² h³³ r³⁴ h³⁵ k³⁶ e³⁷ h³⁸ k³⁹ (SEQ ID NO: 57)
h²⁸ k²⁹ d³⁰ r³¹ e³² h³³ r³⁴ h³⁵ k³⁶ e³⁷ h³⁸ k³⁹ k⁴⁰ d⁴¹ k⁴² (SEQ ID NO: 58)
h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ (SEQ ID NO: 59)
h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ (SEQ ID NO: 60)
h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ (SEQ ID NO: 61)
h²⁸ k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ (SEQ ID NO: 62)
k²⁹ d³⁰ r³¹ e³² h³³ r³⁴ h³⁵ k³⁶ (SEQ ID NO: 69)
k²⁹ d³⁰ r³¹ e³² h³³ r³⁴ h³⁵ k³⁶ e³⁷ h³⁸ (SEQ ID NO: 70)
k²⁹ d³⁰ r³¹ e³² h³³ r³⁴ h³⁵ k³⁶ e³⁷ h³⁸ k³⁹ (SEQ ID NO: 71)
k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 72)
k²⁹ d³⁰ r³¹ e³² h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ (SEQ ID NO: 73)
h³³ r³⁴ h³⁵ k³⁶ e³⁷ h³⁸ k³⁹ k⁴⁰ d⁴¹ k⁴² (SEQ ID NO: 77)
h³³ r³⁴ h³⁵ k³⁶ e³⁷ h³⁸ k³⁹ k⁴⁰ d⁴¹ k⁴² d⁴³ k⁴⁴ (SEQ ID NO: 78)
h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ (SEQ ID NO: 79)
h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ (SEQ ID NO: 80)
h³³ r³⁴ h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ SEQ ID NO: 81)
h³⁵ k³⁶ e³⁷ h³⁸ k³⁹ k⁴⁰ d⁴¹ k⁴² (SEQ ID NO: 90)
h³⁵ k³⁶ e³⁷ h³⁸ k³⁹ k⁴⁰ d⁴¹ k⁴² d⁴³ k⁴⁴ (SEQ ID NO: 91)
h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ (SEQ ID NO: 92)
h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ (SEQ ID NO: 93)
h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ (SEQ ID NO: 94)
h³⁵ k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h^{63 k64} (SEQ ID NO: 95)
k³⁶ e³⁷ h³⁸ k³⁹ k⁴⁰ d⁴¹ k⁴² (SEQ ID NO: 104)
k³⁶ e³⁷ h³⁸ k³⁹ k⁴⁰ d⁴¹ k⁴² d⁴³ k⁴⁴ (SEQ ID NO: 105)
k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 106)
k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ (SEQ ID NO: 107)
k³⁶ e³⁷ h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO:108)
h³⁸ k³⁹ k⁴⁰ d⁴¹ k⁴² d⁴³ k⁴⁴ d⁴⁵ r⁴⁶ e⁴⁷ k⁴⁸ (SEQ ID NO:113)
h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ (SEQ ID NO: 114)
h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ (SEQ ID NO: 115)
h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ (SEQ ID NO: 116)
h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ (SEQ ID NO: 117)
h³⁸ k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h³⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ (SEQ ID NO: 118)
k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 125)
k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ (SEQ ID NO: 126)
k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO: 127)
k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 131)
k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h¹⁶ (SEQ ID NO: 132)
k⁴⁰ d⁴¹ k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO: 133)
k⁴² d⁴³ k⁴⁴ d⁴⁵ r⁴⁶ e⁴⁷ k⁴⁸ s⁴⁹ k⁵⁰ h⁵¹ (SEQ ID NO: 137)
k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ (SEQ ID NO: 138)
k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k³⁷ d⁵⁸ s⁵⁹ e⁶⁰ k⁶¹ k⁶² h⁶³ (SEQ ID NO: 139)
k⁴² d⁴³ k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ (SEQ ID NO: 140)
k⁴⁴ d⁴⁵ r⁴⁶ e⁴⁷ k⁴⁸ s⁴⁹ k⁵⁰ h⁵¹ (SEQ ID NO: 143)
k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ (SEQ ID NO: 144)
k⁴⁴ d⁴⁵ r⁴⁶ e⁴⁷ k⁴⁸ s⁴⁹ k³⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO: 145)
k⁴⁴ d⁴⁵ r⁴⁶ e⁴⁷ k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ (SEQ ID NO: 146)
k⁴⁸ s⁴⁹ k⁵⁰ h⁵¹ s⁵² n⁵³ s⁵⁴ e⁵⁵ h⁵⁶ k⁵⁷ (SEQ ID NO: 149)
k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO: 150)
k⁴⁸ s⁴⁹ k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ (SEQ ID NO: 151)
k⁵⁰ h⁵¹ s⁵² n⁵³ s⁵⁴ e⁵⁵ h⁵⁶ k⁵⁷ (SEQ ID NO: 154)
k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO: 155)
k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ (SEQ ID NO: 156)
k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 157)
h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ (SEQ ID NO: 159)
h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ (SEQ ID NO: 160)
h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ (SEQ ID NO: 161)
h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ (SEQ ID NO: 162)
h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ k⁷² (SEQ ID NO: 163)
h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ (SEQ ID NO:164)
h⁵⁶ k⁵⁷ d⁵⁸ s⁵⁹ e⁶⁰ k⁶¹ k⁶² h⁶³ k⁶⁴ (SEQ ID NO: 172)
h⁵⁶ k⁵⁷ d⁵⁸ s⁵⁹ e⁶⁰ k⁶¹ k⁶² h⁶³ k⁶⁴ e⁶⁵k⁶⁶ (SEQ ID NO: 173)
h⁵⁶ k⁵⁷ d⁵⁸ s⁵⁹ e⁶⁰ k⁶¹k⁶² h⁶³ k⁶⁴ e⁶⁵ k⁶⁶ e⁶⁷ k⁶⁸ (SEQ ID NO: 174)
h⁵⁶ k⁵⁷ d⁵⁸ s⁵⁹ e⁶⁰ k⁶¹k⁶² h⁶³ k⁶⁴ e⁶⁵ k⁶⁶ e⁶⁷k⁶⁸ t⁶⁹k⁷⁰ (SEQ ID NO: 175)
h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ k⁷² (SEQ ID NO: 176)
h⁵⁶ k⁵⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ (SEQ ID NO: 177)
h⁵⁶ k³⁷ d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹k⁷²d⁷³ g⁷⁴ s⁷⁵ s⁷⁶ d⁷⁷ k⁷⁸ h⁷⁹ k⁸⁰ (SEQ ID NO: 178)
h⁵⁶ k⁵⁷ d⁵⁸ s⁵⁹ e⁶⁰ k⁶¹ k⁶² h⁶³ k⁶⁴e⁶⁵k⁶⁶ e⁶⁷k⁶⁸ t⁶⁹k⁷⁰ h⁷¹k⁷²d⁷³ g⁷⁴ s⁷⁵ s⁷⁶ d⁷⁷ k⁷⁸ h⁷⁹ k⁸⁰ d⁸¹ k⁸² (SEQ ID NO: 179)
h⁵⁶ k⁵⁷ d⁵⁸ s⁵⁹ e⁶⁰ k⁶¹k⁶² h⁶³ k⁶⁴ e⁶⁵ k⁶⁶ e⁶⁷k⁶⁸ t⁶⁹k⁷⁰ h⁷¹k⁷²d⁷³ g⁷⁴ s⁷⁵ s⁷⁶ d⁷⁷ k⁷⁸ h⁷⁹ k⁸⁰ d⁸¹ k⁸² h⁸³ k⁸⁴ (SEQ ID NO: 180)
k⁵⁷ d⁵⁸ s⁵⁹ e⁶⁰ k⁶¹ k⁶² h⁶³ k⁶⁴ (SEQ ID NO: 187)
k⁵⁷ d⁵⁸ s⁵⁹ e⁶⁰ k⁶¹ k⁶² h⁶³ k⁶⁴ e⁶⁵k⁶⁶ (SEQ ID NO: 188)
k⁵⁷d⁵⁸ s⁵⁹ e⁶⁰ k⁶¹ k⁶² h⁶³ k⁶⁴ e⁶⁵ k⁶⁶ e⁶⁷ k⁶⁸ t⁶⁹ k⁷⁰ h⁷¹ (SEQ ID NO: 189)
k⁵⁷d⁵⁸ s⁵⁹ e⁶⁰ k⁶¹k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 190)
k⁵⁷ d⁵⁸ s⁵⁹ e⁶⁰ k⁶¹k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 191)
k⁶¹ k⁶² h⁶³ k⁶⁴ e⁶⁵ k⁶⁶ e⁶⁷ k⁶⁸ (SEQ ID NO: 192)
k⁶¹ k⁶² h⁶³ k⁶⁴ e⁶⁵ k⁶⁶ e⁶⁷ k⁶⁸ t⁶⁹ k⁷⁰ h⁷¹ (SEQ ID NO: 193)
k⁶¹k⁶²h⁶³ k⁶⁴ e⁶⁵ k⁶⁶ e⁶⁷ k⁶⁸ t⁶⁹ k⁷⁰ (SEQ ID NO: 194)
k⁶¹k⁶²h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 195)
k⁶¹ k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 196)
k⁶² h⁶³ k⁶⁴ e⁶⁵ k⁶⁶ e⁶⁷ k⁶⁸ (SEQ ID NO: 197)
k⁶² h⁶³ k⁶⁴ e⁶⁵ k⁶⁶ e⁶⁷ k⁶⁸ t⁶⁹ k⁷⁰ h⁷¹ (SEQ ID NO: 198)
k⁶² h⁶³ k⁶⁴ e⁶⁵ k⁶⁶e⁶⁷ k⁶⁸ t⁶⁹ k⁷⁰ (SEQ ID NO: 199)
k⁶² h⁶³ k⁶⁴ e⁶⁵ k⁶⁶ e⁶⁷ k⁶⁸ t⁶⁹ k⁷⁰ h⁷¹ k⁷² (SEQ ID NO: 200)
k⁶² h⁶³ k⁶⁴ e⁶⁵ k⁶⁶e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 201)
k⁶² h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 202)
h⁶³ k⁶⁴ e⁶⁵ k⁶⁶ e⁶⁷ k⁶⁸ t⁶⁹ k⁷⁰ (SEQ ID NO: 203)
h⁶³ k⁶⁴ e⁶⁵ k⁶⁶ e⁶⁷ k⁶⁸ t⁶⁹ k⁷⁰ h⁷¹ k⁷² (SEQ ID NO: 204)
h⁶³ k⁶⁴ e⁶⁵ k⁶⁶ e⁶⁷ k⁶⁸ t⁶⁹ k⁷⁰ h⁷¹k⁷² d⁷³ g⁷⁴ s⁷⁵ s⁷⁶ d⁷⁷ k⁷⁸ (SEQ ID NO: 207)
h⁶³ k⁶⁴ e⁶⁵ k⁶⁶ e⁶⁷ k⁶⁸ t⁶⁹ k⁷⁰ h⁷¹k⁷² d⁷³ g⁷⁴ s⁷⁵ s⁷⁶ d⁷⁷ k⁷⁸ h⁷⁹ k⁸⁰ (SEQ ID NO: 208)
h⁶³ k⁶⁴ e⁶⁵ k⁶⁶ e⁶⁷ k⁶⁸ t⁶⁹ k⁷⁰h⁷¹k⁷² d⁷³ g⁷⁴ s⁷⁵ s⁷⁶ d⁷⁷ k⁷⁸h⁷⁹ k⁸⁰ d⁸¹ k⁸² (SEQ ID NO: 209)
h⁶³ k⁶⁴ e⁶⁵ k⁶⁶ e⁶⁷ k⁶⁸ t⁶⁹ k⁷⁰h⁷¹k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ (SEQ ID NO: 210)
h⁶³ k⁶⁴ e⁶⁵ k⁶⁶ e⁶⁷ k⁶⁸ t⁶⁹ k⁷⁰ h⁷¹k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ (SEQ ID NO: 211)
h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ (SEQ ID NO: 212)
h⁶³ k⁶⁴ e⁶⁵ k⁶⁶ e⁶⁷ k⁶⁸ t⁶⁹ k⁷⁰ h⁷¹k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² (SEQ ID NO: 213)
k⁶⁴ e⁶⁵ k⁶⁶ e⁶⁷ k⁶⁸ t⁶⁹ k⁷⁰ h⁷¹ (SEQ ID NO: 215)
k⁶⁴ e⁶⁵ k⁶⁶ e⁶⁷ k⁶⁸ t⁶⁹ k⁷⁰ h⁷¹ k⁷² (SEQ ID NO: 216)
k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 217)
k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 218)
k⁶⁶ e⁶⁷ k⁶⁸ t⁶⁹ k⁷⁰ h⁷¹ k⁷² (SEQ ID NO: 219)
k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 220)
k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 221)
k⁶⁸ t⁶⁹ k⁷⁰ h⁷¹ k⁷² d⁷³ g⁷⁴ s⁷⁵ s⁷⁶ d⁷⁷ k⁷⁸ (SEQ ID NO: 222)
k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ (SEQ ID NO: 223)
k⁶⁸ t⁶⁹ k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 224)
k⁷⁰ h⁷¹ k⁷² d⁷³ g⁷⁴ s⁷⁵ s⁷⁶ d⁷⁷ k⁷⁸ (SEQ ID NO: 225)
k⁷⁰ h⁷¹ k⁷² d⁷³ g⁷⁴ s⁷⁵ s⁷⁶ d⁷⁷ k⁷⁸ h⁷⁹ (SEQ ID NO: 226)
k⁷⁰ h⁷¹ k⁷² d⁷³ g⁷⁴ s⁷⁵ s⁷⁶ d⁷⁷ k⁷⁸ h⁷⁹ k⁸⁰ (SEQ ID NO: 227)
k⁷⁰ h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 228)
h⁷¹ k⁷² d⁷³ g⁷⁴ s⁷⁵ s⁷⁶ d⁷⁷ k⁷⁸ (SEQ ID NO: 231)
h⁷¹ k⁷² d⁷³ g⁷⁴ s⁷⁵ s⁷⁶ d⁷⁷ k⁷⁸ h⁷⁹ k⁸⁰ (SEQ ID NO: 232)
h⁷¹ k⁷² d⁷³ g⁷⁴ s⁷⁵ s⁷⁶ d⁷⁷ k⁷⁸ h⁷⁹ k⁸⁰ d⁸¹ k⁸² (SEQ ID NO: 233)
h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ (SEQ ID NO: 234)
h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ (SEQ ID NO: 235)
h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ (SEQ ID NO: 236)
h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² (SEQ ID NO: 237)
h⁷¹ k⁷² d⁷³ g⁷⁴ s⁷⁵ s⁷⁶ d⁷⁷ k⁷⁸ h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ (SEQ ID NO: 238)
k⁷² d⁷³ g⁷⁴ s⁷⁵ s⁷⁶ d⁷⁷ k⁷⁸ h⁷⁹ (SEQ ID NO: 239)
k⁷² d⁷³ g⁷⁴ s⁷⁵ s⁷⁶ d⁷⁷ k⁷⁸ h⁷⁹ k⁸⁰ (SEQ ID NO: 240)
k⁷² d⁷³ g⁷⁴ s⁷⁵ s⁷⁶ d⁷⁷ k⁷⁸ h⁷⁹ k⁸⁰ d⁸¹ k⁸² (SEQ ID NO: 241)
k⁷² d⁷³ g⁷⁴ s⁷⁵ s⁷⁶ d⁷⁷ k⁷⁸ h⁷⁹ k⁸⁰ d⁸¹ k⁸² h⁸³ (SEQ ID NO: 242)
k⁷⁸ h⁷⁹ k⁸⁰ d⁸¹ k⁸² h⁸³ k⁸⁴ (SEQ ID NO: 243)
k⁷⁸ h⁷⁹ k⁸⁰ d⁸¹ k⁸² h⁸³ k⁸⁴ d⁸⁵ r⁸⁶ d⁸⁷ k⁸⁸ (SEQ ID NO: 244)
h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ i⁹⁶ r⁹⁷ a⁹⁸ a⁹⁹ g¹⁰⁰ d¹⁰¹ a¹⁰² k¹⁰³ (SEQ ID NO: 245)
h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ i⁹⁶ r⁹⁷ a⁹⁸ a⁹⁹ g¹⁰⁰ d¹⁰¹ a¹⁰² k¹⁰³ i¹⁰⁴ k¹⁰⁵ (SEQ ID NO: 246)
h⁷⁹ k⁸⁰ d⁸¹ k⁸² h⁸³ k⁸⁴ d⁸⁵ r⁸⁶ d⁸⁷ k⁸⁸ (SEQ ID NO: 247)
h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ (SEQ ID NO: 248)
h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² (SEQ ID NO: 249)
h⁷⁹ k⁸⁰ d⁸¹ k⁸² h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ (SEQ ID NO: 250)
k⁸⁰ d⁸¹ k⁸² h⁸³ k⁸⁴ d⁸⁵ r⁸⁶ d⁸⁷ k⁸⁸ (SEQ ID NO: 251)
k⁸⁰ d⁸¹ k⁸² h⁸³ k⁸⁴ d⁸⁵ r⁸⁶ d⁸⁷ k⁸⁸ e⁸⁹ k⁹⁰ (SEQ ID NO: 252)
k⁸² h⁸³ k⁸⁴ d⁸⁵ r⁸⁶ d⁸⁷ k⁸⁸ (SEQ ID NO: 253)
k⁸² h⁸³ k⁸⁴ d⁸⁵ r⁸⁶ d⁸⁷ k⁸⁸ e⁸⁹ k⁹⁰ (SEQ ID NO: 254)
k⁸² h⁸³ k⁸⁴ d⁸⁵ r⁸⁶ d⁸⁷ k⁸⁸ e⁸⁹ k⁹⁰ r⁹¹ k⁹² (SEQ ID NO: 255)
h⁸³ k⁸⁴ d⁸⁵ r⁸⁶ d⁸⁷ k⁸⁸ e⁸⁹ k⁹⁰ (SEQ ID NO: 256)
h⁸³ k⁸⁴ d⁸⁵ r⁸⁶ d⁸⁷ k⁸⁸ e⁸⁹ k⁹⁰ r⁹¹ k⁹² (SEQ ID NO: 257)
h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ i⁹⁶ r⁹⁷ a⁹⁸ a⁹⁹ g¹⁰⁰ d¹⁰¹ a¹⁰² k¹⁰³ (SEQ ID NO: 258)
h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ i⁹⁶ r⁹⁷ a⁹⁸ a⁹⁹ g¹⁰⁰ d¹⁰¹ a¹⁰² k¹⁰³ i¹⁰⁴ k¹⁰⁵ (SEQ ID NO: 259)
h⁸³ k⁸⁴ d⁸⁵ r⁸⁶ d⁸⁷ k⁸⁸ e⁸⁹ k⁹⁰ r⁹¹ k⁹² e⁹³ e⁹⁴ k⁹⁵ (SEQ ID NO: 260)
k²¹⁸ w²¹⁹ k²²⁰ f²²¹ l²²² e²²³ h²²⁴ k²²⁵ (SEQ ID NO: 275)
h²²⁴ k²²⁵ g²²⁶ p²²⁷ v²²⁸ f²²⁹ a²³⁰ p²³¹ p²³² y²³³ e²³⁴ p²³⁵ l²³⁶ p²³⁷ e²³⁸ g²³⁹ v²⁴⁰ k²⁴¹ f²⁴² y²⁴³ y²⁴⁴ d²⁴⁵ g²⁴⁶ k²⁴⁷ (SEQ ID NO: 276)
k²⁴⁷ v²⁴⁸ m²⁴⁹ k²⁵⁰ l²⁵¹ s²⁵² p²⁵³ k²⁵⁴ a²⁵⁵ e²⁵⁶ e²⁵⁷ v²⁵⁸ a²⁵⁹ t²⁶⁰ f²⁶¹ f²⁶² a²⁶³ k²⁶⁴ m²⁶⁵ l²⁶⁶ d²⁶⁷ h²⁶⁸ SEQ ID NO: 282)
k²⁵⁴ a²⁵⁵ e²⁵⁶ e²⁵⁷ v²⁵⁸ a²⁵⁹ t²⁶⁰ f²⁶¹ f²⁶² a²⁶³ k²⁶⁴ m²⁶⁵ l²⁶⁶ d²⁶⁷ h²⁶⁸ (SEQ ID NO: 283).

The following Replikin sequences were identified in the mid-molecule of Accession Number Q9WUL0:
k²⁶⁴ m²⁶⁵ l²⁶⁶ d²⁶⁷ h²⁶⁸ e²⁶⁹ y²⁷⁰ t²⁷¹ t²⁷² k²⁷³ (SEQ ID NO: 284)
h²⁶⁸ e²⁶⁹ y²⁷⁰ t²⁷¹ t²⁷² k²⁷³ e²⁷⁴ i²⁷⁵ f²⁷⁶ r²⁷⁷ k²⁷⁸ n²⁷⁹ f²⁸⁰ f²⁸¹ k²⁸² (SEQ ID NO: 285)
h²⁶⁸ e²⁶⁹ y²⁷⁰ t²⁷¹ t²⁷² k²⁷³ e²⁷⁴ i²⁷⁵ f²⁷⁶ r²⁷⁷ k²⁷⁸ n²⁷⁹ f²⁸⁰ f²⁸¹ k²⁸² d²⁸³ w²⁸⁴ r²⁸⁵ k²⁸⁶ (SEQ ID NO: 286)
k³²⁶ l³²⁷ k³²⁸ i³²⁹ k³³⁰ e³³¹ e³³² n³³³ e³³⁴ k³³⁵ l³³⁶ l³³⁷ k³³⁸ e³³⁹ y³⁴⁰ g³⁴¹ f³⁴² c³⁴³ v³⁴⁴ m³⁴⁵ d³⁴⁶ n³⁴⁷ h³⁴⁸ (SEQ ID NO: 294)
k³²⁸ i³²⁹ k³³⁰ e³³¹ e³³² n³³³ e³³⁴ k³³⁵ l³³⁶ l³³⁷ k³³⁸ e³³⁹ y³⁴⁰ g³⁴¹ f³⁴² c³⁴³ v³⁴⁴ m³⁴⁵ d³⁴⁶ n³⁴⁷ h³⁴⁸ (SEQ ID NO: 296) k³³⁰ e³³¹ e³³² n³³³ e³³⁴ k³³⁵ l³³⁶ l³³⁷ k³³⁸ e³³⁹ y³⁴⁰ g³⁴¹ f³⁴² c³⁴³ v³⁴⁴ m³⁴⁵ d³⁴⁶ n³⁴⁷ h³⁴⁸ (SEQ ID NO: 298)
k³⁹³ v³⁹⁴ p³⁹⁵ s³⁹⁶ p³⁹⁷ p³⁹⁸ p³⁹⁹ g⁴⁰⁰ h⁴⁰¹ k⁴⁰² (SEQ ID NO: 302)
k³⁹³ v³⁹⁴ p³⁹⁵ s³⁹⁶ p³⁹⁷ p³⁹⁸ p³⁹⁹ g⁴⁰⁰ h⁴⁰¹ k⁴⁰² w⁴⁰³ k⁴⁰⁴ e⁴⁰⁵ v⁴⁰⁶ r⁴⁰⁷ h⁴⁰⁸ (SEQ ID NO: 303)
h⁴⁰¹ k⁴⁰² w⁴⁰³ k⁴⁰⁴ e⁴⁰⁵ v⁴⁰⁶ r⁴⁰⁷ h⁴⁰⁸ d⁴⁰⁹ n⁴¹⁰ k⁴¹¹ (SEQ ID NO: 304)
k⁴⁰² w⁴⁰³ k⁴⁰⁴ e⁴⁰⁵ v⁴⁰⁶ r⁴⁰⁷ h⁴⁰⁸ d⁴⁰⁹ n⁴¹⁰ k⁴¹¹ (SEQ ID NO: 306)
k⁴⁰⁴ e⁴⁰⁵ v⁴⁰⁶ r⁴⁰⁷ h⁴⁰⁸ d⁴⁰⁹ n⁴¹⁰ k⁴¹¹ (SEQ ID NO: 307)

The following Replikin sequences were identified in the carboxy terminal of Accession Number Q9WUL0:

h⁶³⁴ q⁶³⁵ r⁶³⁶ a⁶³⁷ p⁶³⁸ p⁶³⁹ k⁶⁴⁰ t⁶⁴¹ f⁶⁴² e⁶⁴³ k⁶⁴⁴ s⁶⁴⁵ m⁶⁴⁶ m⁶⁴⁷ n⁶⁴⁸ l⁶⁴⁹ q⁶⁵⁰ s⁶⁵¹ k⁶⁵² (SEQ ID NO: 322)

Within Accession Number Q9WUL0, which is 767 amino acids in length, 328 Replikin sequences were identified. The Replikin Count of the entire sequence was found to be (328/767)* 100 or 42.8 Replikin sequences per 100 amino acid residues.

A Replikin Peak Gene was identified within the amino acid sequence of Accession Number Q9WUL0 from residue 22 through residue 95 with a length of 74 amino acids. Within the Replikin Peak Gene, 234 continuous and/or overlapping Replikin sequences were identified. The Replikin Count of the Replikin Peak Gene was calculated to be (234/74)*100 or 316 Replikin sequences per 100 amino acid residues.

Because Accession Number Q9WUL0 had the highest Replikin Count in its Replikin Peak Gene among all of the other rat glioblastoma sequences queried at www.pubmed.com (and because the glioblastoma containing the topoisomerase protein reported at Accession Number Q9WUL0 was predicted to have the relative fastest rate of growth and highest lethality), Replikin sequences from Accession Number Q9WUL0 were chosen for design of a vaccine to be tested in the rat model of glioblastoma. Rats are accepted in the art as a good model for development of human glioblastoma therapies.

A vaccine was designed by choosing thirty-two Replikin sequences from the Replikin Peak Gene wherein each of the Replikin sequences had an amino acid length of from seven to sixteen amino acid residues. Five additional Replikin sequences (from eight to fifteen amino acid residues in length) identified in the mid-molecule portion of the gene were added. Finally a Replikin sequence originally identified in human glioblastoma (kagvaflhkk (SEQ ID NO: 330)) was added. SEQ ID NO: 330 is the original prototype Replikin sequence, from which an algorithm was derived by which all other Replikin sequences were identified. The original prototype glioma Replikin Sequence, SEQ ID NO: 330, was first identified in the malignin oncoprotein in glioblastoma in humans and was observed to be related to rapid replication of glioblastoma cells. *See, e.g.*, U.S. Patent No. 7,420,028 for a description of the identification and isolation of SEQ ID NO: 330 as well as a description of the relationship of SEQ ID NO: 330 to rapid replication in glioblastoma cells.

The vaccine as designed contained the following thirty-eight Replikin sequences:
k a g v a f l h k k (SEQ ID NO: 330)
h⁴⁰¹ k⁴⁰² w⁴⁰³ k⁴⁰⁴ e⁴⁰⁵ v⁴⁰⁶ r⁴⁰⁷ h⁴⁰⁸ d⁴⁰⁹ n⁴¹⁰ k⁴¹¹ (SEQ ID NO: 304)
k³⁹³ v³⁹⁴ p³⁹⁵ s³⁹⁶ p³⁹⁷ p³⁹⁸ p³⁹⁹ g⁴⁰⁰ h⁴⁰¹ k⁴⁰² (SEQ ID NO: 302)
k²⁶⁴ m²⁶⁵ l²⁶⁶ d²⁶⁷ h²⁶⁸ e²⁶⁹ y²⁷⁰ t²⁷¹ t²⁷² k²⁷³ (SEQ ID NO: 284)
k²⁵⁴ a²⁵⁵ e²⁵⁶ e²⁵⁷ v²⁵⁸ a²⁵⁹ t²⁶⁰ f²⁶¹ f²⁶² a²⁶³ k²⁶⁴ m²⁶⁵ l²⁶⁶ d²⁶⁷ h²⁶⁸ (SEQ ID NO: 283)
k²¹⁸ w²¹⁹ k²²⁰ f²²¹ l²²² e²²³ h²²⁴ k²²⁵ (SEQ ID NO: 275)
h⁸³ k⁸⁴ d⁸⁵ r⁸⁶ d⁸⁷ k⁸⁸ e⁸⁹ k⁹⁰ (SEQ ID NO: 256)
k⁸² h⁸³ k⁸⁴ d⁸⁵ r⁸⁶ d⁸⁷ k⁸⁸ (SEQ ID NO: 253)
k⁸⁰ d⁸¹ k⁸² h⁸³ k⁸⁴ d⁸⁵ r⁸⁶ d⁸⁷ k⁸⁸ (SEQ ID NO: 251)
h⁷⁹ k⁸⁰ d⁸¹ k⁸² h⁸³ k⁸⁴ d⁸⁵ r⁸⁶ d⁸⁷ k⁸⁸ (SEQ ID NO: 247)
k⁷⁸ h⁷⁹ k⁸⁰ d⁸¹ k⁸² h⁸³ k⁸⁴ (SEQ ID NO: 243)
k⁷² d⁷³ g⁷⁴ s⁷⁵ s⁷⁶ d⁷⁷ k⁷⁸ h⁷⁹ (SEQ ID NO: 239)
h⁷¹ k⁷² d⁷³ g⁷⁴ s⁷⁵ s⁷⁶ d⁷⁷ k⁷⁸ (SEQ ID NO: 231)
k⁷⁰ h⁷¹ k⁷² d⁷³ g⁷⁴ s⁷⁵ s⁷⁶ d⁷⁷ k⁷⁸ (SEQ ID NO: 225)
k⁶⁸ t⁶⁹ k⁷⁰ h⁷¹ k⁷² d⁷³ g⁷⁴ s⁷⁵ s⁷⁶ d⁷⁷ k⁷⁸ (SEQ ID NO: 222)
k⁶⁶ e⁶⁷ k⁶⁸ t⁶⁹ k⁷⁰ h⁷¹ k⁷² (SEQ ID NO: 219)
k⁶⁴ e⁶⁵ k⁶⁶ e⁶⁷ k⁶⁸ t⁶⁹ k⁷⁰ h⁷¹ (SEQ ID NO: 215)
h⁶³ k⁶⁴ e⁶⁵ k⁶⁶ e⁶⁷ k⁶⁸ t⁶⁹ k⁷⁰ h⁷¹ k⁷² d⁷³ g⁷⁴ s⁷⁵ s⁷⁶ d⁷⁷ k⁷⁸ (SEQ ID NO: 207)
k⁶² h⁶³ k⁶⁴ e⁶⁵ k⁶⁶ e⁶⁷ k⁶⁸ (SEQ ID NO: 197
k⁶¹ k⁶² h⁶³ k⁶⁴ e⁶⁵ k⁶⁶ e⁶⁷ k⁶⁸ (SEQ ID NO: 192)
k⁵⁷ d⁵⁸ s⁵⁹ e⁶⁰ k⁶¹ k⁶² h⁶³ k⁶⁴ (SEQ ID NO: 187)
h⁵⁶ k⁵⁷ d⁵⁸ s⁵⁹ e⁶⁰ k⁶¹ k⁶² h⁶³ k⁶⁴ (SEQ ID NO: 172)
k⁵⁰ h⁵¹ s⁵² n⁵³ s⁵⁴ e⁵⁵ h⁵⁶ k⁵⁷ d⁵⁸ s⁵⁹ e⁶⁰ k⁶¹ k⁶² h⁶³ (SEQ ID NO: 155)
k⁴⁸ s⁴⁹ k⁵⁰ h⁵¹ s⁵² n⁵³ s⁵⁴ e⁵⁵ h⁵⁶ k⁵⁷ (SEQ ID NO: 149)
k⁴⁴ d⁴⁵ r⁴⁶ e⁴⁷ k⁴⁸ s⁴⁹ k⁵⁰ h⁵¹ (SEQ ID NO: 143)
k⁴² d⁴³ k⁴⁴ d⁴⁵ r⁴⁶ e⁴⁷ k⁴⁸ s⁴⁹ k⁵⁰ h⁵¹ (SEQ ID NO: 137)
k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 131)
k³⁹ k⁴⁰ d⁴¹ k⁴² d⁴³ k⁴⁴ d⁴⁵ r⁴⁶ e⁴⁷ k⁴⁸ s⁴⁹ k⁵⁰ h⁵¹ (SEQ ID NO: 125)
h³⁸ k³⁹ k⁴⁰ d⁴¹ k⁴² d⁴³ k⁴⁴ d⁴⁵ r⁴⁶ e⁴⁷ k⁴⁸ (SEQ ID NO: 113)
k³⁶ e³⁷ h³⁸ k³⁹ k⁴⁰ d⁴¹ k⁴² (SEQ ID NO: 104)
h³⁵ k³⁶ e³⁷ h³⁸ k³⁹ k⁴⁰ d⁴¹ k⁴² (SEQ ID NO: 90)
h³³ r³⁴ h³⁵ k³⁶ e³⁷ h³⁸ k³⁹ k⁴⁰ d⁴¹ k⁴² (SEQ ID NO: 77)
k²⁹ d³⁰ r³¹ e³² h³³ r³⁴ h³⁵ k³⁶ (SEQ ID NO: 69)
h²⁸ k²⁹ d³⁰ r³¹ e³² h³³ r³⁴ h³⁵ k³⁶ (SEQ ID NO: 56)
k²⁷ h²⁸ k²⁹ d³⁰ r³¹ e³² h³³ r³⁴ h³⁸ k³⁶ (SEQ ID NO: 50)
h²⁴ k²⁵ d²⁶ k²⁷ h²⁸ k²⁹ d³⁰ r³¹ e³² h³³ r³⁴ h³⁵ k³⁶ (SEQ ID NO: 38)
k²³ h²⁴ k²⁵ d²⁶ k²⁷ h²⁸ k²⁹ (SEQ ID NO: 34)
h²² k²³ h²⁴ k²⁵ d²⁶ k²⁷ h²⁸ k²⁹ (SEQ ID NO: 17)

A vaccine comprising the thirty-eight Replikin sequences and a pharmaceutically acceptable carrier is administered to rats either intravenously or intramuscularly. An immune response is detected. The vaccine is then used as a therapy against growth of glioblastoma in rats.

### Example 6

### Survey of Glioblastoma Tissue to Develop Baseline Data for Case-by-Case Prognosis

More than 500 frozen tissue samples from glioblastoma tumors are analyzed to determine the Replikin Count in the topoisomerase gene in each tumor. A Replikin Peak Gene is identified in each topoisomerase gene. The Replikin Count in each Replikin Peak Gene is recorded and compared with the measured lethality of the malignancy in the patient suffering from each tumor and/or the previously recorded histopathologically-determined degree of malignancy for each tumor. A mean Replikin Count plus standard deviation is determined among the more than 500 samples or a correlation between the Replikin Count and the measured lethality of each malignancy is determined by any method. Such method may include, for example, a Replikin Count Expansion Index analysis or a regression analysis or any other method of analysis or the histopathologically-determined degree of malignancy.

A Replikin Count of a malignancy having an unknown lethality may then be compared to the mean Replikin Count plus one standard deviation of the mean. If the Replikin Count of the malignancy having an unknown lethality is greater than the mean Replikin Count plus one standard deviation of the mean, the malignancy of unknown lethality is predicted to have a greater lethality than the mean measured lethality of the more than 500 samples. If the Replikin Count of the malignancy having an unknown lethality is less than the mean Replikin Count minus one standard deviation of the mean, the malignancy of unknown lethality is predicted to have a lower lethality than the mean measured lethality of the more than 500 samples. To provide other kinds of precision in the prediction of lethality, the more than 500 samples may be grouped into a plurality of groups having related lethalities or related types. Mean Replikin Counts plus standard deviation of the mean may be determined for some or all of each these groups. A Replikin Count of a malignancy having an unknown lethality may then be compared to the mean Replikin Count plus or minus one standard deviation of the mean of a particular group having a related lethality.

A regression analysis may also be performed on the Replikin Counts of the more than 500 samples where Replikin Count is compared to measured lethality. The regression analysis may be used to create a predictive formula by which Replikin Count in the Replikin Peak Gene of the topoisomerase gene in individual cases of glioblastoma may be used to provide a predicted lethality prognosis for any biopsy or resection of a glioblastoma growth in a patient suffering from glioblastoma. The statistical formula provides a measure of lethality such as length of life expectancy or expected rate of growth based on the Replikin Count of the Replikin Peak Gene of the topoisomerase of the glioblastoma.

Two or more regression curves may be applied to data from a survey. For example, among the more than 500 samples, samples may be grouped into benign and malignant tumors. A first regression analysis may be performed on the group of benign tumors and a second regression analysis may be performed on the group of malignant tumors. Further, a regression curve may be continuous or broken. It further may reflect types or subtypes of malignancies.

The efficacy of histopathologically-determined degree of malignancy is also determined by comparing the histopathological degree of malignancy previously qualitatively determined by pathologists with a quantitative measure of lethality based on quantitative Replikin Counts.

Additional data is created for other proteins expressed in glioblastoma. The protein, protein fragment, or genome segment of the glioblastoma that provides the strongest correlation is chosen as a standard for determination of lethality using Replikin Count.

A kit comprising the Replikin Count Expansion Index or predictive formula for determining prognosis based on Replikin Count is provided to laboratories and practitioners so that prognosis based on Replikin sequence analysis of individual malignancies may be provided directly from the practitioner or laboratory to the patient. The kit may comprise software containing the Replikin Count Expansion Index or regression formula.

### Example 7

### Comparison of the Replikin Concentration of Four Strains of Taura Syndrome Virus by an Independent Laboratory

The Replikin concentrations of the expressed protein sequences of four taura syndrome virus (TSV) isolates from Hawaii, Belize, Thailand and Venezuela, respectively, were examined. The virulence of each isolate was initially quantitatively ranked based solely on the order of the Replikin concentrations. This quantitative ranking of virulence based on Replikin concentration was undertaken by researchers having no knowledge of the virulence of the isolates based on bioassay methods (such as cumulative survival and time to 50% mortality) undertaken simultaneously.

An independent laboratory simultaneously undertook bioassay comparisons of the four TSV isolates. The independent laboratory had no knowledge of the order of the Replikin concentrations of the TSV isolates. In the independent laboratory, virulence was compared in infected *Litopenaeus vannamei* (Kona stock, Oceanic Institute, Hawaii) shrimp subject to per os viral infection. Cumulative survival results and time to 50% mortality results demonstrated the Belize isolate to be the most virulent, the Thailand isolate to be the second most virulent, the Hawaii isolate to be the third most virulent, and the Venezuela isolate to be the least virulent. TSV infection was confirmed as the cause of death in each bioassay by positive reactions in RT-PCR detection and by the appearance of characteristic lesions observed in histological analysis. A full description of the investigative methods of the shrimp trials is set forth in Example 1 of U.S. Patent Appln. Ser. No. 12/108,458 filed April 23, 2008.

Upon comparison of Replikin concentration for each isolate with cumulative survival of challenged shrimp and 50% mortality of challenged shrimp, a quantitative and substantially linear correlation was observed. The results are set forth in Table 2 below.

Table 2 provides the time to 50% mortality for shrimp challenged with each isolate. Fifty percent mortality resulting from TSV infection with the isolate of Belize, Thailand, Hawaii and Venezuela, respectively, were 2.8, 3.5, 4.5 and 7 days. Table 2 also provides the cumulative mortality of shrimp fifteen days following challenge. Cumulative mortality at fifteen days for shrimp challenged with the Belize, Thailand, Hawaii, and Venezuelans isolates, respectively, was 100%, 80%, 78%, and 58%, respectively. Statistical differences between the Replikin concentration for each isolate are significant at a level of p<0.001. Figures 3 and 4 provide graphical illustration of the data in Table 2.

**Table 2. Results from per os TSV challenge in SPF Litopenaeus vannamei (Kona stock)**

| **TSV isolate** | **GenBank No. (ORF1)** | **Cumulative Mortality (%)(Mean)** | **Day of 50% mortality** | **Blind Replikin Concentration** |
|---|---|---|---|---|
| Belize | AAT81157 | 100 | 2.8 | 3.5 |
| Thailand | AAY56363 | 80 | 3.5 | 3.4 |
| US-Hawaii | AAK72220 | 78 | 4.5 | 3.3 |
| Venezuela | ABB17263 | 58 | 7.0 | 3.0 |

## Claims

1. A method of predicting the relative rate of growth of at least one first malignancy of an unknown or known type of malignancy comprising:
comparing a Replikin Count of the at least one first malignancy with a Replikin Count of at least one second malignancy of an unknown type or of a different type of malignancy than the at least one first malignancy; and
predicting the at least one first malignancy to have a relative rate of growth that is faster than the relative rate of growth of the at least one second malignancy if the Replikin Count of the at least one first malignancy is greater than the Replikin Count of the at least one second malignancy,
wherein a Replikin Count is the number of Replikin sequences per 100 amino acids in an amino acid sequence and a Replikin sequence is determined by identifying an amino acid sequence of 7 to 50 amino acid residues with at least one lysine residue located at a first terminus of said amino acid sequence and at least one lysine residue or at least one histidine residue located at a second terminus of said amino acid sequence wherein the Replikin sequence comprises (1) a first lysine residue located six to ten residues from a second lysine residue; (2) at least one histidine residue; and (3) at least 6% lysine residues, and
wherein said method is performed by a computer system.

2. The method of claim 1, further comprising: determining the Replikin Count of the at least one first malignancy and the Replikin Count of the at least one second malignancy before the comparing step.

3. The method of claim 1, wherein said at least one first malignancy is a metastasis of said at least one second malignancy, is a malignancy of unknown type or unknown origin in a subject suffering from said at least one second malignancy, or is a malignant cell of the same malignancy as said at least one second malignancy, or wherein said at least one first malignancy and at least one second malignancy are metastases of a third malignancy.

4. The method of claim 1, wherein the Replikin Count of said at least one first malignancy is the Replikin Count of a Replikin Peak Gene of the at least one first malignancy and the Replikin Count of said at least one second malignancy is a Replikin Count of a Replikin Peak Gene of said at least one second malignancy.

5. The method of claim 1, wherein (1) said Replikin Count of said at least one second malignancy is a mean Replikin Count of a plurality of malignancies or a mean Replikin Count of a plurality of cells of the at least one second malignancy; (2) said Replikin Count of said at least one first malignancy is a mean Replikin Count of a plurality of malignancies or a mean Replikin Count of a plurality of cells of the at least one first malignancy; or (3) both said Replikin Count of said at least one second malignancy is a mean Replikin Count of a plurality of malignancies or a mean Replikin Count of a plurality of cells of the at least one second malignancy, and said Replikin Count of said at least one first malignancy is a mean Replikin Count of a plurality of malignancies or a mean Replikin Count of a plurality of cells of the at least one first malignancy.

6. The method of claim 1, wherein said Replikin Count of said at least one second malignancy is a mean, median, or other average Replikin Count of a plurality of malignancies, wherein said plurality of malignancies represents a survey of malignancies.

7. The method of claim 5, wherein the Replikin Count of said at least one first malignancy or the mean Replikin Count of said at least one first malignancy is greater than a mean Replikin Count of said at least one second malignancy plus the standard deviation of the mean Replikin Count of said at least one second malignancy.

8. The method of claim 1, wherein said relative rate of growth correlates with a relative lethality.

9. The method of claim 1, wherein said Replikin Count of said at least one first malignancy is a Replikin Count of an expressed protein, protein fragment or peptide isolated from a cell of said at least one first malignancy or a Replikin Count of the genome, a gene, a gene fragment, or any other nucleic acid sequence isolated from a cell of said at least one first malignancy; and wherein said Replikin Count of said at least one second malignancy is a Replikin Count of an expressed protein, protein fragment or peptide isolated from a cell of said at least one second malignancy or a Replikin Count of the genome, a gene, a gene fragment, or any other nucleic acid sequence isolated from a cell of said at least one second malignancy.

10. The method of claim 1, wherein said Replikin Count of said at least one first malignancy is the highest Replikin Count among a plurality of Replikin Counts of a first plurality of malignancies of a first type, and said Replikin Count of said at least one second malignancy is the highest Replikin Count among a plurality of Replikin Counts of a second plurality of malignancies of a second type.

11. The method of claim 10, further comprising:
comparing a Replikin Count of the Replikin Peak Gene of the malignancy having the highest Replikin Count among a plurality of Replikin Counts of said malignancies of the first type to the Replikin Count of the Replikin Peak Gene of the malignancy having the highest Replikin Count among a plurality of Replikin Counts of said malignancies of the second type; and
if said malignancies of the first type has a higher Replikin Count of said Replikin Peak Gene than said malignancies of the second type, then said malignancies of the first type is predicted to have a greater lethality than said malignancies of the second type.

12. The method of claim 1, wherein said at least one first malignancy is a thyroid malignancy, a prostate malignancy, a breast malignancy, a urinary bladder malignancy, a uterine corpus malignancy, a uterine cervix malignancy, a colon malignancy, an ovarian malignancy, a malignancy of the oral cavity, a lymphocytic leukemia malignancy, a multiple myeloma malignancy, a gastric malignancy, a non-small cell lung carcinoma malignancy, or a glioblastoma malignancy.

13. The method of claim 1, wherein the method is performed by a processor and wherein at least one Replikin Count or at least one representation of said prediction of the relative rate of growth of said at least one first malignancy is outputted to a user or display.

14. A machine-readable storage medium having stored thereon executable instructions that, when executed by a processor, performs the method of claim 1.

15. A method of determining the relative rate of increase in the relative rate of growth of at least one first malignancy as compared to the relative rate of increase in the relative rate of growth of at least one second malignancy comprising:
comparing the standard deviation of the mean Replikin Count of a plurality of cells of said at least one first malignancy to the standard deviation of the mean Replikin Count of a plurality of cells of said at least one second malignancy; and
predicting that the relative increase in the relative rate of growth of said at least one first malignancy is greater than the relative rate of increase in the relative rate of growth of said at least one second malignancy if the standard deviation of the mean Replikin Count of the plurality of cells of said at least one first malignancy is greater than the standard deviation of the mean Replikin Count of a plurality of cells of said at least one second malignancy,
wherein a Replikin Count is the number of Replikin sequences per 100 amino acids in an amino acid sequence and a Replikin sequence is determined by identifying an amino acid sequence of 7 to 50 amino acid residues with at least one lysine residue located at a first terminus of said amino acid sequence and at least one lysine residue or at least one histidine residue located at a second terminus of said amino acid sequence wherein the Replikin sequence comprises (1) a first lysine residue located six to ten residues from a second lysine residue; (2) at least one histidine residue; and (3) at least 6% lysine residues, and
wherein said method is performed by a computer system.

16. The method of claim 15, wherein said at least one first malignancy is a metastasis of said at least one second malignancy or wherein said at least one first malignancy is a malignancy of unknown origin or unknown type in a subject suffering from said at least one second malignancy.

17. The method of claim 15, wherein said at least one first malignancy and said at least one second malignancy are both metastases of a third malignancy.

18. The method of claim 15, wherein said at least one first malignancy is a plurality of malignancies differing from said at least one second malignancy, said at least one second malignancy is a plurality of malignancies differing from said at least one first malignancy, or said at least one first malignancy is a plurality of malignancies differing from said at least one second malignancy and said at least one second malignancy is a plurality of malignancies differing from said at least one first malignancy.

19. The method of claim 18, wherein said at least one first malignancy is a plurality of malignancies of a first type of malignancy and said at least one second malignancy is a plurality of malignancies of a second type of malignancy.

20. The method of claim 1 further comprising predicting an expansion of a malignancy comprising:
determining a mean Replikin Count and a standard deviation of said mean Replikin Count for a plurality of cells of said first malignancy of claim 1 for a first time period in a first anatomic region or for a first plurality of malignancies including said first malignancy of claim 1;
determining a Replikin Count of said second malignancy of claim 1 wherein said Replikin Count for said second malignancy is defined as a Replikin Count of at least one other cell of the first malignancy, at least one cell of a metastasis of the first malignancy, or at least one cell of a malignancy of unknown origin or unknown type in a subject suffering from the first malignancy at a second time period and/or in a second anatomic region, wherein said second time period is different from said first time period and/or said second anatomic region is different from said first anatomic region, or determining a Replikin Count of at least one cell of a second malignancy in a different subject; and
predicting an expansion of said first malignancy, said metastasis of said first malignancy, said malignancy of unknown origin or unknown type, or said second malignancy in a different subject if the Replikin Count of said at least one cell is greater than the mean Replikin Count of the plurality of cells of said malignancy from said first time period and from said first anatomic region plus one standard deviation of the mean Replikin Count of the plurality of cells of said malignancy from said first time period and from said first anatomic region or if the Replikin Count of said at least one cell is greater than the mean Replikin Count of the first plurality of malignancies plus one standard deviation of the mean Replikin Count of the first plurality of malignancies, and
wherein said method is performed by a computer system.

21. The method of claim 20, wherein said at least one other cell of the first malignancy, at least one cell of a metastasis of the first malignancy, or at least one cell of a malignancy of unknown origin or unknown type in a subject suffering from the first malignancy at a second time period and/or in a second anatomic region, or said at least one cell of a second malignancy in a different subject is a plurality of cells from said second time period and/or said second anatomic region or said second malignancy in a different subject, and the Replikin Count of each cell of the plurality of cells from said second time period and/or second anatomic region or said second malignancy in a different subject is compared separately to said mean Replikin Count plus one standard deviation of said mean Replikin Count.

22. The method of claim 21, wherein the expansion of said malignancy in said second time period and/or said second anatomic region or the expansion of said second malignancy in a different subject is predicted if the number of Replikin Counts of said plurality of cells from said second period and/or said second anatomic region or the number of Replikin Counts of said second malignancy in a different subject that is greater than said mean Replikin Count of the plurality of cells from said first time period in said first anatomic region or from said first plurality of malignancies plus one standard deviation of the mean of the Replikin Count is greater than the number of Replikin Counts of said plurality of cells from said second time period and/or said second anatomic region or the number of Replikin Counts of said second malignancy in a different subject that is less than said mean Replikin Count of said plurality of cells from said first time period in said first anatomic region or from said first plurality of malignancies minus one standard deviation of the mean.

23. A kit for providing a prognosis of a patient suffering from a malignancy, said prognosis concerning the lethality of the malignancy, comprising a machine readable storage medium having stored thereon software for determining the lethality of a malignancy according to the method of claim 8, wherein said Replikin Count is determined in at least one cell of the malignancy.

24. The kit of claim 23, wherein said formula is derived from a survey of a plurality of malignancies of a first type of malignancy.

25. The kit of claim 24, wherein the first type of malignancy is a glioblastoma.

26. The method of claim 1 further comprising predicting the lethality of said first malignancy comprising:
determining a Replikin Count of said first malignancy;
performing a regression analysis with the Replikin Count of a plurality of second malignancies, including said second malignancy of claim 1, versus the lethality of said plurality of second malignancies; and
predicting the lethality of the first malignancy based on the regression analysis of the plurality of second malignancies, and
wherein said method is performed by a computer system.

27. The method of claim 26, wherein the regression analysis of the plurality of second malignancies is performed using the Replikin Count of each individual second malignancy of the plurality of second malignancies and a patient survival outcome for each individual second malignancy.

28. The method of claim 27, wherein the patient outcome is a length of survival of the patient following diagnosis of malignancy.

29. The method of claim 1 further comprising predicting the relative rate of growth of said at least one first malignancy wherein said at least one first malignancy is of an unknown or known type comprising:
isolating at least one expressed protein, protein fragment, or peptide from a cell of said at least one first malignancy or isolating the genome, at least one gene, at least one gene fragment, or at least one other nucleic acid sequence from the cell of the at least one first malignancy;
isolating at least one expressed protein, protein fragment, or peptide from a cell of said at least one second malignancy of claim 1 wherein said at least one second malignancy is of an unknown type or of a different type of malignancy than the at least one first malignancy or isolating the genome, at least one gene, at least one gene fragment, or at least one other nucleic acid sequence from the cell of the at least one second malignancy of claim 1;
determining a Replikin Count of said at least one first malignancy and a Replikin Count of said at least one second malignancy;
comparing the Replikin Count of the at least one first malignancy with the Replikin Count of the at least one second malignancy; and
predicting the at least one first malignancy to have a relative rate of growth that is faster than the relative rate of growth of the at least one second malignancy if the Replikin Count of the at least one first malignancy is greater than the Replikin Count of the at least one second malignancy.

## Patentansprüche

1. Verfahren zur Vorhersage der relativen Wachstumsrate von mindestens einer ersten bösartigen Erkrankung, wobei die bösartige Erkrankung unbekannter oder bekannter Art ist, wobei das Verfahren Folgendes umfasst:
Vergleichen eines Replikin-Auszählwerts der mindestens einen ersten bösartigen Erkrankung mit einem Replikin-Auszählwert mindestens einer zweiten bösartigen Erkrankung, die von unbekannter Art ist oder eine andere Art von bösartigen Erkrankungen als die mindestens eine erste bösartige Erkrankung darstellt; und
Vorhersagen, dass die mindestens eine erste bösartige Erkrankung eine relative Wachstumsrate aufweist, die schneller als die relative Wachstumsrate der mindestens einen zweiten bösartigen Erkrankung ist, falls der Replikin-Auszählwert der mindestens einen ersten bösartigen Erkrankung höher als der
Replikin-Auszählwert der mindestens einen zweiten bösartigen Erkrankung ist,
wobei es sich bei dem Replikin-Auszählwert um die Anzahl an Replikin-Sequenzen pro 100 Aminosäuren in einer Aminosäuresequenz handelt und eine Replikin-Sequenz bestimmt wird, indem eine Aminosäuresequenz mit 7 bis 50 Aminosäureresten identifiziert wird, bei der sich mindestens ein Lysinrest an einem ersten Ende der Aminosäuresequenz befindet und sich mindestens ein Lysinrest oder mindestens ein Histidinrest an einem zweiten Ende der Aminosäuresequenz befindet, wobei die Replikin-Sequenz (1) einen ersten Lysinrest, der sich sechs bis zehn Reste von einem zweiten Lysinrest entfernt befindet; (2) mindestens einen Histidinrest; und (3) mindestens 6 % Lysinreste umfasst, und
wobei das Verfahren mittels eines Computersystems durchgeführt wird.

2. Verfahren nach Anspruch 1, das weiterhin Folgendes umfasst: Bestimmen des Replikin-Auszählwerts der mindestens einen ersten bösartigen Erkrankung und des Replikin-Auszählwerts der mindestens einen zweiten bösartigen Erkrankung im Vorfeld des Vergleichsschrittes.

3. Verfahren nach Anspruch 1, wobei es sich bei der mindestens einen ersten bösartigen Erkrankung um eine Metastase der mindestens einen zweiten bösartigen Erkrankung, um eine bösartige Erkrankung unbekannter Art oder unbekannter Herkunft bei einem Patienten, der unter der mindestens einen zweiten bösartigen Erkrankung leidet, oder um eine bösartige Zelle derselben bösartige Erkrankung wie die mindestens eine zweite bösartigen Erkrankung handelt, oder wobei es sich bei der mindestens einen ersten bösartigen Erkrankung und mindestens einer zweiten bösartigen Erkrankung um Metastasen einer dritten bösartigen Erkrankung handelt.

4. Verfahren nach Anspruch 1, wobei es sich bei dem Replikin-Auszählwert der mindestens einen ersten bösartigen Erkrankung um den Replikin-Auszählwert eines Genabschnitts mit dem höchsten Replikinvorkommen der mindestens einen ersten bösartige Erkrankung handelt und es sich bei dem Replikin-Auszählwert der mindestens einen zweiten bösartigen Erkrankung um einen Replikin-Auszählwert eines Genabschnitts mit dem höchsten Replikinvorkommen der mindestens einen zweiten bösartigen Erkrankung handelt.

5. Verfahren nach Anspruch 1, wobei (1) es sich bei dem Replikin-Auszählwert der mindestens einen zweiten bösartigen Erkrankung um einen mittleren Replikin-Auszählwert einer Mehrzahl bösartiger Erkrankungen oder um einen mittleren Replikin-Auszählwerts einer Mehrzahl von Zellen der mindestens einen zweiten bösartigen Erkrankung handelt; (2) es sich bei dem Replikin-Auszählwert der mindestens einen ersten bösartigen Erkrankung um einen mittleren Replikin-Auszählwert einer Mehrzahl bösartiger Erkrankungen oder einen mittleren Replikin-Auszählwert einer Mehrzahl von Zellen der mindestens einen ersten bösartigen Erkrankung handelt; oder (3) es sich bei dem Replikin-Auszählwert der mindestens einen zweiten bösartigen Erkrankung um einen mittleren Replikin-Auszählwert einer Mehrzahl bösartiger Erkrankungen oder um einen mittleren Replikin-Auszählwert einer Mehrzahl von Zellen der mindestens einen zweiten bösartigen Erkrankung handelt, und es sich bei dem Replikin-Auszählwert der mindestens einen ersten bösartigen Erkrankung um einen mittleren Replikin-Auszählwert einer Mehrzahl bösartiger Erkrankungen oder um einen mittleren Replikin-Auszählwert einer Mehrzahl von Zellen der mindestens einen ersten bösartigen Erkrankung handelt.

6. Verfahren nach Anspruch 1, wobei es sich bei dem Replikin-Auszählwert der mindestens einen zweiten bösartigen Erkrankung um einen Mittelwert, Medianwert oder sonstigen Durchschnittswert des Replikin-Auszählwerts einer Mehrzahl bösartiger Erkrankungen handelt, wobei die Mehrzahl bösartiger Erkrankungen einer Bestandsaufnahme an bösartigen Erkrankungen entspricht.

7. Verfahren nach Anspruch 5, wobei der Replikin-Auszählwert der mindestens einen ersten bösartigen Erkrankung oder der mittlere Replikin-Auszählwert der mindestens einen ersten bösartigen Erkrankung größer als ein mittlerer Replikin-Auszählwert der mindestens einen zweiten bösartigen Erkrankung zuzüglich der Standardabweichung des mittleren Replikin-Auszählwerts der mindestens einen zweiten bösartigen Erkrankung ist.

8. Verfahren nach Anspruch 1, wobei die relative Wachstumsrate mit einer relativen Sterblichkeit korreliert ist.

9. Verfahren nach Anspruch 1, wobei es sich bei dem Replikin-Auszählwert der mindestens einen ersten bösartigen Erkrankung um einen Replikin-Auszählwert eines exprimierten Proteins, Proteinfragments oder Peptids, das aus einer Zelle der mindestens einen ersten bösartigen Erkrankung isoliert wurde, oder um einen Replikin-Auszählwert des Genoms, eines Gens, eines Genfragments oder einer beliebigen sonstigen Nukleinsäuresequenz, welche(s) aus einer Zelle der mindestens einen ersten bösartigen Erkrankung isoliert wurde, handelt; und wobei es sich bei dem Replikin-Auszählwert der mindestens einen zweiten bösartigen Erkrankung um einen Replikin-Auszählwert eines exprimierten Proteins, Proteinfragments oder Peptids, das aus einer Zelle der mindestens einen zweiten bösartigen Erkrankung isoliert wurde, oder um einen Replikin-Auszählwert des Genoms, eines Gens, eines Gen-Fragments oder einer beliebigen sonstigen Nukleinsäuresequenz, welche(s) aus einer Zelle der mindestens einen zweiten bösartigen Erkrankung isoliert wurde, handelt.

10. Verfahren nach Anspruch 1, wobei es sich bei dem Replikin-Auszählwert der mindestens einen ersten bösartigen Erkrankung um den höchsten Replikin-Auszählwert einer Mehrzahl von Replikin-Auszählwerten einer ersten Mehrzahl bösartiger Erkrankungen einer ersten Art handelt, und es sich bei dem Replikin-Auszählwert der mindestens einen zweiten bösartigen Erkrankung um den höchsten Replikin-Auszählwert einer Mehrzahl von Replikin-Auszählwerten einer zweiten Mehrzahl bösartiger Erkrankungen einer zweiten Art handelt.

11. Verfahren nach Anspruch 10, das weiterhin Folgendes umfasst:
Vergleich zwischen einem Replikin-Auszählwert des Genabschnitts mit dem höchsten Replikinvorkommen der bösartigen Erkrankung mit dem höchsten Replikin-Auszählwert aus einer Mehrzahl an Replikin-Auszählwerten der bösartigen Erkrankungen der ersten Art und dem Replikin-Auszählwert des Genabschnitts mit dem höchsten Replikinvorkommen der bösartigen Erkrankung mit dem höchsten Replikin-Auszählwert aus einer Mehrzahl an Replikin-Auszählwerten der bösartigen Erkrankungen der zweiten Art; und
falls die bösartigen Erkrankungen der ersten Art einen höheren Replikin-Auszählwert bezüglich des Genabschnitts mit dem höchsten Replikinvorkommen aufweisen als die bösartigen Erkrankungen der zweiten Art, Treffen der Vorhersage, dass die bösartigen Erkrankungen der ersten Art mit eine höhere Sterblichkeit als die bösartigen Erkrankungen der zweiten Art aufweisen.

12. Verfahren nach Anspruch 1, wobei es sich bei der mindestens einen ersten bösartigen Erkrankung um eine bösartige Erkrankung der Schilddrüse, eine bösartige Erkrankung der Prostata, eine bösartige Erkrankung der Brust, eine bösartige Erkrankung der Harnblase, eine bösartige Erkrankung des Gebärmutterkorpus, eine bösartige Erkrankung des Gebärmutterhalses, eine bösartige Erkrankung des Dickdarms, eine bösartige Erkrankung der Eierstöcke, eine bösartige Erkrankung der Mundhöhle, eine bösartige Erkrankung in Form einer lymphozytischen Leukämie, eine bösartige Erkrankung in Form eines multiplen Myeloms, ein bösartige Erkrankung in Form eines nicht-kleinzelligen Lungenkarzinoms oder eine bösartige Erkrankung in Form eines Glioblastoms handelt.

13. Verfahren nach Anspruch 1, wobei das Verfahren mittels eines Prozessors durchgeführt wird und wobei mindestens ein Replikin-Auszählwert oder mindestens eine Darstellung der Vorhersage der relativen Wachstumsrate der mindestens einen ersten bösartige Erkrankung dem Benutzer zur Verfügung gestellt wird oder auf einer Anzeige erscheint.

14. Maschinenlesbares Speichermedium, auf dem ausführbare Anweisungen gespeichert sind, deren Ausführung durch einen Prozessor die Durchführung des Verfahrens nach Anspruch 1 bewirkt.

15. Verfahren zur Bestimmung der relativen Rate der Zunahme der relativen Wachstumsrate von mindestens einer ersten bösartigen Erkrankung im Vergleich mit der relativen Rate der Zunahme der relativen Wachstumsrate von mindestens einer zweiten bösartigen Erkrankung, umfassend:
Vergleichen der Standardabweichung des mittleren Replikin-Auszählwerts einer Mehrzahl von Zellen der mindestens einen ersten bösartigen Erkrankung mit der Standardabweichung des mittleren Replikin-Auszählwerts einer Mehrzahl von Zellen der mindestens einen zweiten bösartigen Erkrankung; und
Vorhersagen, dass die relative Rate der Zunahme der relativen Wachstumsrate der mindestens einen ersten bösartigen Erkrankung höher als die relative Rate der Zunahme der relativen Wachstumsrate der mindestens einen zweiten bösartigen Erkrankung ist, falls die Standardabweichung des mittleren Replikin-Auszählwerts einer Mehrzahl von Zellen der mindestens einen ersten bösartigen Erkrankung größer als die Standardabweichung des mittleren Replikin-Auszählwerts einer Mehrzahl von Zellen der mindestens einen zweiten bösartigen Erkrankung ist,
wobei es sich bei dem Replikin-Auszählwert um die Anzahl an Replikin-Sequenzen pro 100 Aminosäuren in einer Aminosäuresequenz handelt und eine Replikin-Sequenz bestimmt wird, indem eine Aminosäuresequenz mit 7 bis 50 Aminosäureresten identifiziert wird, bei der sich mindestens ein Lysinrest an einem ersten Ende der Aminosäuresequenz befindet und sich mindestens ein Lysinrest oder mindestens ein Histidinrest an einem zweiten Ende der Aminosäuresequenz befindet, wobei die Replikin-Sequenz (1) einen ersten Lysinrest, der sich sechs bis zehn Reste von einem zweiten Lysinrest entfernt befindet; (2) mindestens einen Histidinrest; und (3) mindestens 6 % Lysinreste umfasst, und
wobei das Verfahren mittels eines Computersystems durchgeführt wird.

16. Verfahren nach Anspruch 15, wobei es sich bei der mindestens einen ersten bösartigen Erkrankung um eine Metastase der mindestens einen zweiten bösartigen Erkrankung handelt, oder wobei es sich bei der mindestens einen ersten bösartigen Erkrankung um eine bösartige Erkrankung unbekannter Herkunft oder unbekannter Art bei einem Patienten handelt, der unter der mindestens einen zweiten bösartigen Erkrankung leidet.

17. Verfahren nach Anspruch 15, wobei die mindestens eine erste bösartige Erkrankung und die mindestens eine zweite bösartige Erkrankung beide Metastasen einer dritten bösartigen Erkrankung sind.

18. Verfahren nach Anspruch 15, wobei es sich bei der mindestens einen ersten bösartigen Erkrankung um eine Mehrzahl bösartiger Erkrankungen handelt, die sich von der mindestens einen zweiten bösartigen Erkrankung unterscheiden, es sich bei der mindestens einen zweiten bösartigen Erkrankung um eine Mehrzahl bösartiger Erkrankungen handelt, die sich von der mindestens einen ersten bösartigen Erkrankung unterscheiden, oder es sich bei der mindestens einen ersten bösartigen Erkrankung um eine Mehrzahl bösartiger Erkrankungen handelt, die sich von der mindestens einen zweiten bösartigen Erkrankung unterscheiden, und es sich bei der mindestens einen zweiten bösartigen Erkrankung um eine Mehrzahl bösartiger Erkrankungen handelt, die sich von der mindestens einen ersten bösartigen Erkrankung unterscheiden.

19. Verfahren nach Anspruch 18, wobei es sich bei der mindestens einen ersten bösartigen Erkrankung um eine Mehrzahl bösartiger Erkrankungen einer ersten Art bösartiger Erkrankungen handelt und es sich bei der mindestens einen zweiten bösartigen Erkrankung um eine Mehrzahl bösartiger Erkrankungen einer zweiten Art bösartiger Erkrankungen handelt.

20. Verfahren nach Anspruch 1, das weiterhin die Vorhersage einer Ausbreitung einer bösartigen Erkrankung umfasst, wobei das Verfahren umfasst:
Bestimmen eines mittleren Replikin-Auszählwerts und einer Standardabweichung des mittleren Replikin-Auszählwerts für eine Mehrzahl von Zellen der ersten bösartigen Erkrankung nach Anspruch 1 über einen ersten Zeitraum in einer ersten anatomischen Region oder für eine erste Mehrzahl bösartiger Erkrankungen einschließlich der ersten bösartigen Erkrankung nach Anspruch 1;
Bestimmen eines Replikin-Auszählwerts der zweiten bösartigen Erkrankung nach Anspruch 1, wobei der Replikin-Auszählwert für die zweite bösartige Erkrankung einem Replikin-Auszählwert mindestens einer weiteren Zelle der ersten bösartigen Erkrankung, mindestens einer Zelle einer Metastase der ersten bösartigen Erkrankung oder mindestens einer Zelle einer bösartigen Erkrankung unbekannter Herkunft oder unbekannter Art bei einem Patienten entspricht, der zu einem zweiten Zeitraum und/oder in einer zweiten anatomischen Region unter der ersten bösartigen Erkrankung leidet, wobei sich der zweite Zeitraum vom ersten Zeitraum unterscheidet und/oder sich die zweite anatomische Region von der ersten anatomischen Region unterscheidet, oder Bestimmen eines Replikin-Auszählwerts von mindestens einer Zelle einer zweiten bösartigen Erkrankung bei einem anderen Patienten; und
Vorhersage einer Ausbreitung der ersten bösartigen Erkrankung, der Metastase der ersten bösartigen Erkrankung, der bösartigen Erkrankung unbekannter Herkunft oder unbekannter Art, oder der zweiten bösartigen Erkrankung bei einem anderen Patienten, falls der Replikin-Auszählwert der mindestens einen Zelle höher als der mittlere Replikin-Auszählwert der Mehrzahl von Zellen der bösartigen Erkrankung aus dem ersten Zeitraum und aus der ersten anatomischen Region zuzüglich einer Standardabweichung des mittleren Replikin-Auszählwerts der Mehrzahl von Zellen der bösartigen Erkrankung aus dem ersten Zeitraum und aus der ersten anatomische Region ist, oder falls der Replikin-Auszählwert der mindestens einen Zelle höher als der mittlere Replikin-Auszählwert der ersten Mehrzahl bösartiger Erkrankungen zuzüglich einer Standardabweichung des mittleren Replikin-Auszählwerts der ersten Mehrzahl bösartiger Erkrankungen ist, und
wobei das Verfahren mittels eines Computersystems durchgeführt wird.

21. Verfahren nach Anspruch 20, wobei es sich bei der mindestens einen weiteren Zelle der ersten bösartigen Erkrankung, der mindestens einen Zelle einer Metastase der ersten bösartigen Erkrankung oder der mindestens einen Zelle einer bösartigen Erkrankung unbekannter Herkunft oder unbekannter Art bei einem Patienten, der zu einem zweiten Zeitraum und/oder in einer zweiten anatomischen Region unter der ersten bösartigen Erkrankung leidet, oder bei der mindestens einen Zelle einer zweiten bösartigen Erkrankung bei einem anderen Patienten um eine Mehrzahl von Zellen aus dem zweiten Zeitraum und/oder der zweiten anatomischen Region oder der zweiten bösartigen Erkrankung bei einem anderen Patienten handelt, und wobei der Replikin-Auszählwert jeder Zelle der Mehrzahl von Zellen aus dem zweiten Zeitraum und/oder der zweiten anatomischen Region oder der zweiten bösartigen Erkrankung bei einem anderen Patienten einzeln mit dem mittleren Replikin-Auszählwert zuzüglich einer Standardabweichung des mittleren Replikin-Auszählwerts verglichen wird.

22. Verfahren nach Anspruch 21, wobei die Ausbreitung der bösartigen Erkrankung in dem zweiten Zeitraum und/oder der zweiten anatomischen Region oder die Ausbreitung der zweiten bösartigen Erkrankung bei einem anderen Patienten vorhergesagt wird, falls die Anzahl an Replikin-Auszählwerten der Mehrzahl von Zellen aus dem zweiten Zeitraum und/oder der zweiten anatomischen Region oder die Anzahl an Replikin-Auszählwerten der zweiten bösartigen Erkrankung bei einem anderen Patienten, die höher als der mittlere Replikin-Auszählwert der Mehrzahl von Zellen aus dem ersten Zeitraum in der ersten anatomischen Region oder aus der ersten Mehrzahl bösartiger Erkrankungen zuzüglich einer Standardabweichung des mittleren Replikin-Auszählwerts ist, höher ist als die Anzahl an Replikin-Auszählwerten der Mehrzahl von Zellen aus dem zweiten Zeitraum und/oder der zweiten anatomischen Region oder die Anzahl an Replikin-Auszählwerten der zweiten bösartigen Erkrankung bei einem anderen Patienten, die niedriger als der mittlere Replikin-Auszählwert der Mehrzahl von Zellen aus dem ersten Zeitraum in einer ersten anatomischen Region oder aus der ersten Mehrzahl bösartiger Erkrankungen abzüglich einer Standardabweichung des Mittelwerts ist.

23. Kit zum Erstellen einer Prognose für einen Patienten, der unter einer bösartigen Erkrankung leidet, wobei die Prognose die Sterblichkeit der bösartigen Erkrankung betrifft, umfassend ein maschinenlesbares Speichermedium, auf dem eine Software zur Bestimmung der Sterblichkeit einer bösartigen Erkrankung gemäß dem Verfahren nach Anspruch 8 gespeichert ist, wobei der Replikin-Auszählwert bei mindestens einer Zelle der bösartigen Erkrankung bestimmt wird.

24. Kit nach Anspruch 23, wobei die Formel sich aus einer Bestandsaufnahme bezüglich einer Mehrzahl bösartiger Erkrankungen einer ersten Art bösartiger Erkrankungen ableitet.

25. Kit nach Anspruch 24, wobei es sich bei der ersten Art bösartiger Erkrankungen um ein Glioblastom handelt.

26. Verfahren nach Anspruch 1, das weiterhin die Vorhersage der Sterblichkeit der ersten bösartigen Erkrankung umfasst, wobei das Verfahren umfasst:
Bestimmen eines Replikin-Auszählwerts der ersten bösartigen Erkrankung;
Durchführen einer Regressionsanalyse ausgehend einerseits von dem Replikin-Auszählwert einer Mehrzahl von zweiten bösartigen Erkrankungen,
einschließlich der zweiten bösartigen Erkrankung des Anspruchs 1, und andererseits von der Sterblichkeit der Mehrzahl von zweiten bösartigen Erkrankungen; und
Vorhersagen der Sterblichkeit der ersten bösartigen Erkrankung auf Grundlage der Regressionsanalyse der Mehrzahl von zweiten bösartigen Erkrankungen, und
wobei das Verfahren mittels eines Computersystems durchgeführt wird.

27. Verfahren nach Anspruch 26, wobei die Regressionsanalyse der Mehrzahl von zweiten bösartigen Erkrankungen unter Verwendung des Replikin-Auszählwerts jeder einzelnen zweiten bösartigen Erkrankung der Mehrzahl von zweiten bösartigen Erkrankungen und eines patientenbezogenen Ergebnisses zum Überleben für jede einzelne der zweiten bösartigen Erkrankungen durchgeführt wird.

28. Verfahren nach Anspruch 27, wobei es sich bei dem patientenbezogenen Ergebnis um die Überlebensdauer des Patienten nach der Diagnose der bösartigen Erkrankung handelt.

29. Verfahren nach Anspruch 1, das darüber hinaus das Vorhersagen der relativen Wachstumsrate der mindestens einen ersten bösartigen Erkrankung umfasst, wobei die mindestens eine erste bösartige Erkrankung von unbekannter oder bekannter Art ist, wobei das Verfahren umfasst:
Isolieren mindestens eines exprimierten Proteins, Proteinfragments oder Peptids aus einer Zelle der mindestens einen ersten bösartigen Erkrankung oder Isolieren des Genoms, mindestens eines Gens, mindestens eines Genfragments oder mindestens einer sonstigen Nukleinsäuresequenz aus der Zelle der mindestens einen ersten bösartigen Erkrankung;
Isolieren mindestens eines exprimierten Proteins, Proteinfragments oder Peptids aus einer Zelle der mindestens einen zweiten bösartigen Erkrankung nach Anspruch 1, wobei die mindestens eine zweite bösartige Erkrankung von unbekannter Art ist oder eine andere Art von bösartigen Erkrankungen als die mindestens eine erste bösartige Erkrankung darstellt, oder Isolieren des Genoms, mindestens eines Gens, mindestens eines Genfragments oder mindestens einer sonstigen Nukleinsäuresequenz aus der Zelle der mindestens einen zweiten bösartigen Erkrankung nach Anspruch 1;
Bestimmen eines Replikin-Auszählwerts der mindestens einen ersten bösartigen Erkrankung und eines Replikin-Auszählwerts der mindestens einen zweiten bösartigen Erkrankung;
Vergleichen des Replikin-Auszählwerts der mindestens einen ersten bösartigen Erkrankung mit dem Replikin-Auszählwert der mindestens einen zweiten bösartigen Erkrankung; und
Vorhersagen, dass die mindestens eine erste bösartige Erkrankung eine relative Wachstumsrate aufweist, die schneller als die relative Wachstumsrate der mindestens einen zweiten bösartigen Erkrankung ist, falls der Replikin-Auszählwert der mindestens einen ersten bösartigen Erkrankung höher als der Replikin-Auszählwert der mindestens einen zweiten bösartigen Erkrankung ist.

## Revendications

1. Procédé de prédiction du taux de croissance relatif d'au moins une première tumeur maligne de cancer d'un type inconnu ou connu comprenant:
la comparaison du comptage de Replikin de la au moins une première tumeur maligne avec un comptage de Replikin d'au moins une seconde tumeur maligne de type inconnu ou d'un type de tumeur maligne différent de la au moins une première tumeur maligne; et
la prédiction que la au moins une première tumeur maligne a un taux de croissance relatif qui est plus rapide que le taux de croissance relatif de la au moins une seconde tumeur maligne si le comptage de Replikin de la au moins une première tumeur maligne est supérieur au comptage de Replikin de la au moins une seconde tumeur maligne,
dans lequel le comptage de Replikin est le nombre de séquences de Replikin pour 100 acides aminés dans une séquence d'acides aminés et une séquence de Replikin est déterminée par l'identification d'une séquence d'acides aminés de 7 à 50 résidus d'acides aminés avec au moins un résidu lysine situé à la première extrémité terminale de ladite séquence d'acides aminés et au moins un résidu lysine, ou au moins un résidu histidine, situé à la seconde extrémité terminale de ladite séquence d'acides aminés, dans laquelle la séquence de Replikin comprend (1) un premier résidu lysine situé entre six et dix résidus à partir d'un second résidu lysine; (2) au moins un résidu histidine; et (3) au moins 6 % de résidus lysine, et
dans lequel ledit procédé est mis en oeuvre par un système informatique.

2. Procédé selon la revendication 1, comprenant en outre: la détermination du comptage de Replikin de la au moins une première tumeur maligne et le comptage de Replikin de la au moins une seconde tumeur maligne avant l'étape de comparaison.

3. Procédé selon la revendication 1, dans lequel ladite au moins une première tumeur maligne est une métastase de ladite au moins une seconde tumeur maligne, est une tumeur maligne de type inconnu ou d'origine inconnue chez un sujet souffrant de ladite au moins une seconde tumeur maligne, ou est une cellule maligne de la même tumeur maligne que ladite au moins une seconde tumeur maligne, ou dans lequel lesdites au moins une première tumeur maligne et au moins une seconde tumeur maligne sont des métastases d'une troisième tumeur maligne.

4. Procédé selon la revendication 1, dans lequel le comptage de Replikin de ladite au moins une première tumeur maligne est le comptage de Replikin d'un gène Replikin de pointe de la au moins une première tumeur maligne et le comptage de Replikin de ladite au moins une seconde tumeur maligne est le comptage de Replikin d'un gène Replikin de pointe de ladite au moins une seconde tumeur maligne.

5. Procédé selon la revendication 1, dans lequel (1) ledit comptage de Replikin de ladite au moins une seconde tumeur maligne est un comptage de Replikin moyen d'une pluralité de tumeurs malignes ou un comptage de Replikin moyen d'une pluralité de cellules de la au moins une seconde tumeur maligne; (2) ledit comptage de Replikin de ladite au moins une première tumeur maligne est un comptage de Replikin moyen d'une pluralité de tumeurs malignes ou un comptage de Replikin moyen d'une pluralité de cellules de la au moins une première tumeur maligne; ou (3), à la fois, ledit comptage de Replikin de ladite au moins une seconde tumeur maligne est un comptage de Replikin moyen d'une pluralité de tumeurs malignes ou un comptage de Replikin moyen d'une pluralité de cellules de la au moins une seconde tumeur maligne, et ledit comptage de Replikin de ladite au moins une première tumeur maligne est un comptage de Replikin moyen d'une pluralité de tumeurs malignes ou un comptage de Replikin moyen d'une pluralité de cellules de la au moins une première tumeur maligne.

6. Procédé selon la revendication 1, dans lequel ledit comptage de Replikin de ladite au moins une seconde tumeur maligne est un comptage de Replikin moyen, valeur médiane ou une autre moyenne d'une pluralité de tumeurs malignes, dans lequel ladite pluralité de tumeurs malignes représente une étude de tumeurs malignes.

7. Procédé selon la revendication 1, dans lequel le comptage de Replikin de ladite au moins une première tumeur maligne, ou le comptage de Replikin moyen de ladite au moins une première tumeur maligne, est supérieur au comptage de Replikin moyen de ladite au moins une seconde tumeur maligne plus un écart type de comptage de Replikin moyen de ladite au moins une seconde tumeur maligne.

8. Procédé selon la revendication 1, dans lequel ledit taux de croissance relatif est en corrélation avec la létalité relative.

9. Procédé selon la revendication 1, dans lequel ledit comptage de Replikin de ladite au moins une première tumeur maligne est un comptage de Replikin d'une protéine exprimée, d'un fragment de protéine ou d'un peptide isolé d'une cellule de ladite au moins une première tumeur maligne, ou un comptage de Replikin du génome, d'un gène, d'un fragment de gène, ou de toute autre séquence d'acide nucléique isolée à partir d'une cellule de ladite au moins une première tumeur maligne ; et dans lequel ledit comptage de Replikin de ladite au moins une seconde tumeur maligne est un comptage de Replikin d'une protéine exprimée, d'un fragment de protéine ou d'un peptide isolé à partir d'une cellule de ladite au moins une seconde tumeur maligne, ou un comptage de Replikin du génome, d'un gène, d'un fragment de gène, ou de toute autre séquence d'acides nucléiques isolée à partir d'une cellule de ladite au moins une seconde tumeur maligne.

10. Procédé selon la revendication 1, dans lequel ledit comptage de Replikin de ladite au moins une première tumeur maligne est le comptage de Replikin le plus élevé parmi une pluralité des comptages de Replikin d'une première pluralité de tumeurs malignes d'un premier type, et ledit comptage de Replikin de ladite au moins une seconde tumeur maligne est le comptage de Replikin le plus élevé parmi une pluralité des comptages de Replikin d'une seconde pluralité de tumeurs malignes d'un second type.

11. Procédé selon la revendication 10, comprenant en outre:
la comparaison d'un comptage de Replikin d'un gène Replikin de pointe de la tumeur maligne ayant le comptage de Replikin le plus élevé parmi une pluralité de comptages de Replikin desdites tumeurs malignes du premier type par rapport au comptage de Replikin du gène Replikin de pointe de la tumeur maligne ayant le comptage de Replikin le plus élevé parmi une pluralité de comptages de Replikin desdites tumeurs malignes du second type ; et
si lesdites tumeur malignes du premier type ont un comptage de Replikin dudit gène Replikin de pointe plus élevé que lesdites tumeurs malignes du second type, alors il est prédit que lesdites tumeurs malignes de premier type ont un impact plus important sur la létalité que lesdites tumeurs malignes de second type.

12. Procédé selon la revendication 1, dans lequel ladite au moins une première tumeur maligne est une tumeur maligne de la thyroïde, une tumeur maligne de la prostate, une tumeur maligne du sein, une tumeur maligne de la vessie, une tumeur maligne du corps utérin, une tumeur maligne du col de l'utérus, une tumeur maligne du colon, une tumeur maligne de l'ovaire, une tumeur maligne de la cavité buccale, une leucémie lymphoïde maligne, un myélome multiple malin, une tumeur gastrique maligne, un carcinome malin du poumon non à petites cellules, ou un glioblastome malin.

13. Procédé selon la revendication 1, dans lequel le procédé est exécuté par un processeur et dans lequel au moins un comptage de Replikin ou au moins une représentation de ladite prédiction du taux de croissance relative de ladite au moins une première tumeur maligne est restituée à l'utilisateur ou est affichée.

14. Support de stockage lisible par machine sur lequel sont stockées les instructions exécutables, qui, lorsqu'elles sont exécutées par le processeur, mettent en oeuvre le procédé selon la revendication 1.

15. Procédé pour déterminer la vitesse relative de l'augmentation du taux de croissance relatif d'au moins une première tumeur maligne par rapport à la vitesse relative de l'augmentation du taux de croissance relatif de la au moins une seconde tumeur maligne comprenant:
la comparaison de l'écart type du comptage de Replikin moyen d'une pluralité de cellules de ladite au moins une première tumeur maligne avec l'écart type du comptage de Replikin moyen d'une pluralité de cellules de ladite au moins une seconde tumeur maligne; et
la prédiction que l'augmentation relative du taux de croissance relatif de ladite au moins une première tumeur maligne est supérieure à la vitesse relative de l'augmentation du taux de croissance relatif de ladite au moins une seconde tumeur maligne si l'écart type du comptage de Replikin moyen de la pluralité de cellules de ladite au moins une première tumeur maligne est supérieur à l'écart type du comptage de Replikin moyen d'une pluralité de cellules de ladite au moins une seconde tumeur maligne,
dans lequel le comptage de Replikin est le nombre de séquences de Replikin pour 100 acides aminés dans une séquence d'acides aminés et la séquence de Replikin est déterminée en identifiant la séquence d'acides aminés entre 7 et 50 résidus d'acides aminés avec au moins un résidu lysine situé à la première extrémité terminale de ladite séquence d'acides aminés et au moins un résidu lysine, ou au moins un résidu histidine, situé à la seconde extrémité terminale de ladite séquence d'acides aminés dans laquelle la séquence de Replikin comprend (1) un premier résidu lysine situé entre six et dix résidus à partir d'un second résidu lysine; (2) au moins un résidu histidine; et (3) au moins 6 % de résidus lysine, et
dans lequel ledit procédé est mis en oeuvre par un système informatique.

16. Procédé selon la revendication 15, dans lequel ladite au moins une première tumeur maligne est une métastase de ladite au moins une seconde tumeur maligne ou dans lequel ladite au moins une première tumeur maligne est une tumeur maligne d'origine inconnue ou de type inconnu chez un sujet souffrant de ladite au moins une seconde tumeur maligne.

17. Procédé selon la revendication 15, dans lequel ladite au moins une première tumeur maligne et ladite au moins une seconde tumeur maligne sont toutes les deux des métastases d'une troisième tumeur maligne.

18. Procédé selon la revendication 15, dans lequel ladite au moins une première tumeur maligne est une pluralité de tumeurs malignes différentes desdites première et seconde tumeurs malignes, ladite au moins une seconde tumeur maligne est une pluralité de tumeurs malignes différentes de ladite au moins une première tumeur maligne, ou ladite au moins une première tumeur maligne est une pluralité de tumeurs malignes différentes de ladite au moins une seconde tumeur maligne et ladite au moins une seconde tumeur maligne est une pluralité de tumeurs malignes différentes de ladite au moins une première tumeur maligne.

19. Procédé selon la revendication 18, dans lequel ladite au moins une première tumeur maligne est une pluralité de tumeurs malignes d'un premier type de tumeur maligne et ladite au moins une seconde tumeur maligne est une pluralité de tumeurs malignes d'un second type de tumeur maligne.

20. Procédé selon la revendication 1, comprenant en outre la prédiction d'une extension d'une tumeur maligne comprenant:
la détermination du comptage de Replikin moyen et de l'écart type dudit comptage de Replikin moyen pour une pluralité de cellules de ladite première tumeur maligne selon la revendication 1 pendant une première période de temps dans une première région anatomique ou pour une première pluralité de tumeurs malignes incluant ladite première tumeur maligne selon la revendication 1 ;
la détermination du comptage de Replikin de ladite seconde tumeur maligne selon la revendication 1 dans laquelle ledit comptage de Replikin pour ladite seconde tumeur maligne est défini comme étant le comptage de Replikin d'au moins une autre cellule de la première tumeur maligne, au moins une cellule de la métastase de la première tumeur maligne, ou au moins une cellule d'une tumeur maligne d'origine inconnue ou de type inconnu chez un sujet atteint de la première tumeur maligne à une seconde période de temps et/ou dans une seconde région anatomique, dans lequel ladite seconde période de temps est différente de ladite première période de temps et/ou ladite seconde région anatomique est différente de ladite première région anatomique, ou par la détermination du comptage de Replikin de la au moins une cellule d'une seconde tumeur maligne chez un sujet; et
la prédiction de l'extension de ladite première tumeur maligne, de ladite métastase de ladite première tumeur maligne, de ladite tumeur maligne d'origine inconnue ou de type inconnu, ou de ladite seconde tumeur maligne chez un sujet différent si le comptage de Replikin de ladite au moins une cellule est supérieur au comptage de Replikin moyen de la pluralité de cellules de ladite tumeur maligne à partir de ladite première période de temps et à partir de ladite première région anatomique plus un écart type de comptage de Replikin moyen de la pluralité des cellules de ladite tumeur maligne à partir de ladite première période de temps et en provenance de ladite première région anatomique ou si le comptage de Replikin de ladite au moins une cellule est supérieur au comptage de Replikin moyen de la première pluralité de tumeurs malignes plus un écart type de comptage de Replikin moyen de la première pluralité de tumeurs malignes, et
dans lequel ledit procédé est mis en oeuvre par un système informatique.

21. Procédé selon la revendication 20, dans lequel ladite au moins une autre cellule de la première tumeur maligne, au moins une cellule de la métastase de la première tumeur maligne, ou au moins une cellule d'une tumeur maligne d'origine inconnue ou de type inconnu chez un sujet atteint de la première tumeur maligne pendant la seconde période de temps et/ou dans une seconde région anatomique, ou ladite au moins une cellule de la seconde tumeur maligne chez un sujet différent est une pluralité de cellules provenant de ladite seconde période de temps et/ou de ladite seconde région anatomique ou de ladite seconde tumeur maligne chez un sujet différent, et le comptage de Replikin de chaque cellule de la pluralité de cellules provenant de la seconde période de temps et/ou de la seconde région anatomique ou de ladite seconde tumeur maligne chez un sujet différent est comparé de manière séparée audit comptage de Replikin moyen plus un écart type dudit comptage de Replikin moyen.

22. Procédé selon la revendication 21, dans lequel l'extension de ladite tumeur maligne pendant ladite seconde période de temps et/ou dans ladite seconde région anatomique ou l'expansion de ladite seconde tumeur maligne chez un autre sujet est prédite si le nombre de comptages de Replikin de ladite pluralité de cellules à partir de ladite seconde période de temps et/ou de ladite seconde région anatomique ou le nombre de comptages de Replikin de ladite seconde tumeur maligne chez un sujet différent qui est supérieur audit comptage de Replikin moyen de la pluralité de cellules à partir de ladite première période de temps dans ladite première région anatomique ou à partir de ladite première pluralité de tumeurs malignes plus un écart type de la moyenne des comptages de Replikin est supérieur au nombre de comptages de Replikin de ladite pluralité de cellules à partir de ladite seconde période de temps et/ou de ladite seconde région anatomique ou le nombre de comptages de Replikin de ladite seconde tumeur maligne chez un sujet différent qui est inférieur audit comptage de Replikin moyen de ladite pluralité de cellules à partir de la première période de temps dans ladite première région anatomique ou à partir de ladite première pluralité de tumeurs malignes moins un écart type de la moyenne.

23. Kit pour fournir une prédiction pour un patient souffrant d'une tumeur maligne, ledit pronostic concernant la létalité de la tumeur maligne, comprenant un support de stockage lisible par machine sur lequel est stocké un logiciel de détermination de la létalité d'une tumeur maligne selon le procédé de la revendication 8, dans lequel ledit comptage de Replikin est déterminé dans au moins une cellule de la tumeur maligne.

24. Kit selon la revendication 23, dans lequel ladite formule est issue de l'étude d'une pluralité de tumeurs malignes d'un premier type de tumeur maligne.

25. Kit selon la revendication 24, dans lequel le premier type de tumeur maligne est un glioblastome.

26. Procédé selon la revendication 1, comprenant en outre la prédiction de la létalité de ladite première tumeur maligne comprenant:
la détermination du comptage de Replikin de ladite première tumeur maligne;
la réalisation d'une analyse de la régression par le comptage de Replikin d'une pluralité de secondes tumeurs malignes, incluant la seconde tumeur maligne selon la revendication 1, en fonction de la létalité de ladite pluralité des secondes tumeurs malignes; et
la prédiction de la létalité de la première tumeur maligne qui se base sur l'analyse de la régression de la pluralité des secondes tumeurs malignes, et dans lequel ledit procédé est mis en oeuvre par un système informatique.

27. Procédé selon la revendication 26, dans lequel l'analyse de la régression de la pluralité des secondes tumeurs malignes est réalisée en utilisant le comptage de Replikin de chaque seconde tumeur maligne prise individuellement dans la pluralité des secondes tumeurs malignes et le résultat quant à la survie du patient pour chaque seconde tumeur maligne prise individuellement.

28. Procédé selon la revendication 27, dans lequel l'issue pour le patient est la durée de survie du patient à la suite du diagnostic de la tumeur maligne.

29. Procédé selon la revendication 1, comprenant en outre la prédiction du taux de croissance relatif de ladite au moins une première tumeur maligne dans lequel ladite au moins une tumeur maligne est d'un type inconnu ou connu comprenant:
l'isolement d'au moins une protéine exprimée, d'un fragment de protéine, ou d'un peptide provenant d'une cellule de ladite au moins une première tumeur maligne ou l'isolement du génome, d'au moins un gène, d'au moins un fragment de gène, ou d'au moins une séquence d'un autre acide nucléique provenant de la cellule de la au moins une première tumeur maligne ;
l'isolement d'au moins une protéine exprimée, d'un fragment de protéine, ou d'un peptide provenant d'une cellule de ladite au moins une seconde tumeur maligne selon la revendication 1 dans laquelle ladite au moins une seconde tumeur maligne est de type inconnu ou d'un type de tumeur maligne différent de la au moins une première tumeur maligne ou l'isolement du génome, d'au moins un gène, d'au moins un fragment de gène , ou d'au moins une séquence d'un autre acide nucléique provenant de la cellule de la au moins une seconde tumeur maligne selon la revendication 1;
la détermination d'un comptage de Replikin de ladite au moins une première tumeur maligne et d'un comptage de Replikin de ladite au moins une seconde tumeur maligne;
la comparaison du comptage de Replikin de la au moins une première tumeur maligne avec le comptage de Replikin de la au moins une seconde tumeur maligne; et
la prédiction qu'au moins une première tumeur maligne aura un taux de croissance relatif qui sera plus rapide que le taux de croissance relatif de la au moins une seconde tumeur maligne si le comptage de Replikin de la au moins une première tumeur maligne est supérieur au comptage de Replikin de la au moins une seconde tumeur maligne.


### SEQUENCE LISTING

<110> BOGOCH, SAMUEL BOGOCH, ELENORE S. BOGOCH, SAMUEL WINSTON BORSANYI, ANNE ELENORE
<120> METHODS OF PREDICTING CANCER LETHALITY USING REPLIKIN COUNTS
<130> 13794/47801
<140> PCTU50953208
   <141> 2009-08-07
<150> 61/185,160
   <151> 2009-06-08
<150> 61/179,686
   <151> 2009-05-19
<150> 61/172,115
   <151> 2009-04-23
<150> 12/429,044
   <151> 2009-04-23
<150> PCT/U52009/041565
   <151> 2009-04-23
<150> 61/143,618
   <151> 2009-01-09
<150> 61/087,354
   <151> 2008-08-08
<160> 330
<170> PatentIn version 3.5
<210> 1
   <211> 767
   <212> PRT
   <213> Rattus norvegicus
<400> 1
   Met Ser Gly Asp His Leu His Asn Asp Ser Gln Ile Glu Ala Asp Phe
   1               5                   10                  15
   Arg Leu Asn Asp Ser His Lys His Lys Asp Lys His Lys Asp Arg Glu
               20                  25                  30
   His Arg His Lys Glu His Lys Lys Asp Lys Asp Lys Asp Arg Glu Lys
           35                  40                  45
   Ser Lys His Ser Asn Ser Glu His Lys Asp Ser Glu Lys Lys His Lys
   50 55 60
   Glu Lys Glu Lys Thr Lys His Lys Asp Gly Ser Ser Asp Lys His Lys
   65 70 75 80
   Asp Lys His Lys Asp Arg Asp Lys Glu Lys Arg Lys Glu Glu Lys Ile
   85 90 95
   Arg Ala Ala Gly Asp Ala Lys Ile Lys Lys Glu Lys Glu Asn Gly Phe
   100 105 110
   Ser Ser Pro Pro Arg Ile Lys Asp Glu Pro Glu Asp Asp Gly Tyr Phe
   115 120 125
   Ala Pro Pro Lys Glu Asp Ile Lys Pro Leu Lys Arg Pro Arg Asp Glu
   130 135 140
   Asp Asp Ala Asp Tyr Lys Pro Lys Lys Ile Lys Thr Glu Asp Ile Lys
   145 150 155 160
   Lys Glu Lys Lys Arg Lys Leu Glu Glu Glu Glu Asp Gly Lys Leu Lys
   165 170 175
   Lys Pro Lys Asn Lys Asp Lys Asp Lys Lys Val Ala Glu Pro Asp Asn
   180 185 190
   Lys Lys Lys Lys Ala Lys Lys Glu Glu Glu Gln Lys Trp Lys Trp Trp
   195 200 205
   Glu Glu Glu Arg Tyr Pro Glu Gly Ile Lys Trp Lys Phe Leu Glu His
   210 215 220
   Lys Gly Pro Val Phe Ala Pro Pro Tyr Glu Pro Leu Pro Glu Gly Val
   225 230 235 240
   Lys Phe Tyr Tyr Asp Gly Lys Val Met Lys Leu Ser Pro Lys Ala Glu
   245 250 255
   Glu Val Ala Thr Phe Phe Ala Lys Met Leu Asp His Glu Tyr Thr Thr
   260 265 270
   Lys Glu Ile Phe Arg Lys Asn Phe Phe Lys Asp Trp Arg Lys Glu Met
   275 280 285
   Thr Asn Asp Glu Lys Asn Thr Ile Thr Asn Leu Ser Lys Cys Asp Phe
   290 295 300
   Thr Gln Met Ser Gln Tyr Phe Lys Ala Gln Ser Glu Ala Arg Lys Gln
   305 310 315 320
   Met Ser Lys Glu Glu Lys Leu Lys Ile Lys Glu Glu Asn Glu Lys Leu
   325 330 335
   Leu Lys Glu Tyr Gly Phe Cys Val Met Asp Asn His Arg Glu Arg Ile
   340 345 350
   Ala Asn Phe Lys Ile Glu Pro Pro Gly Leu Phe Arg Gly Arg Gly Asn
   355 360 365
   His Pro Lys Met Gly Met Leu Lys Arg Arg Ile Met Pro Glu Asp Ile
   370 375 380
   Ile Ile Asn Cys Ser Lys Asp Ala Lys Val Pro Ser Pro Pro Pro Gly
   385 390 395 400
   His Lys Trp Lys Glu Val Arg His Asp Asn Lys Val Thr Trp Leu Val
   405 410 415
   Ser Trp Thr Glu Asn Ile Gln Gly Ser Ile Lys Tyr Ile Met Leu Asn
   420 425 430
   Pro Ser Ser Arg Ile Lys Gly Glu Lys Asp Trp Gln Lys Tyr Glu Thr
   435 440 445
   Ala Arg Arg Leu Lys Lys Cys Val Asp Lys Ile Arg Asn Gln Tyr Arg
   450 455 460
   Glu Asp Trp Lys Ser Lys Glu Met Lys Val Arg Gln Arg Ala Val Ala
   465 470 475 480
   Leu Tyr Phe Ile Asp Lys Leu Ala Leu Arg Ala Gly Asn Glu Lys Glu
   485 490 495
   Glu Gly Glu Thr Ala Asp Thr Val Gly Cys Cys Ser Leu Arg Val Glu
   500 505 510
   His Ile Asn Leu His Pro Glu Leu Asp Gly Gln Glu Tyr Val Val Glu
   515 520 525
   Phe Asp Phe Pro Gly Lys Asp Ser Ile Arg Tyr Tyr Asn Lys Val Pro
   530 535 540
   Val Glu Lys Arg Val Phe Lys Asn Leu Gln Leu Phe Met Glu Asn Lys
   545 550 555 560
   Gln Pro Glu Asp Asp Leu Phe Asp Arg Leu Asn Thr Gly Ile Leu Asn
   565 570 575
   Lys His Leu Gln Asp Leu Met Glu Gly Leu Thr Ala Lys Val Phe Arg
   580 585 590
   Thr Tyr Asn Ala Ser Ile Thr Leu Gln Gln Gln Leu Lys Glu Leu Thr
   595 600 605
   Ala Pro Asp Glu Asn Val Pro Ala Lys Ile Leu Ser Tyr Asn Arg Ala
   610 615 620
   Asn Arg Ala Val Ala Ile Leu Cys Asn His Gln Arg Ala Pro Pro Lys
   625 630 635 640
   Thr Phe Glu Lys Ser Met Met Asn Leu Gln Ser Lys Ile Asp Ala Lys
   645 650 655
   Lys Asp Gln Leu Ala Asp Ala Arg Lys Asp Leu Lys Ser Ala Lys Ala
   660 665 670
   Asp Ala Lys Val Met Lys Asp Ala Lys Thr Lys Lys Val Val Glu Ser
   675 680 685
   Lys Lys Lys Ala Val Gln Arg Leu Glu Glu Gln Leu Met Lys Leu Glu
   690 695 700
   Val Gln Ala Thr Asp Arg Glu Glu Asn Lys Gln Ile Ala Leu Gly Thr
   705 710 715 720
   Ser Lys Leu Asn Tyr Leu Asp Pro Arg Ile Thr Val Ala Trp Cys Lys
   725 730 735
   Lys Trp Gly Val Pro Ile Glu Lys Ile Tyr Asn Lys Thr Gln Arg Glu
   740 745 750
   Lys Phe Ala Trp Ala Ile Asp Met Thr Asp Glu Asp Tyr Glu Phe
   755 760 765
<210> 2
   <211> 25
   <212> PRT
   <213> Rattus norvegicus
<400> 2
   His Leu His Asn Asp Ser Gln Ile Glu Ala Asp Phe Arg Leu Asn Asp
   1 5 10 15
   Ser His Lys His Lys Asp Lys His Lys
   20 25
<210> 3
   <211> 32
   <212> PRT
   <213> Rattus norvegicus
<400> 3
   His Leu His Asn Asp Ser Gln Ile Glu Ala Asp Phe Arg Leu Asn Asp
   1 5 10 15
   Ser His Lys His Lys Asp Lys His Lys Asp Arg Glu His Arg His Lys
   20 25 30
<210> 4
   <211> 35
   <212> PRT
   <213> Rattus norvegicus
<400> 4
   His Leu His Asn Asp Ser Gln Ile Glu Ala Asp Phe Arg Leu Asn Asp
   1 5 10 15
   Ser His Lys His Lys Asp Lys His Lys Asp Arg Glu His Arg His Lys
   20 25 30
   Glu His Lys
   35
<210> 5
   <211> 38
   <212> PRT
   <213> Rattus norvegicus
<400> 5
   His Leu His Asn Asp Ser Gln Ile Glu Ala Asp Phe Arg Leu Asn Asp
   1               5                   10                  15
   Ser His Lys His Lys Asp Lys His Lys Asp Arg Glu His Arg His Lys
               20                  25                  30
   Glu His Lys Lys Asp Lys
           35
<210> 6
   <211> 40
   <212> PRT
   <213> Rattus norvegicus
<400> 6
   His Leu His Asn Asp Ser Gln Ile Glu Ala Asp Phe Arg Leu Asn Asp
   1               5                   10                  15
   Ser His Lys His Lys Asp Lys His Lys Asp Arg Glu His Arg His Lys
               20                  25                  30
   Glu His Lys Lys Asp Lys Asp Lys
           35                  40
<210> 7
   <211> 44
   <212> PRT
   <213> Rattus norvegicus
<400> 7
   His Leu His Asn Asp Ser Gln Ile Glu Ala Asp Phe Arg Leu Asn Asp
   1               5                   10                  15
   Ser His Lys His Lys Asp Lys His Lys Asp Arg Glu His Arg His Lys
               20                  25                  30
   Glu His Lys Lys Asp Lys Asp Lys Asp Arg Glu Lys
           35                  40
<210> 8
   <211> 46
   <212> PRT
   <213> Rattus norvegicus
<400> 8
   His Leu His Asn Asp Ser Gln Ile Glu Ala Asp Phe Arg Leu Asn Asp
   1               5                   10                  15
   Ser His Lys His Lys Asp Lys His Lys Asp Arg Glu His Arg His Lys
               20                  25                  30
   Glu His Lys Lys Asp Lys Asp Lys Asp Arg Glu Lys Ser Lys
           35                  40                  45
<210> 9
   <211> 23
   <212> PRT
   <213> Rattus norvegicus
<400> 9
   His Asn Asp Ser Gln Ile Glu Ala Asp Phe Arg Leu Asn Asp Ser His
   1               5                   10                  15
   Lys His Lys Asp Lys His Lys
               20
<210> 10
   <211> 30
   <212> PRT
   <213> Rattus norvegicus
<400> 10
   His Asn Asp Ser Gln Ile Glu Ala Asp Phe Arg Leu Asn Asp Ser His
   1               5                   10                  15
   Lys His Lys Asp Lys His Lys Asp Arg Glu His Arg His Lys
               20                  25                  30
<210> 11
   <211> 33
   <212> PRT
   <213> Rattus norvegicus
<400> 11
   His Asn Asp Ser Gln Ile Glu Ala Asp Phe Arg Leu Asn Asp Ser His
   1               5                   10                  15
   Lys His Lys Asp Lys His Lys Asp Arg Glu His Arg His Lys Glu His
               20                  25                  30
   Lys
<210> 12
   <211> 36
   <212> PRT
   <213> Rattus norvegicus
<400> 12
   His Asn Asp Ser Gln Ile Glu Ala Asp Phe Arg Leu Asn Asp Ser His
   1               5                   10                  15
   Lys His Lys Asp Lys His Lys Asp Arg Glu His Arg His Lys Glu His
               20                  25                  30
   Lys Lys Asp Lys
           35
<210> 13
   <211> 38
   <212> PRT
   <213> Rattus norvegicus
<400> 13
   His Asn Asp Ser Gln Ile Glu Ala Asp Phe Arg Leu Asn Asp Ser His
   1               5                   10                  15
   Lys His Lys Asp Lys His Lys Asp Arg Glu His Arg His Lys Glu His
               20                  25                  30
   Lys Lys Asp Lys Asp Lys
           35
<210> 14
   <211> 42
   <212> PRT
   <213> Rattus norvegicus
<400> 14
   His Asn Asp Ser Gln Ile Glu Ala Asp Phe Arg Leu Asn Asp Ser His
   1               5                   10                  15
   Lys His Lys Asp Lys His Lys Asp Arg Glu His Arg His Lys Glu His
               20                  25                  30
   Lys Lys Asp Lys Asp Lys Asp Arg Glu Lys
           35                  40
<210> 15
   <211> 44
   <212> PRT
   <213> Rattus norvegicus
<400> 15
   His Asn Asp Ser Gln Ile Glu Ala Asp Phe Arg Leu Asn Asp Ser His
   1 5 10 15
   Lys His Lys Asp Lys His Lys Asp Arg Glu His Arg His Lys Glu His
   20 25 30
   Lys Lys Asp Lys Asp Lys Asp Arg Glu Lys Ser Lys
   35 40
<210> 16
   <211> 51
   <212> PRT
   <213> Rattus norvegicus
<400> 16
   His Asn Asp Ser Gln Ile Glu Ala Asp Phe Arg Leu Asn Asp Ser His
   1 5 10 15
   Lys His Lys Asp Lys His Lys Asp Arg Glu His Arg His Lys Glu His
   20 25 30
   Lys Lys Asp Lys Asp Lys Asp Arg Glu Lys Ser Lys His Ser Asn Ser
   35 40 45
   Glu His Lys
   50
<210> 17
   <211> 8
   <212> PRT
   <213> Rattus norvegicus
<400> 17
   His Lys His Lys Asp Lys His Lys
   1 5
<210> 18
   <211> 15
   <212> PRT
   <213> Rattus norvegicus
<400> 18
   His Lys His Lys Asp Lys His Lys Asp Arg Glu His Arg His Lys
   1 5 10 15
<210> 19
   <211> 18
   <212> PRT
   <213> Rattus norvegicus
<400> 19
   His Lys His Lys Asp Lys His Lys Asp Arg Glu His Arg His Lys Glu
   1               5                   10                  15
   His Lys
<210> 20
   <211> 21
   <212> PRT
   <213> Rattus norvegicus
<400> 20
   His Lys His Lys Asp Lys His Lys Asp Arg Glu His Arg His Lys Glu
   1               5                   10                  15
   His Lys Lys Asp Lys
               20
<210> 21
   <211> 23
   <212> PRT
   <213> Rattus norvegicus
<400> 21
   His Lys His Lys Asp Lys His Lys Asp Arg Glu His Arg His Lys Glu
   1               5                   10                  15
   His Lys Lys Asp Lys Asp Lys
               20
<210> 22
   <211> 27
   <212> PRT
   <213> Rattus norvegicus
<400> 22
   His Lys His Lys Asp Lys His Lys Asp Arg Glu His Arg His Lys Glu
   1               5                   10                  15
   His Lys Lys Asp Lys Asp Lys Asp Arg Glu Lys
               20                  25
<210> 23
   <211> 29
   <212> PRT
   <213> Rattus norvegicus
<400> 23
   His Lys His Lys Asp Lys His Lys Asp Arg Glu His Arg His Lys Glu
   1               5                   10                  15
   His Lys Lys Asp Lys Asp Lys Asp Arg Glu Lys Ser Lys
               20                  25
<210> 24
   <211> 36
   <212> PRT
   <213> Rattus norvegicus
<400> 24
   His Lys His Lys Asp Lys His Lys Asp Arg Glu His Arg His Lys Glu
   1               5                   10                  15
   His Lys Lys Asp Lys Asp Lys Asp Arg Glu Lys Ser Lys His Ser Asn
               20                  25                  30
   Ser Glu His Lys
           35
<210> 25
   <211> 43
   <212> PRT
   <213> Rattus norvegicus
<400> 25
   His Lys His Lys Asp Lys His Lys Asp Arg Glu His Arg His Lys Glu
   1               5                   10                  15
   His Lys Lys Asp Lys Asp Lys Asp Arg Glu Lys Ser Lys His Ser Asn
               20                  25                  30
   Ser Glu His Lys Asp Ser Glu Lys Lys His Lys
           35                  40
<210> 26
   <211> 45
   <212> PRT
   <213> Rattus norvegicus
<400> 26
   His Lys His Lys Asp Lys His Lys Asp Arg Glu His Arg His Lys Glu
   1 5 10 15
   His Lys Lys Asp Lys Asp Lys Asp Arg Glu Lys Ser Lys His Ser Asn
   20 25 30
   Ser Glu His Lys Asp Ser Glu Lys Lys His Lys Glu Lys
   35 40 45
<210> 27
   <211> 47
   <212> PRT
   <213> Rattus norvegicus
<400> 27
   His Lys His Lys Asp Lys His Lys Asp Arg Glu His Arg His Lys Glu
   1 5 10 15
   His Lys Lys Asp Lys Asp Lys Asp Arg Glu Lys Ser Lys His Ser Asn
   20 25 30
   Ser Glu His Lys Asp Ser Glu Lys Lys His Lys Glu Lys Glu Lys
   35 40 45
<210> 28
   <211> 49
   <212> PRT
   <213> Rattus norvegicus
<400> 28
   His Lys His Lys Asp Lys His Lys Asp Arg Glu His Arg His Lys Glu
   1 5 10 15
   His Lys Lys Asp Lys Asp Lys Asp Arg Glu Lys Ser Lys His Ser Asn
   20 25 30
   Ser Glu His Lys Asp Ser Glu Lys Lys His Lys Glu Lys Glu Lys Thr
   35 40 45
   Lys
<210> 29
   <211> 51
   <212> PRT
   <213> Rattus norvegicus
<400> 29
   His Lys His Lys Asp Lys His Lys Asp Arg Glu His Arg His Lys Glu
   1 5 10 15
   His Lys Lys Asp Lys Asp Lys Asp Arg Glu Lys Ser Lys His Ser Asn
   20 25 30
   Ser Glu His Lys Asp Ser Glu Lys Lys His Lys Glu Lys Glu Lys Thr
   35 40 45
   Lys His Lys
   50
<210> 30
   <211> 29
   <212> PRT
   <213> Rattus norvegicus
<400> 30
   Lys His Lys Asp Lys His Lys Asp Arg Glu His Arg His Lys Glu His
   1 5 10 15
   Lys Lys Asp Lys Asp Lys Asp Arg Glu Lys Ser Lys His
   20 25
<210> 31
   <211> 34
   <212> PRT
   <213> Rattus norvegicus
<400> 31